# EUROPÄISCHE PATENTSCHRIFT

(11) **EP 2 773 626 B1**
(45) Veröffentlichungstag und Bekanntmachung des Hinweises auf die Patenterteilung: **05.08.2015**
(21) Anmeldenummer: 12797732.0
(22) Anmeldetag: 29.10.2012
(51) Int. Cl.: C07D 249/14, C07D 257/04, C07D 257/06, C07D 271/08, C07D 271/113, A01N 43/653, A01N 43/713, A01N 43/82

(54) **HERBIZID WIRKSAME OXIMETHER SUBSTITUIERTER BENZOYLAMIDE**
HERBICIDALLY ACTIVE OXIME-ETHER-SUBSTITUTED BENZOYLAMIDES
BENZOYLAMIDES À SUBSTITUTION ÉTHER D'OXIME AGISSANT COMME HERBICIDES

(30) Priorität: 03.11.2011 EP 11187666
(43) Veröffentlichungstag der Anmeldung: 10.09.2014
(73) Patentinhaber: Bayer Intellectual Property GmbH, 40789 Monheim (DE)
(72) Erfinder: VAN ALMSICK, Andreas, 61184 Karben (DE); AHRENS, Hartmut, 63329 Egelsbach (DE); DIETRICH, Hansjörg, 65835 Liederbach am Taunus (DE); HÄUSER-HAHN, Isolde, 51375 Leverkusen (DE); HEINEMANN, Ines, 65719 Hofheim (DE); GATZWEILER, Elmar, 61231 Bad Nauheim (DE); ROSINGER, Christopher, 65719 Hofheim (DE)
(74) Vertreter: BIP Patents
(86) Internationale Anmeldenummer: PCT/EP2012/071384
(87) Internationale Veröffentlichungsnummer: WO 2013/064459

(56) Entgegenhaltungen:
- EP-A1- 0 049 071
- EP-A2- 0 173 657
- WO-A1-2004/101532
- WO-A1-2007/115403
- WO-A1-2011/035874
- DD-A1- 224 203
- DE-A1- 19 700 019
- DE-A1- 19 700 096
- FR-A1- 2 407 208
- JP-A- 11 043 480

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus WO2003/010143 A1 und WO2003/010153 A1 sind N-(Tetrazol-5-yl)- und N-(Triazol-5-yl)benzamide und ihre pharmakologische Wirkung bekannt. Aus WO 2011/035874 A1 sind N-(1,2,5-Oxadiazol-3-yl)benzamide als Herbizide bekannt. Aus der prioritätsälteren, nicht vorveröffentlichen EP10174893 sind bestimmte N-(Tetrazol-5-yl)- und N-(Triazol-5-yl)benzamide und -nicotinamide als Herbizide bekannt.

Die herbizide Wirksamkeit und/oder die Kulturpflanzenverträglichkeit der in diesen Schriften genannten Verbindungen ist jedoch nicht immer ausreichend.

Aufgabe der vorliegenden Erfindung war die Bereitstellung von herbizid wirksamen Verbindungen mit gegenüber den aus dem Stand der Technik bekannten Verbindungen verbesserten Eigenschaften.

Es wurde nun gefunden, dass Benzamide, die in 3-Position des Phenylrings durch bestimmte Oximetherreste subsituiert sind, als Herbizide besonders gut geeignet sind.

Ein Gegenstand der vorliegenden Erfindung sind somit Benzamide der Formel (I) oder deren Salze 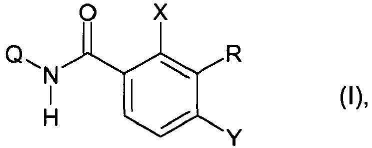 worin die Substituenten folgende Bedeutungen haben
Q bedeutet einen Rest Q1, Q2, Q3 oder Q4, 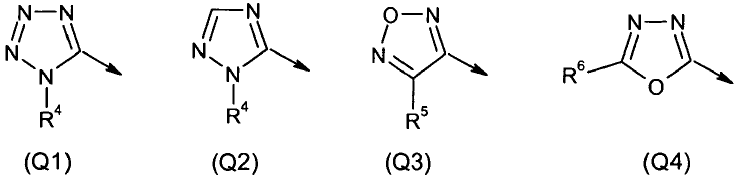
R bedeutet -CH=N-OR¹, -CH₂-O-N=CR²R³,
X bedeutet Nitro, Halogen, Cyano, Formyl, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR⁷, COOR⁷, OCOOR⁸, NR⁷COOR⁸, C(O)N(R⁷)₂, NR⁷C(O)N(R⁷)₂, OC(O)N(R⁷)₂, C(O)NR⁷OR⁷, OR⁷, OCOR⁷, OSO₂R⁸, S(O)ₙR⁸, SO₂OR⁷, SO₂N(R⁷)₂, NR⁷SO₂R⁸, NR⁷OR⁷, (C₁-C₆)-Alkyl-S(O)ₙR⁸, (C₁-C₆)-Alkyl-OR⁷, (C₁-C₆)-Alkyl-OCOR⁷, (C₁-C₆)-Alkyl-OSO₂R⁸, (C₁-C₆)-Alkyl-CO₂R⁷, (C₁-C₆)-Alkyl-SO₂OR⁸, (C₁-C₆)-Alkyl-CON(R⁷)₂, (C₁-C₆)-Alkyl-SO₂N(R⁷)₂, (C₁-C₆)-Alkyl-NR⁷COR⁷, (C₁-C₆)-Alkyl-NR⁷SO₂R⁸, N(R⁷)₂, P(O)(OR⁹)₂, CH₂P(O)(OR⁹)₂, Heteroaryl, Heterocyclyl, Phenyl, (C₁-C₆)-Alkyl-Heteroaryl oder (C₁-C₆)-Alkyl-Heterocyclyl, wobei die letzten fünf letztgenannten Reste jeweils durch s Reste aus der Gruppe bestehend aus Halogen, Nitro, Cyano, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Y bedeutet Nitro, Halogen, Cyano, Formyl, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR⁷, COOR⁷, OCOOR⁸, NR¹COOR⁸, C(O)N(R⁷)₂, NR⁷C(O)N(R⁷)₂, OC(O)N(R⁷)₂, C(O)NR⁷OR⁷, OR⁷, OSO₂R⁸, S(O)ₙR⁸, SO₂OR⁸, SO₂N(R⁷)₂, NR⁷SO₂R⁸, NR⁷COR⁷, (C₁-C₆)-Alkyl-S(O)ₙR⁸, (C₁-C₆)-Alkyl-OR⁷, (C₁-C₆)-Alkyl-OCOR⁷, (C₁-C₆)-Alkyl-OSO₂R⁸, (C₁-C₆)-Alkyl-CO₂R⁷, (C₁-C₆)-Alkyl-SO₂OR⁷, (C₁-C₆)-Alkyl-CON(R⁷)₂, (C₁-C₆)-Alkyl-SO₂N(R⁷)₂, (C₁-C₆)-Alkyl-NR⁷COR⁷, (C₁-C₆)-Alkyl-NR⁷SO₂R⁸, N(R⁷)₂, P(O)(OR⁹)₂, Heteroaryl, Heterocyclyl oder Phenyl, wobei die drei letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₃-C₆)-Cycloalkyl-(C₁-C₆)-alkyl, wobei die letzten sechs Reste jeweils durch s Reste aus der Gruppe bestehend aus Cyano, Halogen, Nitro, Rhodano, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ und (C₁-C₄)-Alkoxy-(C₂-C₆)-alkoxycarbonyl substituiert sind,
   oder R¹ bedeutet Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR¹⁰-Heteroaryl, (C₁-C₆)-Alkyl-NR¹⁰-Heterocyclyl, wobei die zehn letztgenannten Reste jeweils durch s Reste aus der Gruppe bestehend aus Cyano, Halogen, Nitro, Rhodano, (C₁-C₆)-Alkyl, Halogen-(C₁-C₆)-Alkyl, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ und (C₁-C₄)-Alkoxy-(C₂-C₆)-alkoxycarbonyl substituiert sind, und wobei Heterocyclyl n Oxogruppen trägt,
R² und R³ bedeuten unabhängig voneinander jeweils Wasserstoff, (C₁-C₆)-Alkyl, Halogen-(C₁-C₆)-alkyl, (C₂-C₆)-Alkenyl, Ha!ogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, (C₁-C₆)-Alkoxy-(C₁-C₆)-alkyl, Halogen-(C₁-C₆)-Alkoxy-(C₁-C₆)-alkyl Phenyl, Heteroaryl oder Heterocyclyl, wobei die drei letztgenannten Reste jeweils durch s Reste aus der Gruppe bestehend aus Cyano, Halogen, Nitro, Rhodano, (C₁-C₆)-Alkyl, Halogen-(C₁-C₆)-Alkyl, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹. CO₂R¹⁰, COSR¹¹, CON(R¹⁰)₂ und (C₁-C₄)-Alkoxy-(C₂-C₆)-alkoxycarbonyl substituiert sind, und wobei Heterocyclyl n Oxogruppen trägt,
   oder R² und R³ bilden gemeinsam mit dem Atom, an das sie gebunden sind, einen 5-bis 6-gliedrigen ungesättigten, teilgesättigten oder gesättigten Ring, der neben Kohlenstoffatomen jeweils n Sauerstoff- und Schwefelatome enthält,
R⁴ bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, wobei die sechs letztgenannten Reste jeweils durch s Reste aus der Gruppe Nitro, Cyano, SiR⁹₃, PO(OR⁹)₃, S(O)ₙ-(C₁-C₆)-Alkyl, (C₁-C₆)-Alkoxy, Halogen-(C₁-C₆)-alkoxy, N(R⁷)₂, COR⁷, COOR⁷, OCOR⁷, OCO₂R⁸, NR⁷COR⁷, NR⁷SO₂R⁸, (C₃-C₆)-Cycloalkyl, Heteroaryl, Heterocyclyl, Phenyl, W-Heteroaryl, W-Heterocyclyl, W-Phenyl oder W-Benzyl substituiert sind, wobei die sieben letztgenannten Reste selber wieder durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiert sind, und wobei Heterocyclyl n Oxogruppen trägt,
   oder R⁴ bedeutet (C₃-C₇)-Cycloalkyl, Heteroaryl, Heterocyclyl oder Phenyl, wobei die vier letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiert sind,
R⁵ bedeutet Wasserstoff, (C₁-C₆)-Alkyl, Halogen-(C₁-C₆)-alkyl, (C₂-C₆)-Alkenyl, (C₂-C₆)-Halogenalkenyl, (C₂-C₆)-Alkinyl, (C₂-C₆)-Halogenalkinyl, (C₃-C₇)-cyclo-Alkyl, (C₁-C₆)-Alkoxy, Halogen-(C₁-C₆)-alkoxy, (C₂-C₆)-Alkenyloxy, (C₂-C₆)-Alkinyloxy, Cyano, Nitro, Methylsulfenyl, Methylsulfinyl, Methylsulfonyl, Acetylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder
   jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiertes Heteroaryl, Heterocyclyl oder Phenyl und wobei Heterocyclyl n Oxogruppen trägt,
R⁶ bedeutet Wasserstoff, (C₁-C₆)-Alkyl, R⁷O-(C₁-C₆)-Alkyl, CH₂R¹², (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, OR⁷, NHR⁷, Methoxycarbonyl, Ethoxycarbonyl, Methoxycarbonylmethyl, Ethoxycarbonylmethyl, Methylcarbonyl, Trifluormethylcarbonyl, Dimethylamino, Acetylamino, Methylsulfenyl, Methylsulfinyl, Methylsulfonyl oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes Heteroaryl, Heterocyclyl, Benzyl oder Phenyl, wobei Heterocyclyl n Oxogruppen trägt,
R⁷ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR¹⁰-Heteroaryl, (C₁-C₆)-Alkyl-NR¹⁰-Heterocyclyl wobei die 16 letztgenannten Reste jeweils durch s Reste aus der Gruppe bestehend aus Cyano, Halogen, Nitro, Rhodano, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ und (C₁-C₄)-Alkoxy-(C₂-C₆)-alkoxycarbonyl substituiert sind, und wobei Heterocyclyl n Oxogruppen trägt,
R⁸ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR¹⁰-Heteroaryl, (C₁-C₆)-Alkyl-NR¹⁰-Heterocyclyl, wobei die 16 letztgenannten Reste jeweils durch s Reste aus der Gruppe bestehend aus Cyano, Halogen, Nitro, Rhodano, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ und (C₁-C₄)-Alkoxy-(C₂-C₆)-alkoxycarbonyl substituiert sind, und wobei Heterocyclyl n Oxogruppen trägt,
R⁹ bedeutet Methyl oder Ethyl,
R¹⁰ bedeutet Wasserstoff, (C₁-C₆)-Alkyl, Halo-(C₁-C₆)-alkyl (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R¹¹ bedeutet (C₁-C₆)-Alkyl, (C₂-C₆)-Alkenyl oder (C₂-C₆)-Alkinyl,
R¹² bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₁-C₆)-Alkoxy, (C₃-C₆)-Cycloalkyl oder jeweils durch s Reste aus der Gruppe bestehend aus Methyl, Ethyl, Methoxy, Trifluormethyl und Halogen substituiertes Heteroaryl, Heterocyclyl oder Phenyl,
R¹³ bedeutet (C₁-C₆)-Alkyl, (C₂-C₆)-Alkenyl oder (C₂-C₆)-Alkinyl, Phenyl
W bedeutet O, S, oder NR¹³,
n bedeutet 0, 1 oder 2,
s bedeutet 0, 1, 2 oder 3.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Halogen steht für Fluor, Chlor, Brom oder Iod.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl,

Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1 H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, daß diese Gruppe durch ein oder mehrere gleiche oder verschiedene der genannten Reste substituiert ist. Analoges gilt für den Aufbau von Ringsystemen durch verschiedene Atome und Elemente. Dabei sollen solche Verbindungen vom Anspruchsbegehren ausgenommen sein, von denen der Fachmann weiß, dass sie unter Normalbedingungen chemisch instabil sind.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind. Die erfindungsgemäßen Verbindungen können auf Grund der Oximether-Struktur auch als geometrische Isomere (E-/Z-Isomere) auftreten. Die Erfindung betrifft auch alle E-/Z-Isornere und deren Gemische, die von der allgemeinen Formel (I) umfasst, jedoch nicht spezifisch definiert sind.

Die Verbindungen der Formel (I) können Salze bilden. Salzbildung kann durch Einwirkung einer Base auf solche Verbindungen der Formel (I) erfolgen, die ein acides Wasserstoffatom tragen, z.B. im Falle dass R¹ eine COOH-Gruppe oder eine Sulfonamid-Gruppe -NHSO₂- enthält. Geeignete Basen sind beispielsweise organische Amine , wie Trialkylamine, Morpholin, Piperidin oder Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R"R"']⁺, worin R bis R"' jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

Bevorzugt sind Verbindungen der allgemeinen Formel (I), worin
Q bedeutet einen Rest Q1, Q2, Q3 oder Q4, 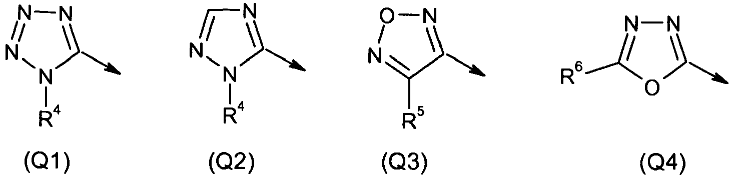
R bedeutet -CH=N-OR¹, -CH₂-O-N=CR²R³,
X bedeutet Nitro, Halogen, Cyano, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, OR⁷, S(O)ₙR⁸, SO₂N(R⁷)₂, NR⁷SO₂R⁸, (C₁-C₆)-Alkyl-S(O)ₙR⁸, (C₁-C₆)-Alkyl-OR⁷, (C₁-C₆)-Alkyl-CO₂R⁷, (C₁-C₆)-Alkyl-CON(R⁷)₂, (C₁-C₆)-Alkyl-SO₂N(R⁷)₂, (C₁-C₆)-Alkyl-NR⁷COR⁷, (C₁-C₆)-Alkyl-NR⁷SO₂R⁸, N(R⁷)₂, P(O)(OR⁹)₂, CH₂P(O)(OR⁹)₂, Heteroaryl, Heterocyclyl, Phenyl, (C₁-C₆)-Alkyl-Heteroaryl oder (C₁-C₆)-Alkyl-Heterocyclyl, wobei die fünf letztgenannten Reste jeweils durch s Reste aus der Gruppe bestehend aus Halogen, Nitro, Cyano, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Y bedeutet Nitro, Halogen, Cyano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, OR⁷, OSO₂R⁸, S(O)ₙR⁸, SO₂OR⁸, SO₂N(R⁷)₂, NR⁷SO₂R⁸, N(R⁷)₂, P(O)(OR⁹)₂, Heteroaryl, Heterocyclyl oder Phenyl, wobei die drei letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, wobei die fünf letztgenannten Reste jeweils durch s Reste aus der Gruppe bestehend aus Cyano, Halogen, Nitro, OR¹⁰, S(O)ₙR¹¹, COR¹⁰, OCOR¹⁰, NR¹⁰COR¹⁰, NR¹⁰SO₂R¹¹, CO₂R¹⁰, CON(R¹⁰)₂ und (C₁-C₄)-Alkoxy-(C₂-C₆)-alkoxycarbonyl substituiert sind, oder R¹ bedeutet Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR¹⁰-Heteroaryl oder (C₁-C₆)-Alkyl-NR¹⁰-Heterocyclyl, wobei die zehn letztgenannten Reste jeweils durch s Reste aus der Gruppe bestehend aus Cyano, Halogen, Nitro, Rhodano, (C₁-C₆)-Alkyl, Halogen-(C₁-C₆)-Alkyl, OR¹⁰, S(O)ₙR¹¹, N(R¹⁰)₂, COR¹⁰, OCOR¹⁰, NR¹⁰COR¹⁰, NR¹⁰SO₂R¹¹, CO₂R¹⁰, CON(R¹⁰)₂ und (C₁-C₄)-Alkoxy-(C₂-C₆)-alkoxycarbonyl substituiert sind, und wobei Heterocyclyl n Oxogruppen trägt,
R² und R³ bedeuten unabhängig voneinander jeweils Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkyl-(C₁-C₆)-alkyl, (C₁-C₆)-Alkoxy-(C₁-C₆)-alkyl, Halogen-(C₁-C₆)-Alkoxy-(C₁-C₆)-alkyl, Phenyl, Heteroaryl oder Heterocyclyl, wobei die drei letztgenannten Reste jeweils durch s Reste aus der Gruppe bestehend aus Cyano, Halogen, Nitro, (C₁-C₆)-Alkyl, Halogen-(C₁-C₆)-Alkyl, OR¹⁰, S(O)ₙR¹¹, N(R¹⁰)₂, COR¹⁰, NR¹⁰COR¹⁰, NR¹⁰SO₂R¹¹, CO₂R¹⁰, CON(R¹⁰)₂ und (C₁-C₄)-Alkoxy-(C₂-C₆)-alkoxycarbonyl substituiert sind, und wobei Heterocyclyl n Oxogruppen trägt,
   oder R² und R³ bilden gemeinsam mit dem Atom, an das sie gebunden sind, einen 5-bis 6-gliedrigen ungesättigten, teilgesättigten oder gesättigten Ring, der neben Kohlenstoffatomen jeweils n Sauerstoff- und Schwefelatome enthält,
R⁴ bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, wobei die sechs letztgenannten Reste jeweils durch s Reste aus der Gruppe bestehend aus Nitro, Cyano, S(O)ₙ-(C₁-C₆)-Alkyl, (C₁-C₆)-Alkoxy, Halogen-(C₁-C₆)-alkoxy, COOR⁷, NR⁷COR⁷, NR⁷SO₂R⁸, (C₃-C₆)-Cycloalkyl, Heteroaryl, Heterocyclyl, Phenyl, W-Heteroaryl, W-Heterocyclyl, W-Phenyl und W-Benzyl substituiert sind, wobei die 7 letztgenannten Reste selber wieder jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiert sind, und wobei Heterocyclyl n Oxogruppen trägt, substituiert sind,
   oder R⁴ bedeutet (C₃-C₇)-Cycloalkyl, Heteroaryl, Heterocyclyl oder Phenyl, wobei die vier letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiert sind,
R⁵ bedeutet Wasserstoff, (C₁-C₆)-Alkyl, Halogen-(C₁-C₆)-alkyl, (C₂-C₆)-Alkenyl, (C₂-C₆)-Halogenalkenyl, (C₂-C₆)-Alkinyl, (C₂-C₆)-Halogenalkinyl, (C₃-C₇)-cyclo-Alkyl, (C₁-C₆)-Alkoxy, Halogen-(C₁-C₆)-alkoxy, (C₂-C₆)-Alkenyloxy, (C₂-C₆)-Alkinyloxy, Cyano, Nitro, Methylsulfenyl, Methylsulfinyl, Methylsulfonyl, Acetylamino, Methoxycarbonyl, Methoxycarbonylmethyl, Methylcarbonyl, Trifluormethylcarbonyl, Halogen, Amino, Aminocarbonyl, Methylaminocarbonyl, Dimethylaminocarbonyl, Methoxymethyl, oder
   jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiertes Heteroaryl, Heterocyclyl oder Phenyl, und wobei Heterocyclyl n Oxogruppen trägt,
R⁶ bedeutet Wasserstoff, (C₁-C₆)-Alkyl, R⁷O-(C₁-C₆)-Alkyl, CH₂R¹², (C₃-C₇)-Cycloalkyl, Halogen-(C₁-C₆)-alkyl, (C₂-C₆)-Alkenyl, (C₂-C₆)-Alkinyl, OR⁷, NHR⁷, Methoxycarbonyl, Methoxycarbonylmethyl, Methylcarbonyl, Trifluormethylcarbonyl, Dimethylamino, Acetylamino, Methylsulfenyl, Methylsulfinyl, Methylsulfonyl oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiertes Heteroaryl, Heterocyclyl, Benzyl oder Phenyl, und wobei Heterocyclyl n Oxogruppen trägt,
R⁷ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₃-C₆)-Cycloalkyl-(C₁-C₆)-alkyl, Phenyl, Heteroaryl, Heterocyclyl, wobei die neun letztgenannten Reste jeweils durch s Reste aus der Gruppe bestehend aus Cyano, Halogen, Nitro, OR¹⁰, S(O)ₙR¹¹, N(R¹⁰)₂, NR¹⁰SO₂R¹¹, CO₂R¹⁰, CON(R¹⁰)₂ und (C₁-C₄)-Alkoxy-(C₂-C₆)-alkoxycarbonyl substituiert sind, und wobei Heterocyclyl n Oxogruppen trägt,
R⁸ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₃-C₆)-Cycloalkyl-(C₁-C₆)-alkyl, Phenyl, Heteroaryl, Heterocyclyl, wobei die neun letztgenannten Reste jeweils durch s Reste aus der Gruppe bestehend aus Cyano, Halogen, Nitro, OR¹⁰, S(O)ₙR¹¹, N(R¹⁰)₂, NR¹⁰SO₂R¹¹, CO₂R¹⁰, CON(R¹⁰)₂ und (C₁-C₄)-Alkoxy-(C₂-C₆)-alkoxycarbonyl substituiert sind, und wobei Heterocyclyl n Oxogruppen trägt,
R⁹ bedeutet Methyl oder Ethyl,
R¹⁰ bedeutet Wasserstoff, (C₁-C₆)-Alkyl, Halo-(C₁-C₆)-alkyl (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R¹¹ bedeutet (C₁-C₆)-Alkyl, (C₂-C₆)-Alkenyl oder (C₂-C₆)-Alkinyl,
R¹² bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Trifluormethylcarbonyl, Aminocarbonyl, Methylaminocarbonyl, Dimethylaminocarbonyl, (C₁-C₆)-Alkoxy, (C₃-C₆)-Cycloalkyl oder jeweils durch s Reste aus der Gruppe bestehend aus Methyl, Methoxy, Trifluormethyl und Halogen substituiertes Heteroaryl, Heterocyclyl oder Phenyl,
R¹³ bedeutet (C₁-C₆)-Alkyl,
W bedeutet O, S oder NR¹³,
n bedeutet 0, 1 oder 2,
s bedeutet 0, 1, 2 oder 3.

Besonders bevorzugt sind Verbindungen der allgemeinen Formel (I), worin
Q bedeutet einen Rest Q1 oder Q2, 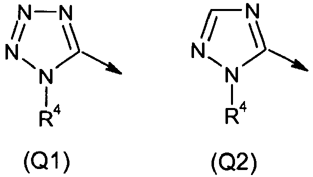
R bedeutet -CH=N-OR¹, -CH₂-O-N=CR²R³,
X bedeutet Nitro, Halogen, (C₁-C₆)-Alkyl, Halogen-(C₁-C₆)-alkyl, S(O)ₙR⁸, (C₁-C₆)-Alkyl-S(O)ₙR⁸, (C₁-C₆)-Alkyl-OR⁷, (C₁-C₆)-Alkyl-SO₂N(R⁷)₂ oder (C₁-C₆)-Alkyl-NR⁷SO₂R⁸,
Y bedeutet Nitro, Halogen, Halogen-(C₁-C₆)-alkyl oder S(O)ₙR⁸,
R¹ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl, wobei die fünf letztgenannten Reste jeweils durch s Reste aus der Gruppe bestehend aus Cyano, Halogen, OR¹⁰ und S(O)ₙR¹¹ substituiert sind,
R² und R³ bedeuten unabhängig voneinander jeweils Wasserstoff, (C₁-C₆)-Alkyl, Halogen-(C₁-C₆)-alkyl, (C₂-C₆)-Alkenyl, (C₂-C₆)-Alkinyl, (C₁-C₆)-Alkoxy-(C₁-C₆)-alkyl oder Halogen-(C₁-C₆)-Alkoxy-(C₁-C₆)-alkyl,
R⁴ bedeutet (C₁-C₆)-Alkyl, (C₂-C₆)-Alkenyl oder (C₂-C₆)-Alkinyl, wobei diese drei Reste jeweils durch s Reste (C₁-C₆)-Alkoxy substituiert sind,
R⁵ bedeutet (C₁-C₆)-Alkyl, (C₂-C₆)-Alkenyl, (C₂-C₆)-Alkinyl, (C₃-C₇)-cyclo-Alkyl, (C₁-C₆)-Alkoxy, (C₂-C₆)-Alkenyloxy, (C₂-C₆)-Alkinyloxy, Acetylamino, Halogen oder Methoxymethyl,
R⁶ bedeutet (C₁-C₆)-Alkyl, R⁷O-(C₁-C₆)-Alkyl, CH₂R¹², (C₃-C₇)-Cycloalkyl, (C₂-C₆)-Alkenyl oder (C₂-C₆)-Alkinyl,
R⁷ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁸ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkenyl oder (C₃-C₆)-Cycloalkyl-(C₁-C₆)-alkyl,
R⁹ bedeutet Methyl oder Ethyl,
R¹⁰ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R¹¹ bedeutet (C₁-C₆)-Alkyl, (C₂-C₆)-Alkenyl oder (C₂-C₆)-Alkinyl,
R¹² bedeutet Acetoxy, Acetamido oder (C₃-C₆)-Cycloalkyl,
R¹³ bedeutet (C₁-C₆)-Alkyl,
W bedeutet O, S oder NR¹³,
n bedeutet 0, 1 oder 2,
s bedeutet 0, 1, 2 oder 3.

Erfindungsgemäße Verbindungen, in denen Q für Q1 oder Q2 steht, können beispielsweise nach der in Schema 1 angegebenen Methode durch basenkatalysierte Umsetzung eines Benzoesäurechlorids (II) mit einem 5-Amino-1-H-1,2,4-triazol, bzw. 5-Amino-1H-tetrazol (III) hergestellt werden:

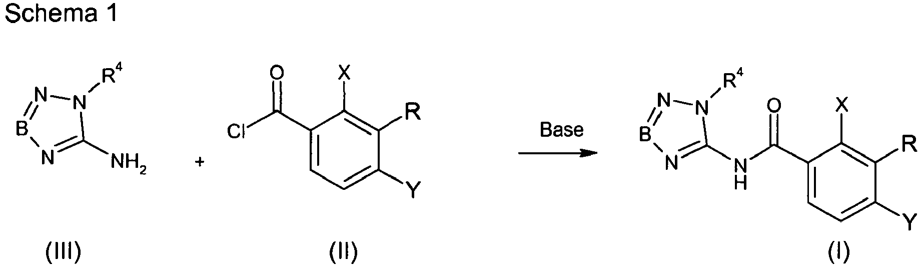

Darin steht B für CH oder N. Die Benzoesäurechloride der Formel (II) beziehungsweise die ihnen zugrunde liegenden Benzoesäuren sind grundsätzlich bekannt und können beispielsweise gemäß den in WO 98/29392, WO98/29384 und JP 11043480 beschriebenen Methoden hergestellt werden.

Erfindungsgemäße Verbindungen, in denen Q für Q1 oder Q2 steht, können auch nach der in Schema 2 angegebenen Methode durch Umsetzung einer Benzoesäure der Formel (IV) mit einem 5-Amino-1-H-1,2,4-triazol, bzw. 5-Amino-1H-tetrazol (III) hergestellt werden:

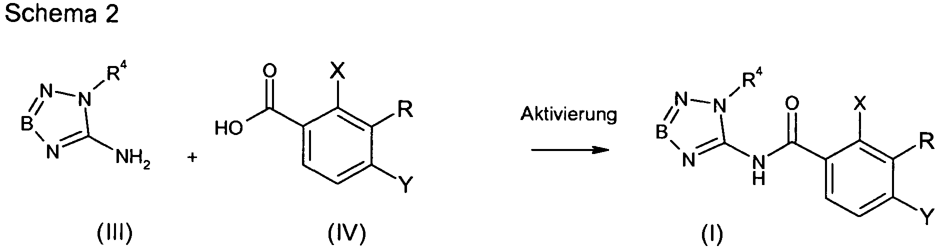

Für die Aktivierung können wasserentziehende Reagenzien, die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc. eingesetzt werden.

Erfindungsgemäße Verbindungen, in denen Q für Q1 oder Q2 steht, können auch nach der in Schema 3 angegebenen Methode durch Umsetzung eines N-(1 H-1,2,4-triazol-5-yl)benzamides, N-(1 H-tetrazol-5-yl)benzamides, N-(1 H-1,2,4-triazol-5-yl)nicotinamide oder N-(1 H-tetrazol-5-yl)nicotinamide hergestellt werden:

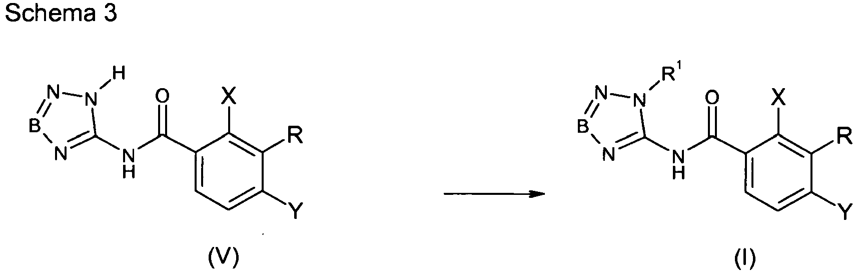

Für diese in Schema 3 genannte Reaktion können z. B. Alkylierungsmittel wie z. B. Alkylhalogenide, -sulfonate oder Dialkylsulfate in Gegenwart einer Base eingesetzt werden.

Es kann zweckmäßig sein, Reaktionsschritte in ihrer Reihenfolge zu ändern. So sind Benzoesäuren, die ein Sulfoxid tragen, nicht ohne weiteres in ihre Säurechloride zu überführen. Hier bietet sich an, zunächst auf Thioether-Stufe das Amid zu herzustellen und danach den Thioether zum Sulfoxid zu oxidieren.

Die 5-Amino-1H-tetrazole der Formel (III) sind entweder käuflich erhältlich oder können analog zu literaturbekannten Methoden hergestellt werden. Beispielsweise können substituierte 5-Aminotetrazole nach der in Journal of the American Chemical Society (1954), 76, 923-924 beschriebenen Methode aus Amino-tetrazol hergestellt werden:

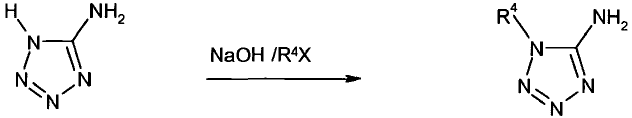

In der vorstehend genannten Reaktion bedeutet X eine Abgangsgruppe wie Iod. Substituierte 5-Aminotetrazole können zum Beispiel auch wie in Journal of the American Chemical Society (1954) 76, 88-89 beschrieben, synthetisiert werden:

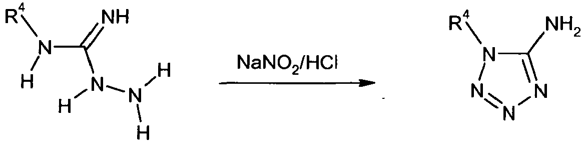

Die 5-Amino-1H-triazole der Formel (III) sind entweder käuflich erhältlich oder können analog zu literaturbekannten Methoden hergestellt werden. Beispielsweise können substituierte 5-Aminotriazole nach der in Zeitschrift füer Chemie (1990), 30(12), 436-437 beschriebenen Methode aus Aminotriazol hergestellt werden:

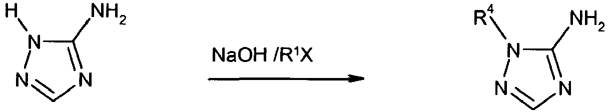

Substituierte 5-Aminotriazole können auch zum Beispiel wie in Chemische Berichte (1964), 97(2), 396-404 beschrieben, synthetisiert werden:

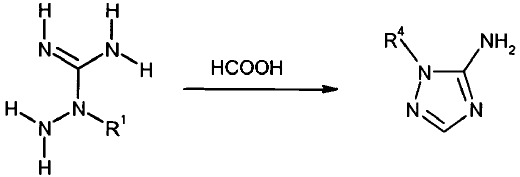

Substituierte 5-Aminotriazole können auch zum Beispiel wie in Angewandte Chemie (1963), 75, 918 beschrieben, synthetisiert werden:

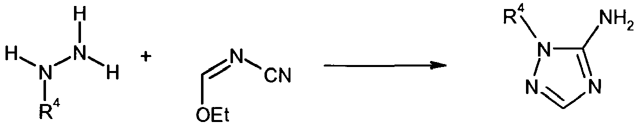

Erfindungsgemäße Verbindungen, in denen Q für Q3 steht, können beispielsweise nach der in Schema 4 angegebenen Methode durch basenkatalysierte Umsetzung eines Benzoesäurechlorids (II) mit einem 4-Amino-1,2,5-oxadiazol (VI) hergestellt werden:

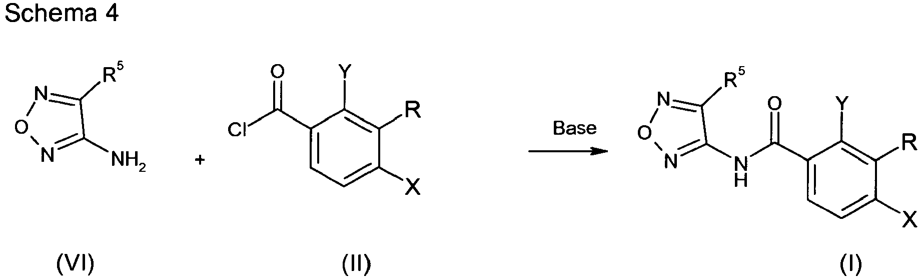

Erfindungsgemäße Verbindungen können auch nach der in Schema 5 angegebenen Methode durch Umsetzung einer Benzoesäure der Formel (IV) mit einem 4-Amino-1,2,5-oxadiazol (VI) hergestellt werden:

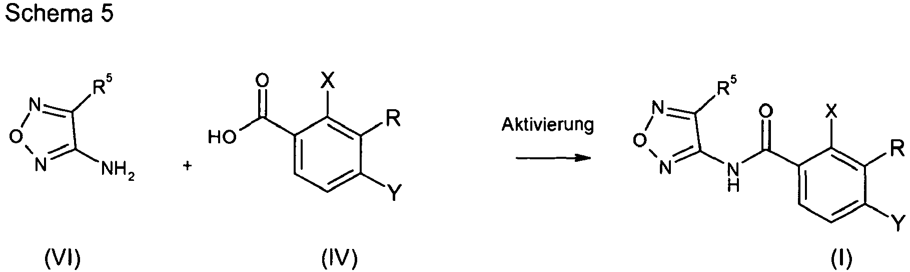

Für die Aktivierung können wasserentziehende Reagenzien die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc. eingesetzt werden.

Die 4-Amino-1,2,5-oxadiazole der Formel (VI) sind entweder käuflich erhältlich oder bekannt oder können analog zu literaturbekannten Methoden hergestellt werden. Beispielsweise können 3-Alkyl-4-amino-1,2,5-oxadiazole nach der in Russian Chemical Bulletin, Int. Ed., Vol. 54, No. 4, S. 1032-1037 (2005) beschriebenen Methode aus β-Ketoestern hergestellt werden:

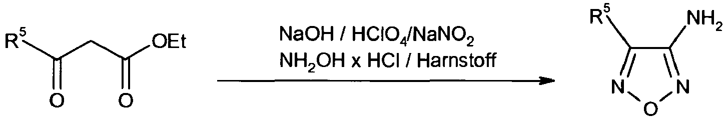

3-Aryl-4-amino-1,2,5-oxadiazole können zum Beispiel wie in Russian Chemical Bulletin, 54(4), 1057-1059, (2005) oder Indian Journal of Chemistry, Section B: Organic Chemistry Including Medicinal Chemistry, 26B(7), 690-2, (1987) beschrieben, synthetisiert werden:

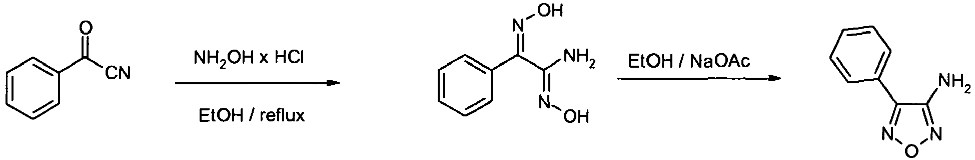

3-Amino-4-Halogen-1,2,5-oxadiazole können beispielsweise nach der in Heteroatom Chemistry 15(3), 199-207 (2004) beschrieben Methode aus dem käuflich erhältlichen 3,4-Diamino-1,2,5-oxadiazol durch eine Sandmeyer-Reaktion hergestellt werden:

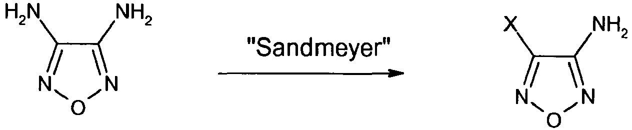

Nucleophile Reste R⁵ können wie in Journal of Chemical Research, Synopses, (6), 190, 1985 oder in oder Izvestiya Akademii Nauk SSSR, Seriya Khimicheskaya, (9), 2086-8, 1986 oder in Russian Chemical Bulletin (Translation of Izvestiya Akademii Nauk, Seriya Khimicheskaya), 53(3), 596-614, 2004 beschrieben, durch Substitution der Austrittsgruppe L in 3-Amino-1,2,5-oxadiazolen eingeführt werden:

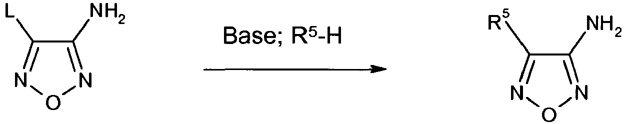

Erfindungsgemäße Verbindungen, in denen Q für Q4 steht, können beispielsweise nach der in Schema 6 angegebenen Methode durch basenkatalysierte Umsetzung eines Benzoesäurechlorids (II) mit einem 2-Amino-1,3,4-oxadiazol (VII) hergestellt werden:

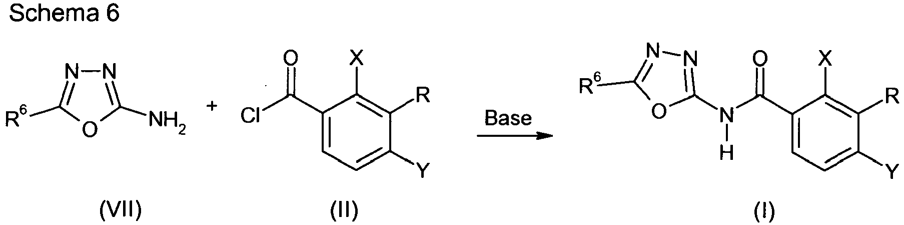

Erfindungsgemäße Verbindungen können auch nach der in Schema 7 angegebenen Methode durch Umsetzung einer Benzoesäure der Formel (IV) mit einem 2-Amino-1,3,4-oxadiazol (VII) hergestellt werden:

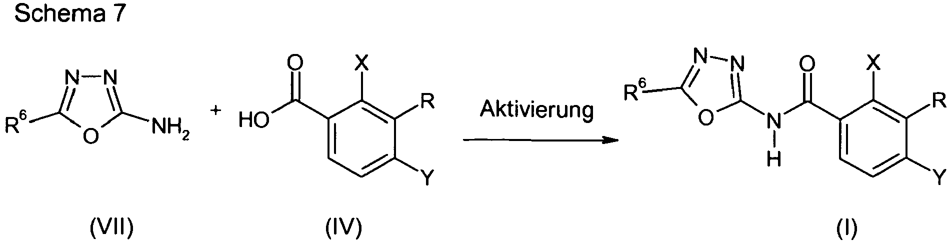

Für die Aktivierung können wasserentziehende Reagenzien, die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc. eingesetzt werden.

Erfindungsgemäße Verbindungen können auch nach der in Schema 8 angegebenen Methode durch Cyclisierung eine Verbindung der Formel VIII hergestellt werden:

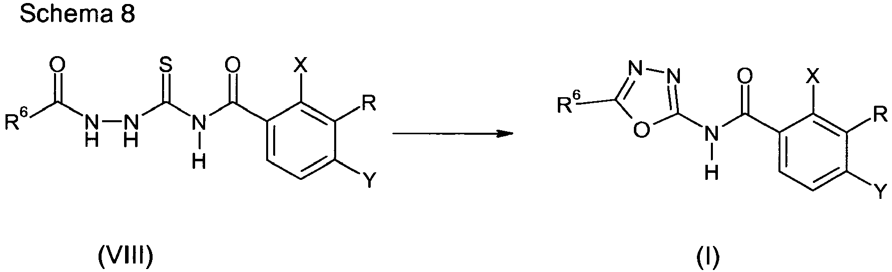

Die Cyclisierung kann, gemäß der in Synth. Commun. 31 (12), 1907-1912 (2001) oder der in Indian J. Chem., Section B: Organic Chemistry Including Medicinal Chemistry; Vol. 43 (10), 2170-2174 (2004) beschriebenen Methoden durchgeführt werden.

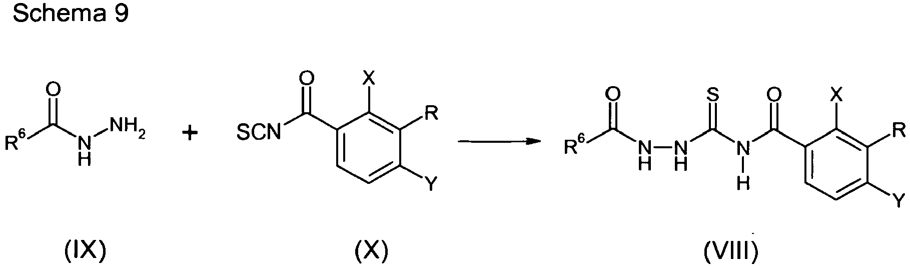

Die in Schema 8 eingesetzte Verbindung der Formel VIII kann durch Umsetzung eines Acylisocyanats der Formel X mit einem Hydrazid der Formel IX gemäß der von in Synth. Commun. 25(12), 1885-1892 (1995) beschriebenen Methode hergestellt werden. Es kann zweckmäßig sein, Reaktionsschritte in ihrer Reihenfolge zu ändern. So sind Benzoesäuren, die ein Sulfoxid tragen, nicht ohne weiteres in ihre Säurechloride zu überführen. Hier bietet sich an, zunächst auf Thioether-Stufe das Amid zu herzustellen und danach den Thioether zum Sulfoxid zu oxidieren.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer Struktur und der Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen. Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.
Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2009 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, Beflubutamid, Benazolin, Benazolin-ethyl, Bencarbazone, Benfluralin, Benfuresate, Bensulide, Bensulfuron, Bensulfuron-methyl, Bentazone, Benzfendizone, Benzobicyclon, Benzofenap, Benzofluor, Benzoylprop, Bicyclopyrone, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenacnatrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop, Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1 H-tetrazol-1-yl]-phenyl]-ethansulfonamid, F-7967, d. h. 3-[7-Chlor-5-fluor-2-(trifluormethyl)-1 H-benzimidazol-4-yl]-1-methyl-6-(trifluormethyl)pyrimidin-2,4(1H,3H)-dion, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, Glufosinate-ammonium, Glufosinate-P, Glufosinate-P-ammonium, Glufosinate-P-natrium, Glyphosate, Glyphosate-isopropylammonium, H-9201, d. h. O-(2,4-Dimethyl-6-nitrophenyl)-O-ethyl-isopropylphosphoramidothioat, Halosafen, Halosulfuron, Halosulfuron-methyl, Haloxyfop, Haloxyfop-P, Haloxyfop-ethoxyethyl, Haloxyfop-P-ethoxyethyl, Haloxyfopmethyl, Haloxyfop-P-methyl, Hexazinone, HW-02, d. h. 1-(Dimethoxyphosphoryl)-ethyl(2,4-dichlorphenoxy)acetat, Imazamethabenz, Imazamethabenz-methyl, Imazamox, Imazamox-ammonium, Imazapic, Imazapyr, Imazapyrisopropylammonium, Imazaquin, Imazaquin-ammonium, Imazethapyr, Imazethapyrammonium, Imazosulfuron, Inabenfide, Indanofan, Indaziflam, Indolessigsäure (IAA), 4-Indol-3-ylbuttersäure (IBA), lodosulfuron, lodosulfuron-methyl-natrium, loxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monosulfuron-ester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-Dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron, Pyrazosulfuron-ethyl, Pyrazoxyfen, Pyribambenz, Pyribambenz-isopropyl, Pyribambenz-propyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulcotrione, Sulfallate (CDEC), Sulfentrazone, Sulfometuron, Sulfometuron-methyl, Sulfosate (Glyphosate-trimesium), Sulfosulfuron, SYN-523, SYP-249, d. h. 1-Ethoxy-3-methyl-1-oxobut-3-en-2-yl-5-[2-chlor-4-(trifluormethyl)phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triafamon, Triallate, Triasulfuron, Triaziflam, Triazofenamide, Tribenuron, Tribenuronmethyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin, sowie die folgenden Verbindungen:

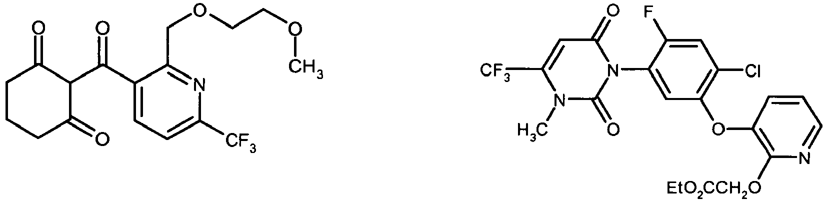

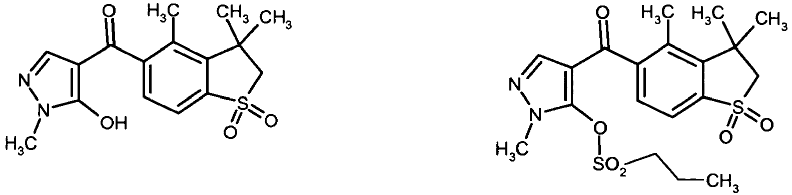

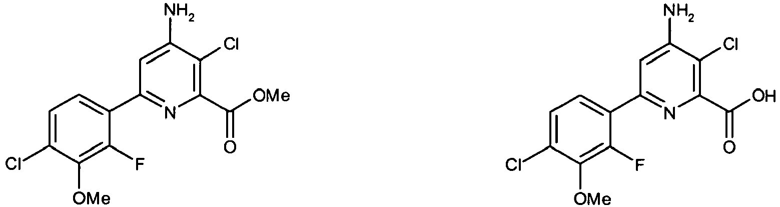

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### 1. Synthese von 2,4-Dichlor-N-(5-ethyl-1,3,4-oxadiazol-2-yl)-3-[(methoxyimino) methyl]benzamid (Tabellenbeispiel 4-040)

400 mg (1.61 mmol) 2,4-Dichlor-3-[(methoxyimino)methyl]benzoesäure und 228 mg (2.0 mmol) 5-Ethyl-1,3,4-oxadiazol-2-amin wurden in 3 ml Pyridin gelöst und mit 10 mg 4-Dimethylaminopyridin und einem Tropfen DMF versetzt. Anschließend wurden bei Raumtemperatur (RT) 255,83 mg (2.016 mmol) Oxalsäuredichlorid zugetropft und 4 h bei 70 °C gerührt. Der wieder abgekühlte Ansatz wurde danach mit 0.5 ml Wasser versetzt, ca. 30 min gerührt, mit einer gesättigten KHSO₄-Lösung sauer gestellt und drei mal mit jeweils 100 ml Essigsäureethylester extrahiert. Die vereinigten organischen Phasen wurden einmal mit 20 ml einer gesättigten NaHCO₃-Lösung gewaschen, über MgSO₄ getrocknet, abfiltriert und eingeengt. Der Rückstand wurde chromatographisch an einer präparativen HPLC gereinigt.
Ausbeute: 99,3 mg (18 %) als farblose Kristalle.
¹H-NMR (400 MHz, CDCl₃ δ, ppm) 8.21 (s,1 H), 7.63 (d,1 H), 7.42 (d,1 H), 4.03 (s,3H), 2.86 (q,2H), 1.38 (t,3H).

### 2. Synthese von 2-Chlor-N-(1-ethyl-1H-tetrazol-5-yl)-4-(methylsulfonyl)-3-{[(prop-2-in-1-yloxy)imino]methyl}benzamid (Tabellenbeispiel 1-057)

400 mg (1.15 mmol) 2-Chlor-4-(methylsulfonyl)-3-{[(prop-2-in-1-yloxy)imino]methyl}benzoesäure und 171.60 mg (1.44 mmol) 1-Ethyl-1H-tetrazol-5-amin wurden in 4 ml Pyridin gelöst und mit 10 mg 4-Dimethylaminopyridin und einem Tropfen DMF versetzt. Anschließend wurden bei RT 186,65 mg (1.441 mmol) Oxalsäuredichlorid zugetropft und 3 h bei 70 °C gerührt. Der wieder abgekühlte Ansatz wurde danach mit 0.5 ml Wasser versetzt, ca. 30 min gerührt, mit einer gesättigten KHSO₄-Lösung sauer gestellt und drei mal mit jeweils 100 ml Essigsäureethylester extrahiert. Die vereinigten organischen Phasen wurden einmal mit 20 ml einer gesättigten NaHCO₃-Lösung gewaschen, über MgSO₄ getrocknet, abfiltriert und eingeengt. Ausbeute: 351.60 mg (67 %) als oranger Feststoff.
¹H-NMR (400 MHz, CDCl₃ δ, ppm) 8.35 (s,1 H), 7.93 (d,1 H), 7.82 (d,1 H), 4.78 (d,2H), 4.30 (q,2H), 3.25 (s,3H), 2.49 (t,1 H), 1.48 (t,3H).

### 3. Synthese von 2-Chlor-4-(methylsulfonyl)-N-(1-methyl-1H-1,2,4-triazol-5-yl)-3-{[(propan-2-ylidenamino)oxy]methyl}benzamid (Tabellenbeispiel 6-017)

500 mg (1.56 mmol) 2-Chlor-4-(methylsulfonyl)-3-{[(propan-2-ylidenamino)oxy] methyl}benzoesäure und 395 mg (1.34 mmol) 1-Methyl-1H-1,2,4-triazol-5-amin wurden in 2,5 ml Pyridin gelöst und mit einer 10 mg 4-Dimethylaminopyridin und einem Tropfen DMF versetzt. Anschließend wurden bei RT 313 mg (2.58 mmol) Thionylchlorid zugetropft und 5 h bei 70 °C gerührt. Der wieder abgekühlte Ansatz wurde danach mit 0.5 ml Wasser versetzt, ca. 30 min gerührt, mit einer gesättigten KHSO₄-Lösung sauer gestellt und drei mal mit jeweils 100 ml Essigsäureethylester extrahiert. Die vereinigten organischen Phasen wurden einmal mit 20 ml einer gesättigten NaHCO₃-Lösung gewaschen, über MgSO₄ getrocknet, abfiltriert und eingeengt. Der Rückstand wurde chromatographisch an einer Kieselgelsäule gereinigt. Ausbeute: 153 mg (24 %) als farbloses Öl.
¹H-NMR (400 MHz, CDCl₃ δ, ppm) 8.14 (d,1H), 7.69 (d,1H), 7.27 (s,1H), 5.73 (s,2H), 3.89 (s,3H), 3.37 (s,3H), 1.84 (s,3H).

### 4. Synthese von 2,4-Dichlor-3-{(E)-[(cyclopropylmethoxy)imino]methyl}-N-(4-methyl-1,2,5-oxadiazol-3-yl)benzamid (Tabellenbeispiel 3-004)

400 mg (1.39 mmol) 2,4-Dichlor-3-{[(cyclopropylmethoxy)imino]methyl}benzoesäure und 14418 mg (1.39 mmol) 4-Methyl-1,2,5-oxadiazol-3-amin wurden in 10 ml CH₂Cl₂ gelöst und bei 10 - 20 °C mit 1.33 g (2.08 mmol) Propanphosphonsäureanhydrid versetzt und 1 h bei RT gerührt. Dann wurden 700 mg Triethylamin und 34,6 mg (0.278 mmol) DMAP zugegeben und 2 Tage bei RT gerührt. Der Ansatz wurde mit Wasser und zweimal mit 6 N Salzsäure gewaschen und die organische Phase anschließend über MgSO₄ getrocket und eingeengt. Der Rückstand wurde chromatographisch an einer Kieselgelsäule gereinigt (Heptan/Essigester 4:1). Ausbeute: 248,6 mg (48 %) als farbloser Feststoff.
¹H-NMR (400 MHz, CDCl₃ δ, ppm) 8.30 (s,1H), 8.19 (br,s,1H), 7.66 (d,1H), 7.51 (d,1 H), 4.06 (d,2H), 2.49 (s,3H), 1.25 (m,1 H), 0.61 (m,2H), 0.37 (m,2H).

Ganz besonders bevorzugt sind die in den Tabellen 1 bis 8 genannten Verbindungen der allgemeinen Formel (I), die analog der hier genannten Methoden erhältlich sind:
Die verwendeten Abkürzungen bedeuten:

| | | | | | | | |
|---|---|---|---|---|---|---|---|
| Et | = Ethyl | Me | = Methyl | n-Pr | = n-Propyl | i-Pr | = Isopropyl |
| c-Pr | = Cyclopropyl | Ph | = Phenyl | | | | |

**Tabelle 1: Erfindungsgemäße Verbindungen der Formel (I), worin Q für Q1, R für CH=N-OR¹ steht, und die anderen Reste die in Tabelle 1 angegebenen Bedeutungen haben**

| | | | | | |
|---|---|---|---|---|---|
| 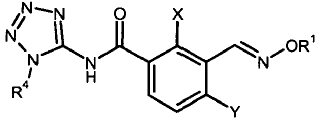 | | | | | |

| Nr. | R⁴ | X | Y | R¹ | Physikalische Daten (¹H-NMR) |
|---|---|---|---|---|---|
| 1-001 | Me | Cl | Cl | Me | (400 MHz, d⁶-DMSO δ, ppm) 11.88 (br,s, 1H), 8.36 (s,1H), 7.80 (d, 1H), 7.74 (d,1H), 4.00 (s,3H), 3.90 (s,3H) |
| 1-002 | Me | Cl | Cl | Et | (400 MHz, d⁶-DMSO δ, ppm) 11.88 (br,s,1H), 8.35 (s,1H), 7.80 (d,1H), 7.74 (d,1H), 4.20 (q,2H), 4.00 (s,3H), 1.28 (t,3H) |
| 1-003 | Me | Cl | Cl | nPr | |
| 1-004 | Me | Cl | Cl | CH₂cPr | (400 MHz, CDCl₃ δ, ppm) 8.20 (s,1H), 7.65 (d,1H), 7.38 (d,1H), 3.98 (d,2H), 3.88 (s,3H), 1.18 (m, 1H), 0.58 (m,2H), 0.32 (m,2H) |
| 1-005 | Me | Cl | Cl | CH₂CCH | (400 MHz, CDCl₃ δ ppm) 10.40 (br,s, 1H), 8.32 (s,1H), 7.68 (d,1H), 7.52 (d,1H), 4.83 (d,2H), 4.11 (s,3H), 2.53 (t,1H) |
| 1-006 | Me | Cl | Cl | CH₂CH=CH₂ | |
| 1-007 | Me | Cl | Cl | CH₂CH(Me)₂ | (400 MHz, d⁶-DMSO δ, ppm) 11.88 (br,s,1H), 8.38 (s,1H), 7.81 (d,1H), 7.73 (d,1H), 4.00 (s,3H), 3.94 (d,2H), 2.01 (m,1 H), 0.93 (d,6H) |
| 1-008 | Me | Cl | Cl | CH₂CH=C(Me)₂ | (400 MHz, CDCl₃ δ, ppm) 10.8 (br,s,1H), 8.25 (s,1H), 7.62 (d,1H), 7.28 (d,1H), 5.50 (t,1H), 4.75 (d,2H), 4.08 (s,3H), 1.82 (s,3H), 1.78 (s,3H) |
| 1-009 | Me | Cl | Cl | CH₂CH₂OMe | |
| 1-010 | Me | Cl | Cl | CH₂CN | |
| 1-011 | Me | Cl | Cl | CH₂CH₂SMe | |
| 1-012 | Me | Cl | Cl | CH₂CF₃ | |
| 1-013 | Me | Cl | Cl | CH₂CHF₂ | |
| 1-014 | Me | Cl | SO₂Me | Me | (400 MHz, CDCl₃ δ, ppm) 8.28 (s,1 H), 7.94 (d,1H), 7.80 (d,1H), 3.98 (s,3H), 3.84 (s,3H), 3.24 (s,3H) |
| 1-015 | Me | Cl | SO₂Me | Et | (400 MHz, CDCl₃ δ, ppm) 8.38 (s,1H), 8.10 (d,1H), 7.88 (d,1H), 4.26 (q,2H), 3.98 (s,3H), 3.28 (s,3H), 1.35 (t,3H) |
| 1-016 | Me | Cl | SO₂Me | nPr | |
| 1-017 | Me | Cl | SO₂Me | CH₂cPr | (400 MHz, CDCl₃ δ, ppm) 8.32 (s,1H), 7.95 (d,1 H), 7.80 (d,1 H), 3.99 (d,2H), 3.87 (s,3H), 3.23 (s,3H), 1.18 (m,3H), 0.58 (m,2H), 0.31 (m,2H) |
| 1-018 | Me | Cl | SO₂Me | CH₂CCH | (400 MHz, CDCl₃ δ, ppm) 8.35 (s,1H), 7.92 (d,1H), 7.82 (d,1H), 4.75 (d,2H), 3.85 (s,3H), 3.25 (s,3H), 2.49 (m,1H) |
| 1-019 | Me | Cl | SO₂Me | CH₂CH=CH₂ | |
| 1-020 | Me | Cl | SO₂Me | CH₂CH(Me)₂ | (400 MHz, CDCl₃ δ, ppm) 8.29 (s,1 H), 7.95 (d,1H), 7.79 (d,1H), 3.95 (d,2H), 3.25 (s,3H), 2.02 (m,1 H), 0.98 (d,6H) |
| 1-021 | Me | Cl | SO₂Me | CH₂CH=C(Me)₂ | |
| 1-022 | Me | Cl | SO₂Me | CH₂CH₂OMe | |
| 1-023 | Me | Cl | SO₂Me | CH₂CN | |
| 1-024 | Me | Cl | SO₂Me | CH₂CH₂SMe | |
| 1-025 | Me | Cl | SO₂Me | CH₂CF₃ | |
| 1-026 | Me | Cl | SO₂Me | CH₂CHF₂ | |
| 1-027 | Me | SO₂Me | Cl | Me | |
| 1-028 | Me | SO₂Me | Cl | Et | |
| 1-029 | Me | SO₂Me | Cl | nPr | |
| 1-030 | Me | SO₂Me | Cl | CH₂cPr | |
| 1-031 | Me | SO₂Me | Cl | CH₂CCH | |
| 1-032 | Me | SO₂Me | Cl | CH₂CH=CH₂ | |
| 1-033 | Me | SO₂Me | Cl | CH₂CH(Me)₂ | (400 MHz, CDCl₃ δ, ppm) 8.30 (s,1 H), 7.92 (d,1H), 7.79 (d,1H), 3.95 (s,3H), 3.93 (d,2H), 3.25 (s,3H), 2.20 (m,1H), 0.95 (d,6H) |
| 1-034 | Me | SO₂Me | Cl | CH₂CH=C(Me)₂ | |
| 1-035 | Me | SO₂Me | Cl | CH₂CH₂OMe | |
| 1-036 | Me | SO₂Me | Cl | CH₂CN | |
| 1-037 | Me | SO₂Me | Cl | CH₂CH₂SMe | |
| 1-038 | Me | SO₂Me | Cl | CH₂CF₃ | |
| 1-039 | Me | SO₂Me | Cl | CH₂CHF₂ | |
| 1-040 | Et | Cl | Cl | Me | (400 MHz, CDCl₃ δ, ppm) 11.45 (br,s,1 H), 8.28 (s,1 H), 7.60 (d,1 H), 7.42 (d,1H), 4.32 (q,2H), 4.02 (s,3H), 1.50 (t,3H) |
| 1-041 | Et | Cl | Cl | Et | (400 MHz, CDCl₃ δ, ppm) 8.18 (s,1H), 7.68 (d,1H), 7.40 (d,1H), 4.32 (q,2H), 4.28 (q,2H), 1.48 (t,3H), 1.38 (t,3H) |
| 1-042 | Et | Cl | Cl | nPr | |
| 1-043 | Et | Cl | Cl | CH₂cPr | (400 MHz, CDCl₃ δ, ppm) 11.7 (br,s,1H), 8.22 (s,1H), 7.58 (d,1H), 7.40 (d,1 H), 4.32 (q,2H), 4.02 (d,2H), 1.49 (t,3H), 1.10 (m,1 H), 0.60 (m,2H), 0.33 (m,2H) |
| 1-044 | Et | Cl | Cl | CH₂CCH | (400 MHz, CDCl₃ δ, ppm) 8.24 (s,1H), 7.61 (d,1H), 7.38 (d,1H), 4.82 (d,2H), 4.3 (q,2H), 2.52 (t,1 H), 1.48 (t,3H) |
| 1-045 | Et | Cl | Cl | CH₂CH=CH₂ | |
| 1-046 | Et | Cl | Cl | CH₂CH(Me)₂ | 400 MHz, CDCl₃ δ, ppm) 8.19 (s,1H), 7.58 (d,1H), 7.28 (d,1H), 4.31 (q,2H), 3.99 (d,2H), 2.09 (m,1H), 1.49 (t,3H), 0.98 (d,6H) |
| 1-047 | Et | Cl | Cl | CH₂CH=C(Me)₂ | (400 MHz, CDCl₃ δ, ppm) 8.18 (s,1H), 7.58 (d,1H), 7.28 (d,1H), 5.50 (m,1H), 4.72 (d,2H), 4.30 (q,2H), 1.80 (s,3H), 1.75 (s,3H), 1.50 (t,3H) |
| 1-048 | Et | Cl | Cl | CH₂CH₂OMe | |
| 1-049 | Et | Cl | Cl | CH₂CN | |
| 1-050 | Et | Cl | Cl | CH₂CH₂SMe | |
| 1-051 | Et | Cl | Cl | CH₂CF₃ | |
| 1-052 | Et | Cl | Cl | CH₂CHF₂ | |
| 1-053 | Et | Cl | SO₂Me | Me | (400 MHz, CDCl₃ δ, ppm) 8.28 (s,1 H), 7.95 (d,1H), 7.82 (d,1H), 4.28 (q,2H), 3.90 (s,3H), 3.25 (s,3H), 1.48 (t,3H) |
| 1-054 | Et | Cl | SO₂Me | Et | (400 MHz, CDCl₃ δ, ppm) 8.40 (s,1 H), 8.00 (d,1H), 7.85 (d,1H), 4.20 (q,2H), 4.18 (q,2H), 3.32 (s,3H), 1.35 (t,3H), 1.25 (t,3H) |
| 1-055 | Et | Cl | SO₂Me | nPr | |
| 1-056 | Et | Cl | SO₂Me | CH₂cPr | (400 MHz, CDCl₃ δ, ppm) 8.32 (s,1H), 7.95 (d,1H), 7.80 (d,1H), 4.31 (q,2H), 3.98 (d,2H), 3.32 (s,3H), 1.48 (t,3H), 1.18 (m,1H), 0.58 (m,2H), 0.32 (m,2H) |
| 1-057 | Et | Cl | SO₂Me | CH₂CCH | (400 MHz, CDCl₃ δ, ppm) 8.35 (s,1H), 7.93 (d,1H), 7.82 (d,1H), 4.78 (d,2H), 4.30 (q,2H), 3.25 (s,3H), 2.49 (t,1H), 1.48 (t,3H) |
| 1-058 | Et | Cl | SO₂Me | CH₂CH=CH₂ | |
| 1-059 | Et | Cl | SO₂Me | CH₂CH(Me)₂ | (400 MHz, CDCl₃ δ, ppm) 8.31 (s,1H), 7.96 (d,1H), 7.80 (d,1H), 4.30 (q,2H), 3.92 (d,2H), 3.22 (s,3H), 2.02 (m,1H), 1.48 (t,1H), 0.98 (d,6H) |
| 1-060 | Et | Cl | SO₂Me | CH₂CH=C(Me)₂ | (400 MHz, CDCl₃ δ, ppm) 8.29 (s,1 H), 7.95 (d,1H), 7.80 (d,1H), 5.46 (t,1H), 4.68 (d,1H), 4.28 (q,2H), 3.25 (s,3H), 1.78 (s,3H), 1.70 (s,3H), 1.46 (t,3H) |
| 1-061 | Et | Cl | SO₂Me | CH₂CH₂OMe | |
| 1-062 | Et | Cl | SO₂Me | CH₂CN | |
| 1-063 | Et | Cl | SO₂Me | CH₂CH₂SMe | |
| 1-064 | Et | Cl | SO₂Me | CH₂CF₃ | |
| 1-065 | Et | Cl | SO₂Me | CH₂CHF₂ | |
| 1-066 | Et | SO₂Me | Cl | Me | |
| 1-067 | Et | SO₂Me | Cl | Et | |
| 1-068 | Et | SO₂Me | Cl | nPr | |
| 1-069 | Et | SO₂Me | Cl | CH₂cPr | |
| 1-070 | Et | SO₂Me | Cl | CH₂CCH | |
| 1-071 | Et | SO₂Me | Cl | CH₂CH=CH₂ | |
| 1-072 | Et | SO₂Me | Cl | CH₂CH(Me)₂ | |
| 1-073 | Et | SO₂Me | Cl | CH₂CH=C(Me)₂ | |
| 1-074 | Et | SO₂Me | Cl | CH₂CH₂OMe | |
| 1-075 | Et | SO₂Me | Cl | CH₂CN | |
| 1-076 | Et | SO₂Me | Cl | CH₂CH₂SMe | |
| 1-077 | Et | SO₂Me | Cl | CH₂CF₃ | |
| 1-078 | Et | SO₂Me | Cl | CH₂CHF₂ | |
| 1-079 | nPr | Cl | Cl | Me | (400 MHz, CDCl₃ δ, ppm) 10.30 (br,s,1H), 8.24 (s,1H), 7.65 (d,1H), 7.50 (d,1H), 4.41 (t,2H), 4.04 (s,3H), 2.01 (m,2H), 0.98 (t,3H) |
| 1-080 | nPr | Cl | Cl | Et | (400 MHz, d⁶-DMSO δ, ppm) 11.78 (br,s,1H), 8.35 (s,1H), 7.75 (d,1H), 7.70 (d,1H), 4.29 (t,2H), 4.18 (q,2H), 1.88 (m,2H), 1.25 (t,3H), 0.85 (t,3H) |
| 1-081 | nPr | Cl | Cl | nPr | |
| 1-082 | nPr | Cl | Cl | CH₂cPr | (400 MHz, CDCl₃ δ, ppm) 8.21 (s,1 H), 7.58 (d,1H), 7.38 (d,1H), 4.42 (t,2H), 4.05 (d,2H), 2.01 (m,2H), 1.25 (m,1H), 0.90 (t,3H), 0.60 (m,2H), 0.35 (m,2H) |
| 1-083 | nPr | Cl | Cl | CH₂CCH | (400 MHz, CDCl₃ δ, ppm) 10.40 (br,s,1H), 8.32 (s,1H), 7.68 (d,1H), 7.50 (d,1H), 4.83 (d,2H), 4.41 (d,2H), 2.54 (t,1H), 2.03 (m,2H), 0.98 (t,3H) |
| 1-084 | nPr | Cl | Cl | CH₂CH=CH₂ | |
| 1-085 | nPr | Cl | Cl | CH₂CH(Me)₂ | (400 MHz, d⁶-DMSO δ, ppm) 11.76 (br,s,1H), 8.38 (s,1H), 7.76 (d,1H), 7.72 (d,1H), 4.28 (t,2H), 3.92 (d,2H), 2.01 (m,1H), 1.86 (m,2H), 0.93 (d,6H), 0.85 (t,3H) |
| 1-086 | nPr | Cl | Cl | CH₂CH=C(Me)₂ | (400 MHz, CDCl₃ δ, ppm) 11.5 (br,s,1H), 8.21 (s,1H), 7.58 (d,1H), 7.42 (d,1H), 5.50 (t,1H), 4.75 (d,2H), 4.38 (t,2H), 1.92 (m,2H), 1.79 (s,3H), 1.75 (s,3H), 0.92 (t,3H) |
| 1-087 | nPr | Cl | Cl | CH₂CH₂OMe | |
| 1-088 | nPr | Cl | Cl | CH₂CN | |
| 1-089 | nPr | Cl | Cl | CH₂CH₂SMe | |
| 1-090 | nPr | Cl | Cl | CH₂CF₃ | |
| 1-091 | nPr | Cl | Cl | CH₂CHF₂ | |
| 1-092 | nPr | Cl | SO₂Me | Me | (400 MHz, CDCl₃ δ, ppm) 8.27 (s,1 H), 7.92 (d,1H), 7.78 (d,1H), 4.19 (t,2H), 3.97 (s,3H), 3.23 (s,3H), 1.88 (m,2H), 2.03 (m,2H), 0.92 (t,3H) |
| 1-093 | nPr | Cl | SO₂Me | Et | (400 MHz, CDCl₃ δ, ppm) 8.30 (s,1H), 7.92 (d,1H), 7.78 (d,1H), 4.21 (q,2H), 4.20 (t,2H), 3.23 (s,3H), 1.88 (m,2H), 1.32 (t,3H), 0.92 (t,3H) |
| 1-094 | nPr | Cl | SO₂Me | nPr | |
| 1-095 | nPr | Cl | SO₂Me | CH₂cPr | (400 MHz, CDCl₃ δ, ppm) 8.33 (s,1 H), 7.92 (d,1H), 7.78 (d,1H), 4.20 (q,2H), 3.98 (d,2H), 3.23 (s,3H), 1.88 (m,2H), 1.18 (m,1H), 0.92 (t,3H), 0.62 (m,2H), 0.30 (m,2H) |
| 1-096 | nPr | Cl | SO₂Me | CH₂CCH | (400 MHz, CDCl₃ δ, ppm) 8.35 (s,1H), 7.92 (d,1H), 7.80 (d,1H), 4.74 (d,2H), 4.20 (q,2H), 3.23 (s,3H), 1.88 (m,2H), 0.92 (t,3H) |
| 1-097 | nPr | Cl | SO₂Me | CH₂CH=CH₂ | |
| 1-098 | nPr | Cl | SO₂Me | CH₂CH(Me)₂ | (400 MHz, d⁶-DMSO δ, ppm) 8.49 (s,1H), 8.07 (d,1H), 7.96 (d,1H), 4.25 (t,2H), 3.90 (d,2H), 3.32 (s,3H), 1.98 (m,1H), 1.95 (m,2H), 0.98 (d,6H), 0.85 (t,3H) |
| 1-099 | nPr | Cl | SO₂Me | CH₂CH=C(Me)₂ | (400 MHz, CDCl₃ δ, ppm) 8.25 (s,1H), 7.92 (d,1H), 7.75 (d,1H), 5.42 (t,1H), 4.65 (d,2H), 4.20 (t,2H), 3.20 (s,3H), 1.90 (m,1H), 1.75 (s,3H), 1.68 (s,3H), 0.92 (t,3H) |
| 1-100 | nPr | Cl | SO₂Me | CH₂CH₂OMe | |
| 1-101 | nPr | Cl | SO₂Me | CH₂CN | |
| 1-102 | nPr | Cl | SO₂Me | CH₂CH₂SMe | |
| 1-103 | nPr | Cl | SO₂Me | CH₂CF₃ | |
| 1-104 | nPr | Cl | SO₂Me | CH₂CHF₂ | |
| 1-105 | nPr | SO₂Me | Cl | Me | |
| 1-106 | nPr | SO₂Me | Cl | Et | |
| 1-107 | nPr | SO₂Me | Cl | nPr | |
| 1-108 | nPr | SO₂Me | Cl | CH₂cPr | |
| 1-109 | nPr | SO₂Me | Cl | CH₂CCH | |
| 1-110 | nPr | SO₂Me | Cl | CH₂CH=CH₂ | |
| 1-111 | nPr | SO₂Me | Cl | CH₂CH(Me)₂ | |
| 1-112 | nPr | SO₂Me | Cl | CH₂CH=C(Me)₂ | |
| 1-113 | nPr | SO₂Me | Cl | CH₂CH₂OMe | |
| 1-114 | nPr | SO₂Me | Cl | CH₂CN | |
| 1-115 | nPr | SO₂Me | Cl | CH₂CH₂SMe | |
| 1-116 | nPr | SO₂Me | Cl | CH₂CF₃ | |
| 1-117 | nPr | SO₂Me | Cl | CH₂CHF₂ | |

**Tabelle 2: Erfindungsgemäße Verbindungen der Formel (I), worin Q für Q2, R für CH=N-OR¹ steht, und die anderen Reste die in Tabelle 2 angegebenen Bedeutungen haben**

| | | | | | |
|---|---|---|---|---|---|
| 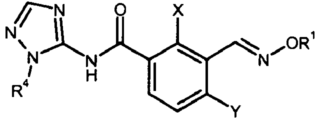 | | | | | |

| Nr. | R⁴ | X | Y | R¹ | Physikalische Daten (¹H-NMR) |
|---|---|---|---|---|---|
| 2-001 | Me | Cl | Cl | Me | |
| 2-002 | Me | Cl | Cl | Et | (400 MHz, CDCl₃ δ, ppm) 10.70 (br,s,1H), 8.22 (s,1H), 7.45 (s,1H), 7.30 (d,2H), 4.29 (q,2H), 3.79 (s,3H), 1.25 (t,3H) |
| 2-003 | Me | Cl | Cl | nPr | |
| 2-004 | Me | Cl | Cl | CH₂cPr | |
| 2-005 | Me | Cl | Cl | CH₂CCH | |
| 2-006 | Me | Cl | Cl | CH₂CH=CH₂ | |
| 2-007 | Me | Cl | Cl | CH₂CH(Me)₂ | |
| 2-008 | Me | Cl | Cl | CH₂CH=C(Me)₂ | |
| 2-009 | Me | Cl | Cl | CH₂CH₂OMe | |
| 2-010 | Me | Cl | Cl | CH₂CN | |
| 2-011 | Me | Cl | Cl | CH₂CH₂SMe | |
| 2-012 | Me | Cl | Cl | CH₂CF₃ | |
| 2-013 | Me | Cl | Cl | CH₂CHF₂ | |
| 2-014 | Me | Cl | SO₂Me | Me | |
| 2-015 | Me | Cl | SO₂Me | Et | (400 MHz, CDCl₃ δ, ppm) 11.30 (br,s,1 H), 8.39 (s,1 H), 8.10 (d,1 H), 7.81 (d,1H), 7.62 (s,1H), 4.27 (q,2H), 3.87 (s,3H), 3.26 (s,3H), 1.35 (t,3H) |
| 2-016 | Me | Cl | SO₂Me | nPr | |
| 2-017 | Me | Cl | SO₂Me | CH₂cPr | |
| 2-018 | Me | Cl | SO₂Me | CH₂CCH | |
| 2-019 | Me | Cl | SO₂Me | CH₂CH=CH₂ | |
| 2-020 | Me | Cl | SO₂Me | CH₂CH(Me)₂ | |
| 2-021 | Me | Cl | SO₂Me | CH₂CH=C(Me)₂ | |
| 2-022 | Me | Cl | SO₂Me | CH₂CH₂OMe | |
| 2-023 | Me | Cl | SO₂Me | CH₂CN | |
| 2-024 | Me | Cl | SO₂Me | CH₂CH₂SMe | |
| 2-025 | Me | Cl | SO₂Me | CH₂CF₃ | |
| 2-026 | Me | Cl | SO₂Me | CH₂CHF₂ | |
| 2-027 | Me | SO₂Me | Cl | Me | |
| 2-028 | Me | SO₂Me | Cl | Et | |
| 2-029 | Me | SO₂Me | Cl | nPr | |
| 2-030 | Me | SO₂Me | Cl | CH₂cPr | |
| 2-031 | Me | SO₂Me | Cl | CH₂CCH | |
| 2-032 | Me | SO₂Me | Cl | CH₂CH=CH₂ | |
| 2-033 | Me | SO₂Me | Cl | CH₂CH(Me)₂ | |
| 2-034 | Me | SO₂Me | Cl | CH₂CH=C(Me)₂ | |
| 2-035 | Me | SO₂Me | Cl | CH₂CH₂OMe | |
| 2-036 | Me | SO₂Me | Cl | CH₂CN | |
| 2-037 | Me | SO₂Me | Cl | CH₂CH₂SMe | |
| 2-038 | Me | SO₂Me | Cl | CH₂CF₃ | |
| 2-039 | Me | SO₂Me | Cl | CH₂CHF₂ | |
| 2-040 | Et | Cl | Cl | Me | |
| 2-041 | Et | Cl | Cl | Et | |
| 2-042 | Et | Cl | Cl | nPr | |
| 2-043 | Et | Cl | Cl | CH₂cPr | |
| 2-044 | Et | Cl | Cl | CH₂CCH | |
| 2-045 | Et | Cl | Cl | CH₂CH=CH₂ | |
| 2-046 | Et | Cl | Cl | CH₂CH(Me)₂ | |
| 2-047 | Et | Cl | Cl | CH₂CH=C(Me)₂ | |
| 2-048 | Et | Cl | Cl | CH₂CH₂OMe | |
| 2-049 | Et | Cl | Cl | CH₂CN | |
| 2-050 | Et | Cl | Cl | CH₂CH₂SMe | |
| 2-051 | Et | Cl | Cl | CH₂CF₃ | |
| 2-052 | Et | Cl | Cl | CH₂CHF₂ | |
| 2-053 | Et | Cl | SO₂Me | Me | |
| 2-054 | Et | Cl | SO₂Me | Et | |
| 2-055 | Et | Cl | SO₂Me | nPr | |
| 2-056 | Et | Cl | SO₂Me | CH₂cPr | |
| 2-057 | Et | Cl | SO₂Me | CH₂CCH | |
| 2-058 | Et | Cl | SO₂Me | CH₂CH=CH₂ | |
| 2-059 | Et | Cl | SO₂Me | CH₂CH(Me)₂ | |
| 2-060 | Et | Cl | SO₂Me | CH₂CH=C(Me)₂ | |
| 2-061 | Et | Cl | SO₂Me | CH₂CH₂OMe | |
| 2-062 | Et | Cl | SO₂Me | CH₂CN | |
| 2-063 | Et | Cl | SO₂Me | CH₂CH₂SMe | |
| 2-064 | Et | Cl | SO₂Me | CH₂CF₃ | |
| 2-065 | Et | Cl | SO₂Me | CH₂CHF₂ | |
| 2-066 | Et | SO₂Me | Cl | Me | |
| 2-067 | Et | SO₂Me | Cl | Et | |
| 2-068 | Et | SO₂Me | Cl | nPr | |
| 2-069 | Et | SO₂Me | Cl | CH₂cPr | |
| 2-070 | Et | SO₂Me | Cl | CH₂CCH | |
| 2-071 | Et | SO₂Me | Cl | CH₂CH=CH₂ | |
| 2-072 | Et | SO₂Me | Cl | CH₂CH(Me)₂ | |
| 2-073 | Et | SO₂Me | Cl | CH₂CH=C(Me)₂ | |
| 2-074 | Et | SO₂Me | Cl | CH₂CH₂OMe | |
| 2-075 | Et | SO₂Me | Cl | CH₂CN | |
| 2-076 | Et | SO₂Me | Cl | CH₂CH₂SMe | |
| 2-077 | Et | SO₂Me | Cl | CH₂CF₃ | |
| 2-078 | Et | SO₂Me | Cl | CH₂CHF₂ | |
| 2-079 | nPr | Cl | Cl | Me | |
| 2-080 | nPr | Cl | Cl | Et | |
| 2-081 | nPr | Cl | Cl | nPr | |
| 2-082 | nPr | Cl | Cl | CH₂cPr | |
| 2-083 | nPr | Cl | Cl | CH₂CCH | |
| 2-084 | nPr | Cl | Cl | CH₂CH=CH₂ | |
| 2-085 | nPr | Cl | Cl | CH₂CH(Me)₂ | |
| 2-086 | nPr | Cl | Cl | CH₂CH=C(Me)₂ | |
| 2-087 | nPr | Cl | Cl | CH₂CH₂OMe | |
| 2-088 | nPr | Cl | Cl | CH₂CN | |
| 2-089 | nPr | Cl | Cl | CH₂CH₂SMe | |
| 2-090 | nPr | Cl | Cl | CH₂CF₃ | |
| 2-091 | nPr | Cl | Cl | CH₂CHF₂ | |
| 2-092 | nPr | Cl | SO₂Me | Me | |
| 2-093 | nPr | Cl | SO₂Me | Et | |
| 2-094 | nPr | Cl | SO₂Me | nPr | |
| 2-095 | nPr | Cl | SO₂Me | CH₂cPr | |
| 2-096 | nPr | Cl | SO₂Me | CH₂CCH | |
| 2-097 | nPr | Cl | SO₂Me | CH₂CH=CH₂ | |
| 2-098 | nPr | Cl | SO₂Me | CH₂CH(Me)₂ | |
| 2-099 | nPr | Cl | SO₂Me | CH₂CH=C(Me)₂ | |
| 2-100 | nPr | Cl | SO₂Me | CH₂CH₂OMe | |
| 2-101 | nPr | Cl | SO₂Me | CH₂CN | |
| 2-102 | nPr | Cl | SO₂Me | CH₂CH₂SMe | |
| 2-103 | nPr | Cl | SO₂Me | CH₂CF₃ | |
| 2-104 | nPr | Cl | SO₂Me | CH₂CHF₂ | |
| 2-105 | nPr | SO₂Me | Cl | Me | |
| 2-106 | nPr | SO₂Me | Cl | Et | |
| 2-107 | nPr | SO₂Me | Cl | nPr | |
| 2-108 | nPr | SO₂Me | Cl | CH₂cPr | |
| 2-109 | nPr | SO₂Me | Cl | CH₂CCH | |
| 2-110 | nPr | SO₂Me | Cl | CH₂CH=CH₂ | |
| 2-111 | nPr | SO₂Me | Cl | CH₂CH(Me)₂ | |
| 2-112 | nPr | SO₂Me | Cl | CH₂CH=C(Me)₂ | |
| 2-113 | nPr | SO₂Me | Cl | CH₂CH₂OMe | |
| 2-114 | nPr | SO₂Me | Cl | CH₂CN | |
| 2-115 | nPr | SO₂Me | Cl | CH₂CH₂SMe | |
| 2-116 | nPr | SO₂Me | Cl | CH₂CF₃ | |
| 2-117 | nPr | SO₂Me | Cl | CH₂CHF₂ | |

**Tabelle 3: Erfindungsgemäße Verbindungen der Formel (I), worin Q für Q3, R für CH=N-OR¹ steht, und die anderen Reste die in Tabelle 3 angegebenen Bedeutungen haben**

| | | | | | |
|---|---|---|---|---|---|
| 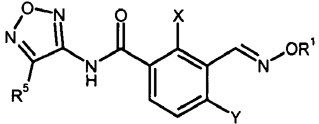 | | | | | |

| Nr. | R⁵ | X | Y | R¹ | Physikalische Daten (¹H-NMR) |
|---|---|---|---|---|---|
| 3-001 | Me | Cl | Cl | Me | (400 MHz, CDCl₃ δ, ppm) 8.24 (s,1H), 8.20 (br,s,1H), 7.67 (d,1H), 7.52 (d,1H), 4.05 (s,1H), 2.49 (s,3H) |
| 3-002 | Me | Cl | Cl | Et | (400 MHz, CDCl₃ δ, ppm) 8.25 (s,1H), 8.19 (br,s,1H), 7.66 (d,1H), 7.52 (d,1H), 4.29 (q,2H), 2.48 (s,3H), 1.36 (t,3H) |
| 3-003 | Me | Cl | Cl | nPr | |
| 3-004 | Me | Cl | Cl | CH₂cPr | (400 MHz, CDCl₃ δ, ppm) 8.30 (s,1H), 8.19 (br,s,1H), 7.66 (d,1H), 7.51 (d, 1H), 4.06 (d,2H), 2.49 (s,3H), 1.25 (m,1H), 0.61 (m,2H), 0.37 (m,2H) |
| 3-005 | Me | Cl | Cl | CH₂CCH | (400 MHz, CDCl₃ δ, ppm) 8.32 (s,1H), 8.20 (br,s,1H), 7.68 (d,1H), 7.52 (d,1H), 4.84 (d,2H), 2.54 (m,1H), 2.49 (s,3H) |
| 3-006 | Me | Cl | Cl | CH₂CH=CH₂ | |
| 3-007 | Me | Cl | Cl | CH₂CH(Me)₂ | |
| 3-008 | Me | Cl | Cl | CH₂CH=C(Me)₂ | |
| 3-009 | Me | Cl | Cl | CH₂CH₂OMe | |
| 3-010 | Me | Cl | Cl | CH₂CN | |
| 3-011 | Me | Cl | Cl | CH₂CH₂SMe | |
| 3-012 | Me | Cl | Cl | CH₂CF₃ | |
| 3-013 | Me | Cl | Cl | CH₂CHF₂ | |
| 3-014 | Me | Cl | SO₂Me | Me | (400 MHz, CDCl₃ δ, ppm) 8.73 (br,s,1H), 8.28 (s,1H), 7.98 (d,1H), 7.79 (d,1H), 4.01 (s,3H), 3.27 (s,3H), 2.50 (s,3H) |
| 3-015 | Me | Cl | SO₂Me | Et | (400 MHz, CDCl₃ δ, ppm) 8.58 (br,s,1H), 8.31 (s,1H), 8.01 (d,1H), 7.81 (d,1H), 4.25 (q,2H), 3.25 (s,3H), 2.50 (s,3H), 1.32 (t,3H) |
| 3-016 | Me | Cl | SO₂Me | nPr | |
| 3-017 | Me | Cl | SO₂Me | CH₂cPr | (400 MHz, CDCl₃ δ, ppm) 8.75 (br,s,1H), 8.32 (s,1H), 7.97 (d,1H), 7.78 (d,1H), 4.01 (d,2H), 3.25 (s,3H), 2.50 (s,3H), 1.18 (m,1H), 0.60 (m,2H), 0.32 (m,2H) |
| 3-018 | Me | Cl | SO₂Me | CH₂CCH | (400 MHz, CDCl₃ δ ppm) 8.42 (s, 1H), 8.31 (br,s, 1H), 8.11 (d,1H), 7.88 (d,1H), 4.78 (d,2H), 3.29 (s,3H), 2.50 (s,3H), 2.50 (t,1H), 1.18 (m,1H), 0.60 (m,2H), 0.32 (m,2H) |
| 3-019 | Me | Cl | SO₂Me | CH₂CH=CH₂ | |
| 3-020 | Me | Cl | SO₂Me | CH₂CH(Me)₂ | (400 MHz, CDCl₃ δ, ppm) 8.74 (br,s,1H), 8.30 (s,1H), 7.96 (d,1H), 7.78 (d,1H), 3.96 (d,2H), 3.24 (s,3H), 2.50 (s,3H), 2.02 (m,1H), 0.97 (d,6H) |
| 3-021 | Me | Cl | SO₂Me | CH₂CH=C(Me)₂ | |
| 3-022 | Me | Cl | SO₂Me | CH₂CH₂OMe | |
| 3-023 | Me | Cl | SO₂Me | CH₂CN | |
| 3-024 | Me | Cl | SO₂Me | CH₂CH₂SMe | |
| 3-025 | Me | Cl | SO₂Me | CH₂CF₃ | |
| 3-026 | Me | Cl | SO₂Me | CH₂CHF₂ | |
| 3-027 | Me | SO₂Me | Cl | Me | |
| 3-028 | Me | SO₂Me | Cl | Et | |
| 3-029 | Me | SO₂Me | Cl | nPr | |
| 3-030 | Me | SO₂Me | Cl | CH₂cPr | |
| 3-031 | Me | SO₂Me | Cl | CH₂CCH | |
| 3-032 | Me | SO₂Me | Cl | CH₂CH=CH₂ | |
| 3-033 | Me | SO₂Me | Cl | CH₂CH(Me)₂ | |
| 3-034 | Me | SO₂Me | Cl | CH₂CH=C(Me)₂ | |
| 3-035 | Me | SO₂Me | Cl | CH₂CH₂OMe | |
| 3-036 | Me | SO₂Me | Cl | CH₂CN | |
| 3-037 | Me | SO₂Me | Cl | CH₂CH₂SMe | |
| 3-038 | Me | SO₂Me | Cl | CH₂CF₃ | |
| 3-039 | Me | SO₂Me | Cl | CH₂CHF₂ | |
| 3-040 | OMe | Cl | Cl | Me | |
| 3-041 | OMe | Cl | Cl | Et | |
| 3-042 | OMe | Cl | Cl | nPr | |
| 3-043 | OMe | Cl | Cl | CH₂cPr | |
| 3-044 | OMe | Cl | Cl | CH₂CCH | |
| 3-045 | OMe | Cl | Cl | CH₂CH=CH₂ | |
| 3-046 | OMe | Cl | Cl | CH₂CH(Me)₂ | |
| 3-047 | OMe | Cl | Cl | CH₂CH=C(Me)₂ | |
| 3-048 | OMe | Cl | Cl | CH₂CH₂OMe | |
| 3-049 | OMe | Cl | Cl | CH₂CN | |
| 3-050 | OMe | Cl | Cl | CH₂CH₂SMe | |
| 3-051 | OMe | Cl | Cl | CH₂CF₃ | |
| 3-052 | OMe | Cl | Cl | CH₂CHF₂ | |
| 3-053 | OMe | Cl | SO₂Me | Me | |
| 3-054 | OMe | Cl | SO₂Me | Et | |
| 3-055 | OMe | Cl | SO₂Me | nPr | |
| 3-056 | OMe | Cl | SO₂Me | CH₂cPr | |
| 3-057 | OMe | Cl | SO₂Me | CH₂CCH | |
| 3-058 | OMe | Cl | SO₂Me | CH₂CH=CH₂ | |
| 3-059 | OMe | Cl | SO₂Me | CH₂CH(Me)₂ | |
| 3-060 | OMe | Cl | SO₂Me | CH₂CH=C(Me)₂ | |
| 3-061 | OMe | Cl | SO₂Me | CH₂CH₂OMe | |
| 3-062 | OMe | Cl | SO₂Me | CH₂CN | |
| 3-063 | OMe | Cl | SO₂Me | CH₂CH₂SMe | |
| 3-064 | OMe | Cl | SO₂Me | CH₂CF₃ | |
| 3-065 | OMe | Cl | SO₂Me | CH₂CHF₂ | |
| 3-066 | OMe | SO₂Me | Cl | Me | |
| 3-067 | OMe | SO₂Me | Cl | Et | |
| 3-068 | OMe | SO₂Me | Cl | nPr | |
| 3-069 | OMe | SO₂Me | Cl | CH₂cPr | |
| 3-070 | OMe | SO₂Me | Cl | CH₂CCH | |
| 3-071 | OMe | SO₂Me | Cl | CH₂CH=CH₂ | |
| 3-072 | OMe | SO₂Me | Cl | CH₂CH(Me)₂ | |
| 3-073 | OMe | SO₂Me | Cl | CH₂CH=C(Me)₂ | |
| 3-074 | OMe | SO₂Me | Cl | CH₂CH₂OMe | |
| 3-075 | OMe | SO₂Me | Cl | CH₂CN | |
| 3-076 | OMe | SO₂Me | Cl | CH₂CH₂SMe | |
| 3-077 | OMe | SO₂Me | Cl | CH₂CF₃ | |
| 3-078 | OMe | SO₂Me | Cl | CH₂CHF₂ | |
| 3-079 | Cl | Cl | Cl | Me | |
| 3-080 | Cl | Cl | Cl | Et | |
| 3-081 | Cl | Cl | Cl | nPr | |
| 3-082 | Cl | Cl | Cl | CH₂cPr | |
| 3-083 | Cl | Cl | Cl | CH₂CCH | |
| 3-084 | Cl | Cl | Cl | CH₂CH=CH₂ | |
| 3-085 | Cl | Cl | Cl | CH₂CH(Me)₂ | |
| 3-086 | Cl | Cl | Cl | CH₂CH=C(Me)₂ | |
| 3-087 | Cl | Cl | Cl | CH₂CH₂OMe | |
| 3-088 | Cl | Cl | Cl | CH₂CN | |
| 3-089 | Cl | Cl | Cl | CH₂CH₂SMe | |
| 3-090 | Cl | Cl | Cl | CH₂CF₃ | |
| 3-091 | Cl | Cl | Cl | CH₂CHF₂ | |
| 3-092 | Cl | Cl | SO₂Me | Me | |
| 3-093 | Cl | Cl | SO₂Me | Et | |
| 3-094 | Cl | Cl | SO₂Me | nPr | |
| 3-095 | Cl | Cl | SO₂Me | CH₂cPr | |
| 3-096 | Cl | Cl | SO₂Me | CH₂CCH | |
| 3-097 | Cl | Cl | SO₂Me | CH₂CH=CH₂ | |
| 3-098 | Cl | Cl | SO₂Me | CH₂CH(Me)₂ | |
| 3-099 | Cl | Cl | SO₂Me | CH₂CH=C(Me)₂ | |
| 3-100 | Cl | Cl | SO₂Me | CH₂CH₂OMe | |
| 3-101 | Cl | Cl | SO₂Me | CH₂CN | |
| 3-102 | Cl | Cl | SO₂Me | CH₂CH₂SMe | |
| 3-103 | Cl | Cl | SO₂Me | CH₂CF₃ | |
| 3-104 | Cl | Cl | SO₂Me | CH₂CHF₂ | |
| 3-105 | Cl | SO₂Me | Cl | Me | |
| 3-106 | Cl | SO₂Me | Cl | Et | |
| 3-107 | Cl | SO₂Me | Cl | nPr | |
| 3-108 | Cl | SO₂Me | Cl | CH₂cPr | |
| 3-109 | Cl | SO₂Me | Cl | CH₂CCH | |
| 3-110 | Cl | SO₂Me | Cl | CH₂CH=CH₂ | |
| 3-111 | Cl | SO₂Me | Cl | CH₂CH(Me)₂ | |
| 3-112 | Cl | SO₂Me | Cl | CH₂CH=C(Me)₂ | |
| 3-113 | Cl | SO₂Me | Cl | CH₂CH₂OMe | |
| 3-114 | Cl | SO₂Me | Cl | CH₂CN | |
| 3-115 | Cl | SO₂Me | Cl | CH₂CH₂SMe | |
| 3-116 | Cl | SO₂Me | Cl | CH₂CF₃ | |
| 3-117 | Cl | SO₂Me | Cl | CH₂CHF₂ | |

**Tabelle 4: Erfindungsgemäße Verbindungen der Formel (I), worin Q für Q4, R für CH=N-OR¹ steht, und die anderen Reste die in Tabelle 4 angegebenen Bedeutungen haben**

| | | | | | |
|---|---|---|---|---|---|
| 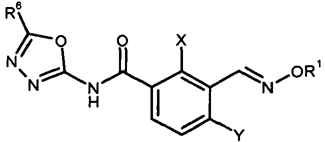 | | | | | |

| Nr. | R⁶ | X | Y | R¹ | Physikalische Daten (¹H-NMR) |
|---|---|---|---|---|---|
| 4-001 | Me | Cl | Cl | Me | 400 MHz, DMSO-d₆, 12.31 (bs, 1H); 8.34 (s, 1H), 7.70 (s, 2H), 3.94 (s, 3H), 2.50 (s, 3H) |
| 4-002 | Me | Cl | Cl | Et | |
| 4-003 | Me | Cl | Cl | nPr | |
| 4-004 | Me | Cl | Cl | CH₂cPr | |
| 4-005 | Me | Cl | Cl | CH₂CCH | |
| 4-006 | Me | Cl | Cl | CH₂CH=CH₂ | |
| 4-007 | Me | Cl | Cl | CH₂CH(Me)₂ | |
| 4-008 | Me | Cl | Cl | CH₂CH=C(Me)₂ | |
| 4-009 | Me | Cl | Cl | CH₂CH₂OMe | |
| 4-010 | Me | Cl | Cl | CH₂CN | |
| 4-011 | Me | Cl | Cl | CH₂CH₂SMe | |
| 4-012 | Me | Cl | Cl | CH₂CF₃ | |
| 4-013 | Me | Cl | Cl | CH₂CHF₂ | |
| 4-014 | Me | Cl | SO₂Me | Me | |
| 4-015 | Me | Cl | SO₂Me | Et | |
| 4-016 | Me | Cl | SO₂Me | nPr | |
| 4-017 | Me | Cl | SO₂Me | CH₂cPr | 400 MHz, DMSO-d₆, 12.48 (bs, 1H); 8.49 (s, 1H), 8.09 (d, 1H), 7.97 (d, 1H), 3.97 (d, 2H), 3.38 (s, 3H), 2.50 (s, 3H), 1.16 (m, 1H), 0.55 (m, 2H), 0.28 (m, 2H). |
| 4-018 | Me | Cl | SO₂Me | CH₂CCH | |
| 4-019 | Me | Cl | SO₂Me | CH₂CH=CH₂ | |
| 4-020 | Me | Cl | SO₂Me | CH₂CH(Me)₂ | |
| 4-021 | Me | Cl | SO₂Me | CH₂CH=C(Me)₂ | |
| 4-022 | Me | Cl | SO₂Me | CH₂CH₂OMe | |
| 4-023 | Me | Cl | SO₂Me | CH₂CN | |
| 4-024 | Me | Cl | SO₂Me | CH₂CH₂SMe | |
| 4-025 | Me | Cl | SO₂Me | CH₂CF₃ | |
| 4-026 | Me | Cl | SO₂Me | CH₂CHF₂ | |
| 4-027 | Me | SO₂Me | Cl | Me | |
| 4-028 | Me | SO₂Me | Cl | Et | |
| 4-029 | Me | SO₂Me | Cl | nPr | |
| 4-030 | Me | SO₂Me | Cl | CH₂cPr | |
| 4-031 | Me | SO₂Me | Cl | CH₂CCH | |
| 4-032 | Me | SO₂Me | Cl | CH₂CH=CH₂ | |
| 4-033 | Me | SO₂Me | Cl | CH₂CH(Me)₂ | |
| 4-034 | Me | SO₂Me | Cl | CH₂CH=C(Me)₂ | |
| 4-035 | Me | SO₂Me | Cl | CH₂CH₂OMe | |
| 4-036 | Me | SO₂Me | Cl | CH₂CN | |
| 4-037 | Me | SO₂Me | Cl | CH₂CH₂SMe | |
| 4-038 | Me | SO₂Me | Cl | CH₂CF₃ | |
| 4-039 | Me | SO₂Me | Cl | CH₂CHF₂ | |
| 4-040 | Et | Cl | Cl | Me | (400 MHz, CDCL₃ δ, ppm) 8.21 (s, 1H), 7.63 (d,1H), 7.42 (d,1H), 4.03 (s,3H), 2.86 (q,2H), 1.38 (t,3H) |
| 4-041 | Et | Cl | Cl | Et | |
| 4-042 | Et | Cl | Cl | nPr | |
| 4-043 | Et | Cl | Cl | CH₂cPr | |
| 4-044 | Et | Cl | Cl | CH₂CCH | |
| 4-045 | Et | Cl | Cl | CH₂CH=CH₂ | |
| 4-046 | Et | Cl | Cl | CH₂CH(Me)₂ | |
| 4-047 | Et | Cl | Cl | CH₂CH=C(Me)₂ | |
| 4-048 | Et | Cl | Cl | CH₂CH₂OMe | |
| 4-049 | Et | Cl | Cl | CH₂CN | |
| 4-050 | Et | Cl | Cl | CH₂CH₂SMe | |
| 4-051 | Et | Cl | Cl | CH₂CF₃ | |
| 4-052 | Et | Cl | Cl | CH₂CHF₂ | |
| 4-053 | Et | Cl | SO₂Me | Me | |
| 4-054 | Et | Cl | SO₂Me | Et | |
| 4-055 | Et | Cl | SO₂Me | nPr | |
| 4-056 | Et | Cl | SO₂Me | CH₂cPr | |
| 4-057 | Et | Cl | SO₂Me | CH₂CCH | |
| 4-058 | Et | Cl | SO₂Me | CH₂CH=CH₂ | |
| 4-059 | Et | Cl | SO₂Me | CH₂CH(Me)₂ | |
| 4-060 | Et | Cl | SO₂Me | CH₂CH=C(Me)₂ | |
| 4-061 | Et | Cl | SO₂Me | CH₂CH₂OMe | |
| 4-062 | Et | Cl | SO₂Me | CH₂CN | |
| 4-063 | Et | Cl | SO₂Me | CH₂CH₂SMe | |
| 4-064 | Et | Cl | SO₂Me | CH₂CF₃ | |
| 4-065 | Et | Cl | SO₂Me | CH₂CHF₂ | |
| 4-066 | Et | SO₂Me | Cl | Me | |
| 4-067 | Et | SO₂Me | Cl | Et | |
| 4-068 | Et | SO₂Me | Cl | nPr | |
| 4-069 | Et | SO₂Me | Cl | CH₂cPr | |
| 4-070 | Et | SO₂Me | Cl | CH₂CCH | |
| 4-071 | Et | SO₂Me | Cl | CH₂CH=CH₂ | |
| 4-072 | Et | SO₂Me | Cl | CH₂CH(Me)₂ | |
| 4-073 | Et | SO₂Me | Cl | CH₂CH=C(Me)₂ | |
| 4-074 | Et | SO₂Me | Cl | CH₂CH₂OMe | |
| 4-075 | Et | SO₂Me | Cl | CH₂CN | |
| 4-076 | Et | SO₂Me | Cl | CH₂CH₂SMe | |
| 4-077 | Et | SO₂Me | Cl | CH₂CF₃ | |
| 4-078 | Et | SO₂Me | Cl | CH₂CHF₂ | |
| 4-079 | CH₂OMe | Cl | Cl | Me | |
| 4-080 | CH₂OMe | Cl | Cl | Et | |
| 4-081 | CH₂OMe | Cl | Cl | nPr | |
| 4-082 | CH₂OMe | Cl | Cl | CH₂cPr | |
| 4-083 | CH₂OMe | Cl | Cl | CH₂CCH | |
| 4-084 | CH₂OMe | Cl | Cl | CH₂CH=CH₂ | |
| 4-085 | CH₂OMe | Cl | Cl | CH₂CH(Me)₂ | |
| 4-086 | CH₂OMe | Cl | Cl | CH₂CH=C(Me)₂ | |
| 4-087 | CH₂OMe | Cl | Cl | CH₂CH₂OMe | |
| 4-088 | CH₂OMe | Cl | Cl | CH₂CN | |
| 4-089 | CH₂OMe | Cl | Cl | CH₂CH₂SMe | |
| 4-090 | CH₂OMe | Cl | Cl | CH₂CF₃ | |
| 4-091 | CH₂OMe | Cl | Cl | CH₂CHF₂ | |
| 4-092 | CH₂OMe | Cl | SO₂Me | Me | (400 MHz, CDCl₃ δ, ppm) 8.26 (s,1H), 7.95 (d,1H), 7.84 (d,1H), 4.49 (s,2H), 4.00 (s,3H), 3.42 (s,3H), 3.24 (s,3H) |
| 4-093 | CH₂OMe | Cl | SO₂Me | Et | (400 MHz, CDCl₃ δ, ppm) 8.36 (s,1H), 8.12 (d,1H), 7.85 (d,1H), 4.60 (s,2H), 4.27 (q,2H), 3.49 (s,3H), 3.28 (s,3H), 1.35 (t,3H) |
| 4-094 | CH₂OMe | Cl | SO₂Me | nPr | |
| 4-095 | CH₂OMe | Cl | SO₂Me | CH₂cPr | |
| 4-096 | CH₂OMe | Cl | SO₂Me | CH₂CCH | 400 MHz, DMSO-d₆, 12.49 (bs, 1H); 8.57 (s, 1H), 8.10 (d, 1H), 8.00 (d, 1H), 4.81 (s, 2H), 3.38 (s, 3H), 3.32 (s, 1H), 2.50 (s, 3H) |
| 4-097 | CH₂OMe | Cl | SO₂Me | CH₂CH=CH₂ | |
| 4-098 | CH₂OMe | Cl | SO₂Me | CH₂CH(Me)₂ | |
| 4-099 | CH₂OMe | Cl | SO₂Me | CH₂CH=C(Me)₂ | |
| 4-100 | CH₂OMe | Cl | SO₂Me | CH₂CH₂OMe | |
| 4-101 | CH₂OMe | Cl | SO₂Me | CH₂CN | |
| 4-102 | CH₂OMe | Cl | SO₂Me | CH₂CH₂SMe | |
| 4-103 | CH₂OMe | Cl | SO₂Me | CH₂CF₃ | |
| 4-104 | CH₂OMe | Cl | SO₂Me | CH₂CHF₂ | |
| 4-105 | CH₂OMe | SO₂Me | Cl | Me | |
| 4-106 | CH₂OMe | SO₂Me | Cl | Et | |
| 4-107 | CH₂OMe | SO₂Me | Cl | nPr | |
| 4-108 | CH₂OMe | SO₂Me | Cl | CH₂cPr | |
| 4-109 | CH₂OMe | SO₂Me | Cl | CH₂CCH | |
| 4-110 | CH₂OMe | SO₂Me | Cl | CH₂CH=CH₂ | |
| 4-111 | CH₂OMe | SO₂Me | Cl | CH₂CH(Me)₂ | |
| 4-112 | CH₂OMe | SO₂Me | Cl | CH₂CH=C(Me)₂ | |
| 4-113 | CH₂OMe | SO₂Me | Cl | CH₂CH₂OMe | |
| 4-114 | CH₂OMe | SO₂Me | Cl | CH₂CN | |
| 4-115 | CH₂OMe | SO₂Me | Cl | CH₂CH₂SMe | |
| 4-116 | CH₂OMe | SO₂Me | Cl | CH₂CF₃ | |
| 4-117 | CH₂OMe | SO₂Me | Cl | CH₂CHF₂ | |

**Tabelle 5: Erfindungsgemäße Verbindungen der Formel (I), worin Q für Q1, R für CH₂-O-N=CR²R³ steht, und die anderen Reste die in Tabelle 5 angegebenen Bedeutungen haben**

| | | | | | | |
|---|---|---|---|---|---|---|
| 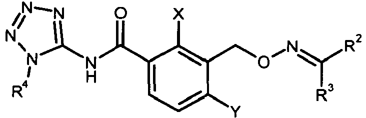 | | | | | | |

| Nr. | R⁴ | X | Y | R² | R³ | Physikal. Daten (¹H-NMR) |
|---|---|---|---|---|---|---|
| 5-001 | Me | Cl | Cl | H | H | |
| 5-002 | Me | Cl | Cl | H | Me | |
| 5-003 | Me | Cl | Cl | H | Et | |
| 5-004 | Me | Cl | Cl | Me | Me | (400 MHz, CDCl₃ δ, ppm) 10.55 (br,s,1H), 7.65 (d,1H), 7.49 (d,1H), 5.38 (s,2H), 4.11 (s,3H), 1.86 (s,3H), 1.83 (s,3H) |
| 5-005 | Me | Ct | Cl | Et | Et | |
| 5-006 | Me | Cl | Cl | H | Ph | |
| 5-007 | Me | Cl | Cl | H | CH₂OMe | |
| 5-008 | Me | Cl | Cl | H | CH₂cPr | |
| 5-009 | Me | Cl | Cl | H | CH₂CH₂OMe | |
| 5-010 | Me | Cl | Cl | Me | Ph | |
| 5-011 | Me | Cl | Cl | Me | CH₂OMe | |
| 5-012 | Me | Cl | Cl | Me | CH₂cPr | |
| 5-013 | Me | Cl | Cl | Me | CH₂CH₂OMe | |
| 5-014 | Me | Cl | SO₂Me | H | H | |
| 5-015 | Me | Cl | SO₂Me | H | Me | |
| 5-016 | Me | Cl | SO₂Me | H | Et | |
| 5-017 | Me | Cl | SO₂Me | Me | Me | (400 MHz, CDCl₃ δ, ppm) 10.80 (br,s,1H), 8.11 (d,1H), 7.77 (d,1H), 5.69 (s,2H), 4.12 (s,3H), 3.36 (s,3H), 1.82 (s,3H), 1.81 (s,3H) |
| 5-018 | Me | Cl | SO₂Me | Et | Et | |
| 5-019 | Me | Cl | SO₂Me | H | Ph | |
| 5-020 | Me | Cl | SO₂Me | H | CH₂OMe | |
| 5-021 | Me | Cl | SO₂Me | H | CH₂cPr | |
| 5-022 | Me | Cl | SO₂Me | H | CH₂CH₂OMe | |
| 5-023 | Me | Cl | SO₂Me | Me | Ph | |
| 5-024 | Me | Cl | SO₂Me | Me | CH₂OMe | |
| 5-025 | Me | Cl | SO₂Me | Me | CH₂cPr | |
| 5-026 | Me | Cl | SO₂Me | Me | CH₂CH₂OMe | |
| 5-027 | Me | SO₂Me | Cl | H | H | |
| 5-028 | Me | SO₂Me | Cl | H | Me | |
| 5-029 | Me | SO₂Me | Cl | H | Et | |
| 5-030 | Me | SO₂Me | Cl | Me | Me | |
| 5-031 | Me | SO₂Me | Cl | Et | Et | |
| 5-032 | Me | SO₂Me | Cl | H | Ph | |
| 5-033 | Me | SO₂Me | Cl | H | CH₂OMe | |
| 5-034 | Me | SO₂Me | Cl | H | CH₂cPr | |
| 5-035 | Me | SO₂Me | Cl | H | CH₂CH₂OMe | |
| 5-036 | Me | SO₂Me | Cl | Me | Ph | |
| 5-037 | Me | SO₂Me | Cl | Me | CH₂OMe | |
| 5-038 | Me | SO₂Me | Cl | Me | CH₂cPr | |
| 5-039 | Me | SO₂Me | Cl | Me | CH₂CH₂OMe | |
| 5-040 | Et | Cl | Cl | H | H | |
| 5-041 | Et | Cl | Cl | H | Me | |
| 5-042 | Et | Cl | Cl | H | Et | |
| 5-043 | Et | Cl | Cl | Me | Me | (400 MHz, CDCl₃ δ, ppm) 10.04 (br,s,1H), 7.66 (d,1H), 7.49 (d,1H), 5.39 (s,2H), 4.48 (q,2H), 1.88 (s,3H), 1.83 (s,3H), 1.62 (t,3H) |
| 5-044 | Et | Cl | Cl | Et | Et | |
| 5-045 | Et | Cl | Cl | H | Ph | |
| 5-046 | Et | Cl | Cl | H | CH₂OMe | |
| 5-047 | Et | Cl | Cl | H | CH₂cPr | |
| 5-048 | Et | Cl | Cl | H | CH₂CH₂OMe | |
| 5-049 | Et | Cl | Cl | Me | Ph | |
| 5-050 | Et | Cl | Cl | Me | CH₂OMe | |
| 5-051 | Et | Cl | Cl | Me | CH₂cPr | |
| 5-052 | Et | Cl | Cl | Me | CH₂CH₂OMe | |
| 5-053 | Et | Cl | SO₂Me | H | H | |
| 5-054 | Et | Cl | SO₂Me | H | Me | |
| 5-055 | Et | Cl | SO₂Me | H | Et | |
| 5-056 | Et | Cl | SO₂Me | Me | Me | (400 MHz, CDCl₃ δ, ppm) 10.38 (br,s,1H), 8.16 (d,1H), 7.78 (d,1H), 5.70 (s,2H), 4.48 (q,2H), 3.35 (s,3H), 1.80 (s,3H), 1.79 (s,3H), 1.64 (t,3H) |
| 5-057 | Et | Cl | SO₂Me | Et | Et | |
| 5-058 | Et | Cl | SO₂Me | H | Ph | |
| 5-059 | Et | Cl | SO₂Me | H | CH₂OMe | |
| 5-060 | Et | Cl | SO₂Me | H | CH₂cPr | |
| 5-061 | Et | Cl | SO₂Me | H | CH₂CH₂OMe | |
| 5-062 | Et | Cl | SO₂Me | Me | Ph | |
| 5-063 | Et | Cl | SO₂Me | Me | CH₂OMe | |
| 5-064 | Et | Cl | SO₂Me | Me | CH₂cPr | |
| 5-065 | Et | Cl | SO₂Me | Me | CH₂CH₂OMe | |
| 5-066 | Et | SO₂Me | Cl | H | H | |
| 5-067 | Et | SO₂Me | Cl | H | Me | |
| 5-068 | Et | SO₂Me | Cl | H | Et | |
| 5-069 | Et | SO₂Me | Cl | Me | Me | |
| 5-070 | Et | SO₂Me | Cl | Et | Et | |
| 5-071 | Et | SO₂Me | Cl | H | Ph | |
| 5-072 | Et | SO₂Me | Cl | H | CH₂OMe | |
| 5-073 | Et | SO₂Me | Cl | H | CH₂cPr | |
| 5-074 | Et | SO₂Me | Cl | H | CH₂CH₂OMe | |
| 5-075 | Et | SO₂Me | Cl | Me | Ph | |
| 5-076 | Et | SO₂Me | Cl | Me | CH₂OMe | |
| 5-077 | Et | SO₂Me | Cl | Me | CH₂cPr | |
| 5-078 | Et | SO₂Me | Cl | Me | CH₂CH₂OMe | |
| 5-079 | nPr | Cl | Cl | H | H | |
| 5-080 | nPr | Cl | Cl | H | Me | |
| 5-081 | nPr | Cl | Cl | H | Et | |
| 5-082 | nPr | Cl | Cl | Me | Me | (400 MHz, CDCl₃ δ, ppm) 10.32 (br,s,1H), 7.63 (d,1H), 7.47 (d,1H), 5.38 (s,2H), 4.41 (t,2H), 2.02 (m,2H), 1.87 (s,3H), 1.83 (s,3H), 0.98 (t,3H) |
| 5-083 | nPr | Cl | Cl | Et | Et | |
| 5-084 | nPr | Cl | Cl | H | Ph | |
| 5-085 | nPr | Cl | Cl | H | CH₂OMe | |
| 5-086 | nPr | Cl | Cl | H | CH₂cPr | |
| 5-087 | nPr | Cl | Cl | H | CH₂CH₂OMe | |
| 5-088 | nPr | Cl | Cl | Me | Ph | |
| 5-089 | nPr | Cl | Cl | Me | CH₂OMe | |
| 5-090 | nPr | Cl | Cl | Me | CH₂cPr | |
| 5-091 | nPr | Cl | Cl | Me | CH₂CH₂OMe | |
| 5-092 | nPr | Cl | SO₂Me | H | H | |
| 5-093 | nPr | Cl | SO₂Me | H | Me | |
| 5-094 | nPr | Cl | SO₂Me | H | Et | |
| 5-095 | nPr | Cl | SO₂Me | Me | Me | (400 MHz, CDCl₃ δ, ppm) 10.50 (br,s,1H), 8.15 (d,1H), 7.80 (d,1H), 5.70 (s,2H), 4.44 (t,2H), 3.36 (s,3H), 2.05 (m,2H), 1.82 (s,3H), 1.81 (s,3H), 1.00 (t,3H) |
| 5-096 | nPr | Cl | SO₂Me | Et | Et | |
| 5-097 | nPr | Cl | SO₂Me | H | Ph | |
| 5-098 | nPr | Cl | SO₂Me | H | CH₂OMe | |
| 5-099 | nPr | Cl | SO₂Me | H | CH₂cPr | |
| 5-100 | nPr | Cl | SO₂Me | H | CH₂CH₂OMe | |
| 5-101 | nPr | Cl | SO₂Me | Me | Ph | |
| 5-102 | nPr | Cl | SO₂Me | Me | CH₂OMe | |
| 5-103 | nPr | Cl | SO₂Me | Me | CH₂cPr | |
| 5-104 | nPr | Cl | SO₂Me | Me | CH₂CH₂OMe | |
| 5-105 | nPr | SO₂Me | Cl | H | H | |
| 5-106 | nPr | SO₂Me | Cl | H | Me | |
| 5-107 | nPr | SO₂Me | Cl | H | Et | |
| 5-108 | nPr | SO₂Me | Cl | Me | Me | |
| 5-109 | nPr | SO₂Me | Cl | Et | Et | |
| 5-110 | nPr | SO₂Me | Cl | H | Ph | |
| 5-111 | nPr | SO₂Me | Cl | H | CH₂OMe | |
| 5-112 | nPr | SO₂Me | Cl | H | CH₂cPr | |
| 5-113 | nPr | SO₂Me | Cl | H | CH₂CH₂OMe | |
| 5-114 | nPr | SO₂Me | Cl | Me | Ph | |
| 5-115 | nPr | SO₂Me | Cl | Me | CH₂OMe | |
| 5-116 | nPr | SO₂Me | Cl | Me | CH₂cPr | |
| 5-117 | nPr | SO₂Me | Cl | Me | CH₂CH₂OMe | |

**Tabelle 6: Erfindungsgemäße Verbindungen der Formel (I), worin Q für Q2, R für CH₂-O-N=CR²R³ steht, und die anderen Reste die in Tabelle 6 angegebenen Bedeutungen haben**

| | | | | | | |
|---|---|---|---|---|---|---|
| 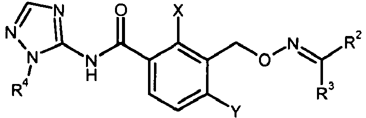 | | | | | | |

| Nr. | R⁴ | X | Y | R² | R³ | Physikal. Daten (¹H-NMR) |
|---|---|---|---|---|---|---|
| 6-001 | Me | Cl | Cl | H | H | |
| 6-002 | Me | Cl | Cl | H | Me | |
| 6-003 | Me | Cl | Cl | H | Et | |
| 6-004 | Me | Cl | Cl | Me | Me | (400 MHz, CDCl₃ δ, ppm) 7.52 (d,1H), 7.46 (s,1H), 7.42 (d,1H), 5.38 (s,2H), 3.88 (s,3H), 1.88 (s,3H), 1.83 (s,3H) |
| 6-005 | Me | Cl | Cl | Et | Et | |
| 6-006 | Me | Cl | Cl | H | Ph | |
| 6-007 | Me | Cl | Cl | H | CH₂OMe | |
| 6-008 | Me | Cl | Cl | H | CH₂cPr | |
| 6-009 | Me | Cl | Cl | H | CH₂CH₂OMe | |
| 6-010 | Me | Cl | Cl | Me | Ph | |
| 6-011 | Me | Cl | Cl | Me | CH₂OMe | |
| 6-012 | Me | Cl | Cl | Me | CH₂cPr | |
| 6-013 | Me | Cl | Cl | Me | CH₂CH₂OMe | |
| 6-014 | Me | Cl | SO₂Me | H | H | |
| 6-015 | Me | Cl | SO₂Me | H | Me | |
| 6-016 | Me | Cl | SO₂Me | H | Et | |
| 6-017 | Me | Cl | SO₂Me | Me | Me | (400 MHz, CDCl₃ δ, ppm) 8.14 (d,1H), 7.69 (d,1H), 7.27 (s,1H), 5.73 (s,2H), 3.89 (s,3H), 3.37 (s,3H), 1.84 (s,3H) |
| 6-018 | Me | Cl | SO₂Me | Et | Et | |
| 6-019 | Me | Cl | SO₂Me | H | Ph | |
| 6-020 | Me | Cl | SO₂Me | H | CH₂OMe | |
| 6-021 | Me | Cl | SO₂Me | H | CH₂cPr | |
| 6-022 | Me | Cl | SO₂Me | H | CH₂CH₂OMe | |
| 6-023 | Me | Cl | SO₂Me | Me | Ph | |
| 6-024 | Me | Cl | SO₂Me | Me | CH₂OMe | |
| 6-025 | Me | Cl | SO₂Me | Me | CH₂cPr | |
| 6-026 | Me | Cl | SO₂Me | Me | CH₂CH₂OMe | |
| 6-027 | Me | SO₂Me | Cl | H | H | |
| 6-028 | Me | SO₂Me | Cl | H | Me | |
| 6-029 | Me | SO₂Me | Cl | H | Et | |
| 6-030 | Me | SO₂Me | Cl | Me | Me | |
| 6-031 | Me | SO₂Me | Cl | Et | Et | |
| 6-032 | Me | SO₂Me | Cl | H | Ph | |
| 6-033 | Me | SO₂Me | Cl | H | CH₂OMe | |
| 6-034 | Me | SO₂Me | Cl | H | CH₂cPr | |
| 6-035 | Me | SO₂Me | Cl | H | CH₂CH₂OMe | |
| 6-036 | Me | SO₂Me | Cl | Me | Ph | |
| 6-037 | Me | SO₂Me | Cl | Me | CH₂OMe | |
| 6-038 | Me | SO₂Me | Cl | Me | CH₂cPr | |
| 6-039 | Me | SO₂Me | Cl | Me | CH₂CH₂OMe | |
| 6-040 | Et | Cl | Cl | H | H | |
| 6-041 | Et | Cl | Cl | H | Me | |
| 6-042 | Et | Cl | Cl | H | Et | |
| 6-043 | Et | Cl | Cl | Me | Me | |
| 6-044 | Et | Cl | CI | Et | Et | |
| 6-045 | Et | Cl | Cl | H | Ph | |
| 6-046 | Et | Cl | Cl | H | CH₂OMe | |
| 6-047 | Et | Cl | Cl | H | CH₂cPr | |
| 6-048 | Et | Cl | Cl | H | CH₂CH₂OMe | |
| 6-049 | Et | Cl | Cl | Me | Ph | |
| 6-050 | Et | Cl | Cl | Me | CH₂OMe | |
| 6-051 | Et | Cl | Cl | Me | CH₂cPr | |
| 6-052 | Et | Cl | Cl | Me | CH₂CH₂OMe | |
| 6-053 | Et | Cl | SO₂Me | H | H | |
| 6-054 | Et | Cl | SO₂Me | H | Me | |
| 6-055 | Et | Cl | SO₂Me | H | Et | |
| 6-056 | Et | Cl | SO₂Me | Me | Me | |
| 6-057 | Et | Cl | SO₂Me | Et | Et | |
| 6-058 | Et | Cl | SO₂Me | H | Ph | |
| 6-059 | Et | CI | SO₂Me | H | CH₂OMe | |
| 6-060 | Et | Cl | SO₂Me | H | CH₂cPr | |
| 6-061 | Et | Cl | SO₂Me | H | CH₂CH₂OMe | |
| 6-062 | Et | Cl | SO₂Me | Me | Ph | |
| 6-063 | Et | Cl | SO₂Me | Me | CH₂OMe | |
| 6-064 | Et | Cl | SO₂Me | Me | CH₂cPr | |
| 6-065 | Et | Cl | SO₂Me | Me | CH₂CH₂OMe | |
| 6-066 | Et | SO₂Me | Cl | H | H | |
| 6-067 | Et | SO₂Me | Cl | H | Me | |
| 6-068 | Et | SO₂Me | Cl | H | Et | |
| 6-069 | Et | SO₂Me | Cl | Me | Me | |
| 6-070 | Et | SO₂Me | Cl | Et | Et | |
| 6-071 | Et | SO₂Me | Cl | H | Ph | |
| 6-072 | Et | SO₂Me | Cl | H | CH₂OMe | |
| 6-073 | Et | SO₂Me | Cl | H | CH₂cPr | |
| 6-074 | Et | SO₂Me | Cl | H | CH₂CH₂OMe | |
| 6-075 | Et | SO₂Me | Cl | Me | Ph | |
| 6-076 | Et | SO₂Me | Cl | Me | CH₂OMe | |
| 6-077 | Et | SO₂Me | Cl | Me | CH₂cPr | |
| 6-078 | Et | SO₂Me | Cl | Me | CH₂CH₂OMe | |
| 6-079 | nPr | Cl | Cl | H | H | |
| 6-080 | nPr | Cl | Cl | H | Me | |
| 6-081 | nPr | Cl | Cl | H | Et | |
| 6-082 | nPr | Cl | Cl | Me | Me | |
| 6-083 | nPr | Cl | Cl | Et | Et | |
| 6-084 | nPr | Cl | Cl | H | Ph | |
| 6-085 | nPr | Cl | Cl | H | CH₂OMe | |
| 6-086 | nPr | Cl | Cl | H | CH₂cPr | |
| 6-087 | nPr | Cl | Cl | H | CH₂CH₂OMe | |
| 6-088 | nPr | Cl | Cl | Me | Ph | |
| 6-089 | nPr | Cl | Cl | Me | CH₂OMe | |
| 6-090 | nPr | Cl | Cl | Me | CH₂cPr | |
| 6-091 | nPr | Cl | Cl | Me | CH₂CH₂OMe | |
| 6-092 | nPr | Cl | SO₂Me | H | H | |
| 6-093 | nPr | Cl | SO₂Me | H | Me | |
| 6-094 | nPr | Cl | SO₂Me | H | Et | |
| 6-095 | nPr | Cl | SO₂Me | Me | Me | |
| 6-096 | nPr | Cl | SO₂Me | Et | Et | |
| 6-097 | nPr | Cl | SO₂Me | H | Ph | |
| 6-098 | nPr | Cl | SO₂Me | H | CH₂OMe | |
| 6-099 | nPr | Cl | SO₂Me | H | CH₂cPr | |
| 6-100 | nPr | Cl | SO₂Me | H | CH₂CH₂OMe | |
| 6-101 | nPr | Cl | SO₂Me | Me | Ph | |
| 6-102 | nPr | Cl | SO₂Me | Me | CH₂OMe | |
| 6-103 | nPr | Cl | SO₂Me | Me | CH₂cPr | |
| 6-104 | nPr | Cl | SO₂Me | Me | CH₂CH₂OMe | |
| 6-105 | nPr | SO₂Me | Cl | H | H | |
| 6-106 | nPr | SO₂Me | Cl | H | Me | |
| 6-107 | nPr | SO₂Me | Cl | H | Et | |
| 6-108 | nPr | SO₂Me | Cl | Me | Me | |
| 6-109 | nPr | SO₂Me | Cl | Et | Et | |
| 6-110 | nPr | SO₂Me | Cl | H | Ph | |
| 6-111 | nPr | SO₂Me | Cl | H | CH₂OMe | |
| 6-112 | nPr | SO₂Me | Cl | H | CH₂cPr | |
| 6-113 | nPr | SO₂Me | Cl | H | CH₂CH₂OMe | |
| 6-114 | nPr | SO₂Me | Cl | Me | Ph | |
| 6-115 | nPr | SO₂Me | Cl | Me | CH₂OMe | |
| 6-116 | nPr | SO₂Me | Cl | Me | CH₂cPr | |
| 6-117 | nPr | SO₂Me | Cl | Me | CH₂CH₂OMe | |

**Tabelle 7: Erfindungsgemäße Verbindungen der Formel (I), worin Q für Q3, R für CH₂-O-N=CR²R³ steht, und die anderen Reste die in Tabelle 7 angegebenen Bedeutungen haben**

| | | | | | | |
|---|---|---|---|---|---|---|
| 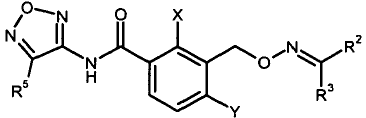 | | | | | | |

| Nr. | R⁵ | X | Y | R² | R³ | Physikal. Daten (¹H-NMR) |
|---|---|---|---|---|---|---|
| 7-001 | Me | Cl | Cl | H | H | |
| 7-002 | Me | Cl | Cl | H | Me | |
| 7-003 | Me | Cl | Cl | H | Et | |
| 7-004 | Me | Cl | Cl | Me | Me | |
| 7-005 | Me | Cl | Cl | Et | Et | |
| 7-006 | Me | Cl | Cl | H | Ph | |
| 7-007 | Me | Cl | Cl | H | CH₂OMe | |
| 7-008 | Me | Cl | Cl | H | CH₂cPr | |
| 7-009 | Me | Cl | Cl | H | CH₂CH₂OMe | |
| 7-010 | Me | Cl | Cl | Me | Ph | |
| 7-011 | Me | Cl | Cl | Me | CH₂OMe | |
| 7-012 | Me | Cl | Cl | Me | CH₂cPr | |
| 7-013 | Me | Cl | Cl | Me | CH₂CH₂OMe | |
| 7-014 | Me | Cl | SO₂Me | H | H | |
| 7-015 | Me | CI | SO₂Me | H | Me | |
| 7-016 | Me | Cl | SO₂Me | H | Et | |
| 7-017 | Me | Cl | SO₂Me | Me | Me | (400 MHz, CDCl₃ δ, ppm) 8.60 (br,s,1H), 8.00 (d,1H), 7.72 (d,1H), 5.69 (s,2H), 3.33 (s,3H), 2.52 (s,3H), 1.82 (s,3H), 181 (s,3H) |
| 7-018 | Me | Cl | SO₂Me | Et | Et | |
| 7-019 | Me | Cl | SO₂Me | H | Ph | |
| 7-020 | Me | Cl | SO₂Me | H | CH₂OMe | |
| 7-021 | Me | CI | SO₂Me | H | CH₂cPr | |
| 7-022 | Me | Cl | SO₂Me | H | CH₂CH₂OMe | |
| 7-023 | Me | Cl | SO₂Me | Me | Ph | |
| 7-024 | Me | Cl | SO₂Me | Me | CH₂OMe | |
| 7-025 | Me | Cl | SO₂Me | Me | CH₂cPr | |
| 7-026 | Me | Cl | SO₂Me | Me | CH₂CH₂OMe | |
| 7-027 | Me | SO₂Me | Cl | H | H | |
| 7-028 | Me | SO₂Me | Cl | H | Me | |
| 7-029 | Me | SO₂Me | Cl | H | Et | |
| 7-030 | Me | SO₂Me | Cl | Me | Me | |
| 7-031 | Me | SO₂Me | Cl | Et | Et | |
| 7-032 | Me | SO₂Me | Cl | H | Ph | |
| 7-033 | Me | SO₂Me | Cl | H | CH₂OMe | |
| 7-034 | Me | SO₂Me | Cl | H | CH₂cPr | |
| 7-035 | Me | SO₂Me | Cl | H | CH₂CH₂OMe | |
| 7-036 | Me | SO₂Me | Cl | Me | Ph | |
| 7-037 | Me | SO₂Me | Cl | Me | CH₂OMe | |
| 7-038 | Me | SO₂Me | Cl | Me | CH₂cPr | |
| 7-039 | Me | SO₂Me | Cl | Me | CH₂CH₂OMe | |
| 7-040 | OMe | Cl | Cl | H | H | |
| 7-041 | OMe | Cl | Cl | H | Me | |
| 7-042 | OMe | Cl | Cl | H | Et | |
| 7-043 | OMe | Cl | Cl | Me | Me | |
| 7-044 | OMe | Cl | Cl | Et | Et | |
| 7-045 | OMe | Cl | Cl | H | Ph | |
| 7-046 | OMe | Cl | Cl | H | CH₂OMe | |
| 7-047 | OMe | Cl | Cl | H | CH₂cPr | |
| 7-048 | OMe | Cl | Cl | H | CH₂CH₂OMe | |
| 7-049 | OMe | Cl | Cl | Me | Ph | |
| 7-050 | OMe | Cl | Cl | Me | CH₂OMe | |
| 7-051 | OMe | Cl | Cl | Me | CH₂cPr | |
| 7-052 | OMe | Cl | Cl | Me | CH₂CH₂OMe | |
| 7-053 | OMe | Cl | SO₂Me | H | H | |
| 7-054 | OMe | Cl | SO₂Me | H | Me | |
| 7-055 | OMe | Cl | SO₂Me | H | Et | |
| 7-056 | OMe | Cl | SO₂Me | Me | Me | |
| 7-057 | OMe | Cl | SO₂Me | Et | Et | |
| 7-058 | OMe | Cl | SO₂Me | H | Ph | |
| 7-059 | OMe | Cl | SO₂Me | H | CH₂OMe | |
| 7-060 | OMe | Cl | SO₂Me | H | CH₂cPr | |
| 7-061 | OMe | Cl | SO₂Me | H | CH₂CH₂OMe | |
| 7-062 | OMe | Cl | SO₂Me | Me | Ph | |
| 7-063 | OMe | Cl | SO₂Me | Me | CH₂OMe | |
| 7-064 | OMe | Cl | SO₂Me | Me | CH₂cPr | |
| 7-065 | OMe | Cl | SO₂Me | Me | CH₂CH₂OMe | |
| 7-066 | OMe | SO₂Me | Cl | H | H | |
| 7-067 | OMe | SO₂Me | Cl | H | Me | |
| 7-068 | OMe | SO₂Me | Cl | H | Et | |
| 7-069 | OMe | SO₂Me | Cl | Me | Me | |
| 7-070 | OMe | SO₂Me | Cl | Et | Et | |
| 7-071 | OMe | SO₂Me | Cl | H | Ph | |
| 7-072 | OMe | SO₂Me | Cl | H | CH₂OMe | |
| 7-073 | OMe | SO₂Me | Cl | H | CH₂cPr | |
| 7-074 | OMe | SO₂Me | Cl | H | CH₂CH₂OMe | |
| 7-075 | OMe | SO₂Me | Cl | Me | Ph | |
| 7-076 | OMe | SO₂Me | Cl | Me | CH₂OMe | |
| 7-077 | OMe | SO₂Me | Cl | Me | CH₂cPr | |
| 7-078 | OMe | SO₂Me | Cl | Me | CH₂CH₂OMe | |
| 7-079 | Cl | Cl | Cl | H | H | |
| 7-080 | Cl | Cl | Cl | H | Me | |
| 7-081 | Cl | Cl | Cl | H | Et | |
| 7-082 | Cl | Cl | Cl | Me | Me | |
| 7-083 | Cl | Cl | Cl | Et | Et | |
| 7-084 | Cl | Cl | Cl | H | Ph | |
| 7-085 | Cl | Cl | Cl | H | CH₂OMe | |
| 7-086 | Cl | Cl | Cl | H | CH₂cPr | |
| 7-087 | Cl | Cl | Cl | H | CH₂CH₂OMe | |
| 7-088 | Cl | Cl | Cl | Me | Ph | |
| 7-089 | Cl | Cl | Cl | Me | CH₂OMe | |
| 7-090 | Cl | Cl | Cl | Me | CH₂cPr | |
| 7-091 | Cl | Cl | Cl | Me | CH₂CH₂OMe | |
| 7-092 | Cl | Cl | SO₂Me | H | H | |
| 7-093 | Cl | Cl | SO₂Me | H | Me | |
| 7-094 | Cl | Cl | SO₂Me | H | Et | |
| 7-095 | Cl | Cl | SO₂Me | Me | Me | |
| 7-096 | Cl | Cl | SO₂Me | Et | Et | |
| 7-097 | Cl | Cl | SO₂Me | H | Ph | |
| 7-098 | Cl | Cl | SO₂Me | H | CH₂OMe | |
| 7-099 | Cl | Cl | SO₂Me | H | CH₂cPr | |
| 7-100 | Cl | Cl | SO₂Me | H | CH₂CH₂OMe | |
| 7-101 | Cl | Cl | SO₂Me | Me | Ph | |
| 7-102 | Cl | Cl | SO₂Me | Me | CH₂OMe | |
| 7-103 | Cl | Cl | SO₂Me | Me | CH₂cPr | |
| 7-104 | Cl | Cl | SO₂Me | Me | CH₂CH₂OMe | |
| 7-105 | Cl | SO₂Me | Cl | H | H | |
| 7-106 | Cl | SO₂Me | Cl | H | Me | |
| 7-107 | Cl | SO₂Me | Cl | H | Et | |
| 7-108 | Cl | SO₂Me | Cl | Me | Me | |
| 7-109 | Cl | SO₂Me | Cl | Et | Et | |
| 7-110 | Cl | SO₂Me | Cl | H | Ph | |
| 7-111 | Cl | SO₂Me | Cl | H | CH₂OMe | |
| 7-112 | Cl | SO₂Me | Cl | H | CH₂cPr | |
| 7-113 | Cl | SO₂Me | Cl | H | CH₂CH₂OMe | |
| 7-114 | Cl | SO₂Me | Cl | Me | Ph | |
| 7-115 | Cl | SO₂Me | Cl | Me | CH₂OMe | |
| 7-116 | Cl | SO₂Me | Cl | Me | CH₂cPr | |
| 7-117 | Cl | SO₂Me | Cl | Me | CH₂CH₂OMe | |

**Tabelle 8: Erfindungsgemäße Verbindungen der Formel (I), worin Q für Q4, R für CH₂-O-N=CR²R³ steht, und die anderen Reste die in Tabelle 8 angegebenen Bedeutungen haben**

| 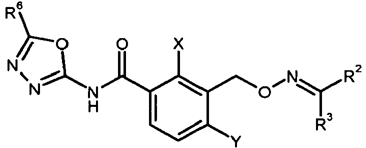 | | | | | | |
|---|---|---|---|---|---|---|
| Nr. | R⁶ | X | Y | R² | R³ | Physikal. Daten (¹H-NMR) |
| 8-001 | Me | Cl | Cl | H | H | |
| 8-002 | Me | Cl | Cl | H | Me | |
| 8-003 | Me | Cl | Cl | H | Et | |
| 8-004 | Me | Cl | Cl | Me | Me | |
| 8-005 | Me | Cl | Cl | Et | Et | |
| 8-006 | Me | Cl | Cl | H | Ph | |
| 8-007 | Me | Cl | Cl | H | CH₂OMe | |
| 8-008 | Me | Cl | Cl | H | CH₂cPr | |
| 8-009 | Me | Cl | Cl | H | CH₂CH₂OMe | |
| 8-010 | Me | Cl | Cl | Me | Ph | |
| 8-011 | Me | Cl | Cl | Me | CH₂OMe | |
| 8-012 | Me | Cl | Cl | Me | CH₂cPr | |
| 8-013 | Me | Cl | Cl | Me | CH₂CH₂OMe | |
| 8-014 | Me | Cl | SO₂Me | H | H | 400 MHz, DMSO-d₆, 12.46 (bs, 1H); 8.11 (d, 1H), 7,.92 (d, 1H), 7.13 (d, 1H), 6.71 (d, 1 H), 5.70 (s, 2H), 3.38 (s, 3H), 2.50 (s, 3H) |
| 8-015 | Me | Cl | SO₂Me | H | Me | 400 MHz, DMSO-d₆, 12.49 (bs, 1H); 8.11 (d, 1H), 7.91 (d, 1H), 7,.50 (q, 1 H), 5.67 (s, 2H), 3.39 (s, 3H), 2.50 (s, 3H), 1.78 (d, 3H) |
| 8-016 | Me | Cl | SO₂Me | H | Et | |

| Nr. | R⁶ | X | Y | R² | R³ | Physikal. Daten (¹H-NMR) |
|---|---|---|---|---|---|---|
| 8-017 | Me | Cl | SO₂Me | Me | Me | 400 MHz, DMSO-d₆, 12.45 (bs, 1H); 8.10 (d, 1H), 7,91 (d, 1H), 5.60 (s, 2H), 3.43 (s, 3H), 2.50 (s, 3H), 1.81 (s, 3H), 1.77 (s, 3H). |
| 8-018 | Me | Cl | SO₂Me | Et | Et | |
| 8-019 | Me | Cl | SO₂Me | H | Ph | |
| 8-020 | Me | Cl | SO₂Me | H | CH₂OMe | |
| 8-021 | Me | Cl | SO₂Me | H | CH₂cPr | |
| 8-022 | Me | Cl | SO₂Me | H | CH₂CH₂OMe | |
| 8-023 | Me | Cl | SO₂Me | Me | Ph | |
| 8-024 | Me | Cl | SO₂Me | Me | CH₂OMe | |
| 8-025 | Me | Cl | SO₂Me | Me | CH₂cPr | |
| 8-026 | Me | Cl | SO₂Me | Me | CH₂CH₂OMe | |
| 8-027 | Me | SO₂Me | Cl | H | H | |
| 8-028 | Me | SO₂Me | Cl | H | Me | |
| 8-029 | Me | SO₂Me | Cl | H | Et | |
| 8-030 | Me | SO₂Me | Cl | Me | Me | |
| 8-031 | Me | SO₂Me | Cl | Et | Et | |
| 8-032 | Me | SO₂Me | Cl | H | Ph | |
| 8-033 | Me | SO₂Me | Cl | H | CH₂OMe | |
| 8-034 | Me | SO₂Me | Cl | H | CH₂cPr | |
| 8-035 | Me | SO₂Me | Cl | H | CH₂CH₂OMe | |
| 8-036 | Me | SO₂Me | Cl | Me | Ph | |
| 8-037 | Me | SO₂Me | Cl | Me | CH₂OMe | |
| 8-038 | Me | SO₂Me | Cl | Me | CH₂cPr | |
| 8-039 | Me | SO₂Me | Cl | Me | CH₂CH₂OMe | |
| 8-040 | Et | Cl | Cl | H | H | |
| 8-041 | Et | Cl | Cl | H | Me | |
| 8-042 | Et | Cl | Cl | H | Et | |
| 8-043 | Et | Cl | Cl | Me | Me | |
| 8-044 | Et | Cl | Cl | Et | Et | |
| 8-045 | Et | Cl | Cl | H | Ph | |
| 8-046 | Et | Cl | Cl | H | CH₂OMe | |
| 8-047 | Et | Cl | Cl | H | CH₂cPr | |
| 8-048 | Et | Cl | Cl | H | CH₂CH₂OMe | |
| 8-049 | Et | Cl | Cl | Me | Ph | |
| 8-050 | Et | Cl | Cl | Me | CH₂OMe | |
| 8-051 | Et | Cl | Cl | Me | CH₂cPr | |
| 8-052 | Et | Cl | Cl | Me | CH₂CH₂OMe | |
| 8-053 | Et | Cl | SO₂Me | H | H | |
| 8-054 | Et | Cl | SO₂Me | H | Me | |
| 8-055 | Et | Cl | SO₂Me | H | Et | |
| 8-056 | Et | Cl | SO₂Me | Me | Me | |
| 8-057 | Et | Cl | SO₂Me | Et | Et | |
| 8-058 | Et | Cl | SO₂Me | H | Ph | |
| 8-059 | Et | Cl | SO₂Me | H | CH₂OMe | |
| 8-060 | Et | Cl | SO₂Me | H | CH₂cPr | |
| 8-061 | Et | Cl | SO₂Me | H | CH₂CH₂OMe | |
| 8-062 | Et | Cl | SO₂Me | Me | Ph | |
| 8-063 | Et | Cl | SO₂Me | Me | CH₂OMe | |
| 8-064 | Et | Cl | SO₂Me | Me | CH₂cPr | |
| 8-065 | Et | Cl | SO₂Me | Me | CH₂CH₂OMe | |
| 8-066 | Et | SO₂Me | Cl | H | H | |
| 8-067 | Et | SO₂Me | Cl | H | Me | |
| 8-068 | Et | SO₂Me | Cl | H | Et | |
| 8-069 | Et | SO₂Me | Cl | Me | Me | |
| 8-070 | Et | SO₂Me | Cl | Et | Et | |
| 8-071 | Et | SO₂Me | Cl | H | Ph | |
| 8-072 | Et | SO₂Me | Cl | H | CH₂OMe | |
| 8-073 | Et | SO₂Me | Cl | H | CH₂cPr | |
| 8-074 | Et | SO₂Me | Cl | H | CH₂CH₂OMe | |
| 8-075 | Et | SO₂Me | Cl | Me | Ph | |
| 8-076 | Et | SO₂Me | Cl | Me | CH₂OMe | |
| 8-077 | Et | SO₂Me | Cl | Me | CH₂cPr | |
| 8-078 | Et | SO₂Me | Cl | Me | CH₂CH₂OMe | |
| 8-079 | CH₂OMe | Cl | Cl | H | H | |
| 8-080 | CH₂OMe | Cl | Cl | H | Me | |
| 8-081 | CH₂OMe | Cl | Cl | H | Et | |
| 8-082 | CH₂OMe | Cl | Cl | Me | Me | |
| 8-083 | CH₂OMe | Cl | Cl | Et | Et | |
| 8-084 | CH₂OMe | Cl | Cl | H | Ph | |
| 8-085 | CH₂OMe | Cl | Cl | H | CH₂OMe | |
| 8-086 | CH₂OMe | Cl | Cl | H | CH₂cPr | |
| 8-087 | CH₂OMe | Cl | Cl | H | CH₂CH₂OMe | |
| 8-088 | CH₂OMe | Cl | Cl | Me | Ph | |
| 8-089 | CH₂OMe | Cl | Cl | Me | CH₂OMe | |
| 8-090 | CH₂OMe | Cl | Cl | Me | CH₂cPr | |
| 8-091 | CH₂OMe | Cl | Cl | Me | CH₂CH₂OMe | |
| 8-092 | CH₂OMe | Cl | SO₂Me | H | H | |
| 8-093 | CH₂OMe | Cl | SO₂Me | H | Me | |
| 8-094 | CH₂OMe | Cl | SO₂Me | H | Et | |
| 8-095 | CH₂OMe | Cl | SO₂Me | Me | Me | |
| 8-096 | CH₂OMe | Cl | SO₂Me | Et | Et | |
| 8-097 | CH₂OMe | Cl | SO₂Me | H | Ph | |
| 8-098 | CH₂OMe | Cl | SO₂Me | H | CH₂OMe | |
| 8-099 | CH₂OMe | Cl | SO₂Me | H | CH₂cPr | |
| 8-100 | CH₂OMe | Cl | SO₂Me | H | CH₂CH₂OMe | |
| 8-101 | CH₂OMe | Cl | SO₂Me | Me | Ph | |
| 8-102 | CH₂OMe | Cl | SO₂Me | Me | CH₂OMe | |
| 8-103 | CH₂OMe | Cl | SO₂Me | Me | CH₂cPr | |
| 8-104 | CH₂OMe | Cl | SO₂Me | Me | CH₂CH₂OMe | |
| 8-105 | CH₂OMe | SO₂Me | Cl | H | H | |
| 8-106 | CH₂OMe | SO₂Me | Cl | H | Me | |
| 8-107 | CH₂OMe | SO₂Me | Cl | H | Et | |
| 8-108 | CH₂OMe | SO₂Me | Cl | Me | Me | |
| 8-109 | CH₂OMe | SO₂Me | Cl | Et | Et | |
| 8-110 | CH₂OMe | SO₂Me | Cl | H | Ph | |
| 8-111 | CH₂OMe | SO₂Me | Cl | H | CH₂OMe | |
| 8-112 | CH₂OMe | SO₂Me | Cl | H | CH₂cPr | |
| 8-113 | CH₂OMe | SO₂Me | Cl | H | CH₂CH₂OMe | |
| 8-114 | CH₂OMe | SO₂Me | Cl | Me | Ph | |
| 8-115 | CH₂OMe | SO₂Me | Cl | Me | CH₂OMe | |
| 8-116 | CH₂OMe | SO₂Me | Cl | Me | CH₂cPr | |
| 8-117 | CH₂OMe | SO₂Me | Cl | Me | CH₂CH₂OMe | |

Im Falle von Mischungen von E- und Z-Isomeren werden nur die Signale des hauptsächlich entstandenen E Isomers angegeben.

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontroll-pflanzen). Dabei zeigen beispielsweise die Verbindungen der Nr. 1-002, 1-004, 1-007, 1-008, 1-043, 1-044, 1-046, 1-047, 1-060, 1-079, 1-080, 1-082, 1-083, 1-085, 1-095, 1-096, 1-098, 1-099, 1-059, 2-015, 3-015, 3-018, 3-020, 4-001, 4-118, 5-004, 5-017, 5-043, 5-056, 5-082, 5-095, 6-017, 7-017, 8-017 und 8-014 bei einer Aufwandmenge von 320 g/ha jeweils eine mindestens 80 %-ige Wirkung gegen eine Abuthilon theophrasti und Amaranthus retroflexus. Die Verbindungen der Nr. 1-001, 1-005, 1-014, 1-015, 1-017, 1-018, 1-020, 1-033, 1-040, 1-041, 1-053, 1-054, 1-056, 1-057, 1-092, 1-093, 3-014, 3-015, 8-014, 8-017 und 8-015 zeigen bei einer Aufwandmenge von 320 g/ha jeweils eine mindestens 80 %-ige Wirkung gegen Setaria viridis und Stellaria media. Die Verbindungen der Nr. 3-017, 4-092 und 6-004 zeigen bei einer Aufwandmenge von 320 g/ha jeweils eine mindestens 80 %-ige Wirkung gegen Amaranthus retroflexus und Veronica persica.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen beispielsweise die Verbindungen der Nr. 1-001, 1-002, 1-004, 1-005, 1-007, 1-008, 1-014, 1-015, 1-017, 1-018, 1-020, 1-033, 1-040, 1-041, 1-043, 1-044, 1-046, 1-047, 1-053, 1-054, 1-056, 1-057, 1-059, 1-060, 1-079, 1-080, 1-082, 1-083, 1-086, 1-092, 1-093, 1-095, 1-096, 1-098, 1-099, 2-015, 3-014, 3-015, 3-017, 3-018, 3-020, 5-082, 5-095, 6-017, 7-017 und 8-014 bei einer Aufwandmenge von 80 g/ha jeweils eine mindestens 80 %-ige Wirkung gegen eine Abuthilon theophrasti, Amaranthus retroflexus und Stellaria media. Die Verbindungen der Nr. 4-092, 4-093, 5-004, 5-017, 5-043 und 5-056 zeigen bei einer Aufwandmenge von 80 g/ha jeweils eine mindestens 80 %-ige Wirkung gegen Echinocloa crus galli, Pharbitis purpureum und Stellaria media.

## Patentansprüche

1. Benzamide der Formel (I) oder deren Salze 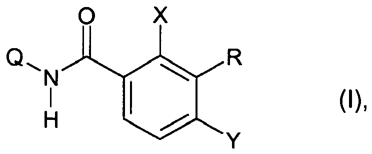 worin die Substituenten folgende Bedeutungen haben:
Q bedeutet einen Rest Q1, Q2, Q3 oder Q4, 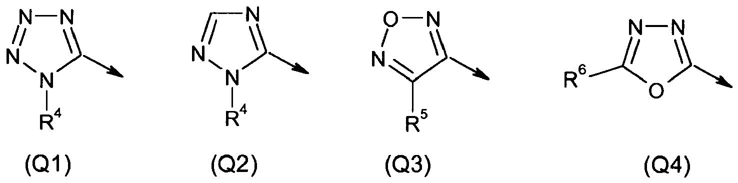
R bedeutet -CH=N-OR¹, -CH₂-O-N=CR²R³,
X bedeutet Nitro, Halogen, Cyano, Formyl, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(Ci-C₆)-alkyl, Halogen-(C₃-C₆)-cycloalkyl-(C₁-C₆)-alkyl, COR⁷, COOR⁷, OCOOR⁸, NR⁷COOR⁸, C(O)N(R⁷)₂, NR⁷C(O)N(R⁷)₂, OC(O)N(R⁷)₂, C(O)NR⁷OR⁷, OR⁷, OCOR⁷, OSO₂R⁸, S(O)ₙR⁸, SO₂OR⁷, SO₂N(R⁷)₂, NR⁷SO₂R⁸, NR⁷OR⁷, (C₁-C₆)-Alkyl-S(O)ₙR⁸, (C₁-C₆)-Alkyl-OR⁷, (C₁-C₆)-Alkyl-OCOR⁷, (C₁-C₆)-Alkyl-OSO₂R⁸, (C₁-C₆)-Alkyl-CO₂R⁷, (C₁-C₆)-Alkyl-SO₂OR⁸, (C₁-C₆)-Alkyl-CON(R⁷)₂, (C₁-C₆)-Alkyl-SO₂N(R⁷)₂, (C₁-C₆)-Alkyl-NR⁷COR⁷, (C₁-C₆)-Alkyl-NR⁷SO₂R⁸, N(R⁷)₂, P(O)(OR⁹)₂, CH₂P(O)(OR⁹)₂, Heteroaryl, Heterocyclyl, Phenyl, (C₁-C₆)-Alkyl-Heteroaryl oder (C₁-C₆)-Alkyl-Heterocyclyl, wobei die letzten fünf letztgenannten Reste jeweils durch s Reste aus der Gruppe bestehend aus Halogen, Nitro, Cyano, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Y bedeutet Nitro, Halogen, Cyano, Formyl, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR⁷, COOR⁷, OCOOR⁸, NR¹COOR⁸, C(O)N(R⁷)₂, NR⁷C(O)N(R⁷)₂, OC(O)N(R⁷)₂, C(O)NR⁷OR⁷, OR⁷, OSO₂R⁸, S(O)ₙR⁸, SO₂OR⁸, SO₂N(R⁷)₂, NR⁷SO₂R⁸, NR⁷COR⁷, (C₁-C₆)-Alkyl-S(O)ₙR⁸, (C₁-C₆)-Alkyl-OR⁷, (C₁-C₆)-Alkyl-OCOR⁷, (C₁-C₆)-Alkyl-OSO₂R⁸, (C₁-C₆)-Alkyl-CO₂R⁷, (C₁-C₆)-Alkyl-SO₂OR⁷, (C₁-C₆)-Alkyl-CON(R⁷)₂, (C₁-C₆)-Alkyl-SO₂N(R⁷)₂, (C₁-C₆)-Alkyl-NR⁷COR⁷ (C₁-C₆)-Alkyl-NR⁷SO₂R⁸, N(R⁷)₂, P(O)(OR⁹)₂, Heteroaryl, Heterocyclyl oder Phenyl,
wobei die drei letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₃-C₆)-Cycloalkyl-(C₁-C₆)-alkyl, wobei die letzten sechs Reste jeweils durch s Reste aus der Gruppe bestehend aus Cyano, Halogen, Nitro, Rhodano, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ und (C₁-C₄)-Alkoxy-(C₂-C₆)-alkoxycarbonyl substituiert sind,
oder R¹ bedeutet Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR¹⁰-Heteroaryl, (C₁-C₆)-Alkyl-NR¹⁰-Heterocyclyl, wobei die zehn letztgenannten Reste jeweils durch s Reste aus der Gruppe bestehend aus Cyano, Halogen, Nitro, Rhodano, (C₁-C₆)-Alkyl, Halogen-(C₁-C₆)-Alkyl, OR¹⁰, S(O)ₙR¹⁰, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ und (C₁-C₄)-Alkoxy-(C₂-C₆)-alkoxycarbonyl substituiert sind, und wobei Heterocyclyl n Oxogruppen trägt,
R² und R³ bedeuten unabhängig voneinander jeweils Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, (C₁-C₆)-Alkoxy-(C₁-C₆)-alkyl, Halogen-(C₁-C₆)-Alkoxy-(C₁-C₆)-alkyl Phenyl, Heteroaryl oder Heterocyclyl,
wobei die drei letztgenannten Reste jeweils durch s Reste aus der Gruppe bestehend aus Cyano, Halogen, Nitro, Rhodano, (C₁-C₆)-Alkyl, Halogen-(C₁-C₆)-Alkyl, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ und (C₁-C₄)-Alkoxy-(C₂-C₆)-alkoxycarbonyl substituiert sind, und wobei Heterocyclyl n Oxogruppen trägt,
oder R² und R³ bilden gemeinsam mit dem Atom, an das sie gebunden sind, einen 5-bis 6-gliedrigen ungesättigten, teilgesättigten oder gesättigten Ring, der neben Kohlenstoffatomen jeweils n Sauerstoff- und Schwefelatome enthält,
R⁴ bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, wobei die sechs letztgenannten Reste jeweils durch s Reste aus der Gruppe Nitro, Cyano, SiR⁹₃, PO(OR⁹)₃, S(O)ₙ-(C₁-C₆)-Alkyl, (C₁-C₆)-Alkoxy, Halogen-(C₁-C₆)-alkoxy, N(R⁷)₂, COR⁷, COOR⁷, OCOR⁷, OCO₂R⁸, NR⁷COR⁷, NR⁷SO₂R⁸, (C₃-C₆)-Cycloalkyl, Heteroaryl, Heterocyclyl, Phenyl, W-Heteroaryl, W-Heterocyclyl, W-Phenyl oder W-Benzyl substituiert sind, wobei die sieben letztgenannten Reste selber wieder durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiert sind, und wobei Heterocyclyl n Oxogruppen trägt,
oder R⁴ bedeutet (C₃-C₇)-Cycloalkyl, Heteroaryl, Heterocyclyl oder Phenyl, wobei die vier letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiert sind,
R⁵ bedeutet Wasserstoff, (C₁-C₆)-Alkyl, Halogen-(C₁-C₆)-alkyl, (C₂-C₆)-Alkenyl, (C₂-C₆)-Halogenalkenyl, (C₂-C₆)-Alkinyl, (C₂-C₆)-Halogenalkinyl, (C₃-C₇)-cyclo-Alkyl, (C₁-C₆)-Alkoxy, Halogen-(C₁-C₆)-alkoxy, (C₂-C₆)-Alkenyloxy, (C₂-C₆)-Alkinyloxy, Cyano, Nitro, Methylsulfenyl, Methylsulfinyl, Methylsulfonyl, Acetylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder
jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiertes Heteroaryl, Heterocyclyl oder Phenyl und wobei Heterocyclyl n Oxogruppen trägt,
R⁶ bedeutet Wasserstoff, (C₁-C₆)-Alkyl, R⁷O-(C₁-C₆)-Alkyl, CH₂R¹², (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, OR⁷, NHR⁷, Methoxycarbonyl, Ethoxycarbonyl, Methoxycarbonylmethyl, Ethoxycarbonylmethyl, Methylcarbonyl, Trifluormethylcarbonyl, Dimethylamino, Acetylamino, Methylsulfenyl, Methylsulfinyl, Methylsulfonyl oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes Heteroaryl, Heterocyclyl, Benzyl oder Phenyl, wobei Heterocyclyl n Oxogruppen trägt,
R⁷ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR¹⁰-Heteroaryl, (C₁-C₆)-Alkyl-NR¹⁰-Heterocyclyl wobei die 16 letztgenannten Reste jeweils durch s Reste aus der Gruppe bestehend aus Cyano, Halogen, Nitro, Rhodano, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ und (C₁-C₄)-Alkoxy-(C₂-C₆)-alkoxycarbonyl substituiert sind, und wobei Heterocyclyl n Oxogruppen trägt,
R⁸ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR¹⁰-Heteroaryl, (C₁-C₆)-Alkyl-NR¹⁰-Heterocyclyl, wobei die 16 letztgenannten Reste jeweils durch s Reste aus der Gruppe bestehend aus Cyano, Halogen, Nitro, Rhodano, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ und (C₁-C₄)-Alkoxy-(C₂-C₆)-alkoxycarbonyl substituiert sind, und wobei Heterocyclyl n Oxogruppen trägt,
R⁹ bedeutet Methyl oder Ethyl,
R¹⁰ bedeutet Wasserstoff, (C₁-C₆)-Alkyl, Halo-(C₁-C₆)-alkyl (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R¹¹ bedeutet (C₁-C₆)-Alkyl, (C₂-C₆)-Alkenyl oder (C₂-C₆)-Alkinyl,
R¹² bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₁-C₆)-Alkoxy, (C₃-C₆)-Cycloalkyl oder jeweils durch s Reste aus der Gruppe bestehend aus Methyl, Ethyl, Methoxy, Trifluormethyl und Halogen substituiertes Heteroaryl, Heterocyclyl oder Phenyl,
R¹³ bedeutet (C₁-C₆)-Alkyl, (C₂-C₆)-Alkenyl oder (C₂-C₆)-Alkinyl, Phenyl
W bedeutet O, S, oder NR¹³,
n bedeutet 0, 1 oder 2,
s bedeutet 0, 1, 2 oder 3.

2. Benzamide nach Anspruch 1, worin
Q bedeutet einen Rest Q1, Q2, Q3 oder Q4, 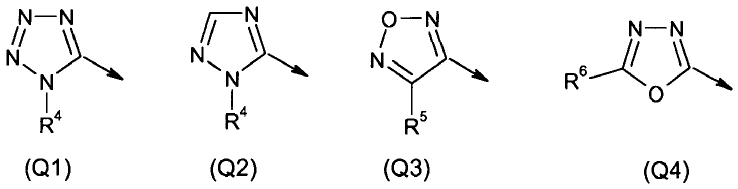
R bedeutet -CH=N-OR¹, -CH₂-O-N=CR²R³,
X bedeutet Nitro, Halogen, Cyano, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, OR⁷, S(O)ₙR⁸, SO₂N(R⁷)₂, NR⁷SO₂R⁸, (C₁-C₆)-Alkyl-S(O)ₙR⁸, (C₁-C₆)-Alkyl-OR⁷, (C₁-C₆)-Alkyl-CO₂R⁷, (C₁-C₆)-Alkyl-CON(R⁷)₂, (C₁-C₆)-Alkyl-SO₂N(R⁷)₂, (C₁-C₆)-Alkyl-NR⁷COR⁷, (C₁-C₆)-Alkyl-NR⁷SO₂R⁸, N(R⁷)₂, P(O)(OR⁹)₂, CH₂P(O)(OR⁹)₂, Heteroaryl, Heterocyclyl, Phenyl, (C₁-C₆)-Alkyl-Heteroaryl oder (C₁-C₆)-Alkyl-Heterocyclyl, wobei die fünf letztgenannten Reste jeweils durch s Reste aus der Gruppe bestehend aus Halogen, Nitro, Cyano, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Y bedeutet Nitro, Halogen, Cyano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, OR⁷, OSO₂R⁸, S(O)ₙR⁸, SO₂OR⁸, SO₂N(R⁷)₂, NR⁷SO₂R⁸, N(R⁷)₂, P(O)(OR⁹)₂, Heteroaryl, Heterocyclyl oder Phenyl, wobei die drei letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, wobei die fünf letztgenannten Reste jeweils durch s Reste aus der Gruppe bestehend aus Cyano, Halogen, Nitro, OR¹⁰, S(O)ₙR¹¹, COR¹⁰, OCOR¹⁰, NR¹⁰COR¹⁰, NR¹⁰SO₂R¹¹, CO₂R¹⁰, CON(R¹⁰)₂ und (C₁-C₄)-Alkoxy-(C₂-C₆)-alkoxycarbonyl substituiert sind,
oder R¹ bedeutet Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR¹⁰-Heteroaryl oder (C₁-C₆)-Alkyl-NR¹⁰-Heterocyclyl, wobei die zehn letztgenannten Reste jeweils durch s Reste aus der Gruppe bestehend aus Cyano, Halogen, Nitro, Rhodano, (C₁-C₆)-Alkyl, Halogen-(C₁-C₆)-Alkyl, OR¹⁰, S(O)ₙR¹¹, N(R¹⁰)₂, COR¹⁰, OCOR¹⁰, NR¹⁰COR¹⁰, NR¹⁰SO₂R¹¹CO₂R¹⁰, CON(R¹⁰)₂ und (C₁-C₄)-Alkoxy-(C₂-C₆)-alkoxycarbonyl substituiert sind, und wobei Heterocyclyl n Oxogruppen trägt,
R² und R³ bedeuten unabhängig voneinander jeweils Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkyl-(C₁-C₆)-alkyl, (C₁-C₆)-Alkoxy-(C₁-C₆)-alkyl, Halogen-(C₁-C₆)-Alkoxy-(C₁-C₆)-alkyl, Phenyl, Heteroaryl oder Heterocyclyl, wobei die drei letztgenannten Reste jeweils durch s Reste aus der Gruppe bestehend aus Cyano, Halogen, Nitro, (C₁-C₆)-Alkyl, Halogen-(C₁-C₆)-Alkyl, OR¹⁰, S(O)ₙR¹¹, N(R¹⁰)₂, COR¹⁰, NR¹⁰COR¹⁰, NR¹⁰SO₂R¹¹, CO₂R¹⁰, CON(R¹⁰)₂ und (C₁-C₄)-Alkoxy-(C₂-C₆)-alkoxycarbonyl substituiert sind, und wobei Heterocyclyl n Oxogruppen trägt,
oder R² und R³ bilden gemeinsam mit dem Atom, an das sie gebunden sind, einen 5-bis 6-gliedrigen ungesättigten, teilgesättigten oder gesättigten Ring, der neben Kohlenstoffatomen jeweils n Sauerstoff- und Schwefelatome enthält,
R⁴ bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, wobei die sechs letztgenannten Reste jeweils durch s Reste aus der Gruppe bestehend aus Nitro, Cyano, S(O)ₙ-(C₁-C₆)-Alkyl, (C₁-C₆)-Alkoxy, Halogen-(C₁-C₆)-alkoxy, COOR⁷, NR⁷COR⁷, NR⁷SO₂R⁸, (C₃-C₆)-Cycloalkyl, Heteroaryl, Heterocyclyl, Phenyl, W-Heteroaryl, W-Heterocyclyl, W-Phenyl und W-Benzyl substituiert sind, wobei die 7 letztgenannten Reste selber wieder jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiert sind, und wobei Heterocyclyl n Oxogruppen trägt, substituiert sind,
oder R⁴ bedeutet (C₃-C₇)-Cycloalkyl, Heteroaryl, Heterocyclyl oder Phenyl, wobei die vier letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiert sind,
R⁵ bedeutet Wasserstoff, (C₁-C₆)-Alkyl, Halogen-(C₁-C₆)-alkyl, (C₂-C₆)-Alkenyl, (C₂-C₆)-Halogenalkenyl, (C₂-C₆)-Alkinyl, (C₂-C₆)-Halogenalkinyl, (C₃-C₇)-cyclo-Alkyl, (C₁-C₆)-Alkoxy, Halogen-(C₁-C₆)-alkoxy, (C₂-C₆)-Alkenyloxy, (C₂-C₆)-Alkinyloxy, Cyano, Nitro, Methylsulfenyl, Methylsulfinyl, Methylsulfonyl, Acetylamino, Methoxycarbonyl, Methoxycarbonylmethyl, Methylcarbonyl, Trifluormethylcarbonyl, Halogen, Amino, Aminocarbonyl, Methylaminocarbonyl, Dimethylaminocarbonyl, Methoxymethyl, oder
jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiertes Heteroaryl, Heterocyclyl oder Phenyl, und wobei Heterocyclyl n Oxogruppen trägt,
R⁶ bedeutet Wasserstoff, (C₁-C₆)-Alkyl, R⁷O-(C₁-C₆)-Alkyl, CH₂R¹², (C₃-C₇)-Cycloalkyl, Halogen-(C₁-C₆)-alkyl, (C₂-C₆)-Alkenyl, (C₂-C₆)-Alkinyl, OR⁷, NHR⁷, Methoxycarbonyl, Methoxycarbonylmethyl, Methylcarbonyl, Trifluormethylcarbonyl, Dimethylamino, Acetylamino, Methylsulfenyl, Methylsulfinyl, Methylsulfonyl oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiertes Heteroaryl, Heterocyclyl, Benzyl oder Phenyl, und wobei Heterocyclyl n Oxogruppen trägt,
R⁷ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₃-C₆)-Cycloalkyl-(C₁-C₆)-alkyl, Phenyl, Heteroaryl, Heterocyclyl, wobei die neun letztgenannten Reste jeweils durch s Reste aus der Gruppe bestehend aus Cyano, Halogen, Nitro, OR¹⁰, S(O)ₙR¹¹, N(R¹⁰)₂, NR¹⁰SO₂R¹¹, CO₂R¹⁰, CON(R¹⁰)₂ und (C₁-C₄)-Alkoxy-(C₂-C₆)-alkoxycarbonyl substituiert sind, und wobei Heterocyclyl n Oxogruppen trägt,
R⁸ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₃-C₆)-Cycloalkyl-(C₁-C₆)-alkyl, Phenyl, Heteroaryl, Heterocyclyl, wobei die neun letztgenannten Reste jeweils durch s Reste aus der Gruppe bestehend aus Cyano, Halogen, Nitro, OR¹⁰, S(O)ₙR¹¹, N(R¹⁰)₂, NR¹⁰SO₂R¹¹, CO₂R¹⁰, CON(R¹⁰)₂ und (C₁-C₄)-Alkoxy-(C₂-C₆)-alkoxycarbonyl substituiert sind, und wobei Heterocyclyl n Oxogruppen trägt,
R⁹ bedeutet Methyl oder Ethyl,
R¹⁰ bedeutet Wasserstoff, (C₁-C₆)-Alkyl, Halo-(C₁-C₆)-alkyl (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R¹¹ bedeutet (C₁-C₆)-Alkyl, (C₂-C₆)-Alkenyl oder (C₂-C₆)-Alkinyl,
R¹² bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Trifluormethylcarbonyl, Aminocarbonyl, Methylaminocarbonyl, Dimethylaminocarbonyl, (C₁-C₆)-Alkoxy, (C₃-C₆)-Cycloalkyl oder jeweils durch s Reste aus der Gruppe bestehend aus Methyl, Methoxy, Trifluormethyl und Halogen substituiertes Heteroaryl, Heterocyclyl oder Phenyl,
R¹³ bedeutet (C₁-C₆)-Alkyl,
W bedeutet O, S oder NR¹³,
n bedeutet 0,1 oder 2,
s bedeutet 0, 1, 2 oder 3.

3. Benzamide nach Anspruch 1 oder 2, worin
Q bedeutet einen Rest Q1 oder Q2, 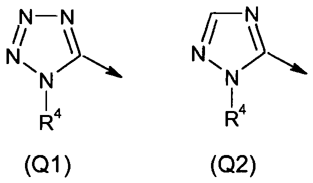
R bedeutet -CH=N-OR¹, -CH₂-O-N=CR²R³,
X bedeutet Nitro, Halogen, (C₁-C₆)-Alkyl, Halogen-(C₁-C₆)-alkyl, S(O)ₙR⁸, (C₁-C₆)-Alkyl-S(O)ₙR⁸, (C₁-C₆)-Alkyl-OR⁷, (C₁-C₆)-Alkyl-SO₂N(R⁷)₂ oder (C₁-C₆)-Alkyl-NR⁷SO₂R⁸,
Y bedeutet Nitro, Halogen, Halogen-(C₁-C₆)-alkyl oder S(O)ₙR⁸,
R¹ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl, wobei die fünf letztgenannten Reste jeweils durch s Reste aus der Gruppe bestehend aus Cyano, Halogen, OR¹⁰ und S(O)ₙR¹¹ substituiert sind,
R² und R³ bedeuten unabhängig voneinander jeweils Wasserstoff, (C₁-C₆)-Alkyl, Halogen-(C₁-C₆)-alkyl, (C₂-C₆)-Alkenyl, (C₂-C₆)-Alkinyl, (C₁-C₆)-Alkoxy-(C₁-C₆)-alkyl oder Halogen-(C₁-C₆)-Alkoxy-(C₁-C₆)-alkyl,
R⁴ bedeutet (C₁-C₆)-Alkyl, (C₂-C₆)-Alkenyl oder (C₂-C₆)-Alkinyl, wobei diese drei Reste jeweils durch s Reste (C₁-C₆)-Alkoxy substituiert sind,
R⁵ bedeutet (C₁-C₆)-Alkyl, (C₂-C₆)-Alkenyl, (C₂-C₆)-Alkinyl, (C₃-C₇)-cyclo-Alkyl, (C₁-C₆)-Alkoxy, (C₂-C₆)-Alkenyloxy, (C₂-C₆)-Alkinyloxy, Acetylamino, Halogen oder Methoxymethyl,
R⁶ bedeutet (C₁-C₆)-Alkyl, R⁷O-(C₁-C₆)-Alkyl, CH₂R¹², (C₃-C₇)-Cycloalkyl, (C₂-C₆)-Alkenyl oder (C₂-C₆)-Alkinyl,
R⁷ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁸ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkenyl oder (C₃-C₆)-Cycloalkyl-(C₁-C₆)-alkyl,
R⁹ bedeutet Methyl oder Ethyl,
R¹⁰ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R¹¹ bedeutet (C₁-C₆)-Alkyl, (C₂-C₆)-Alkenyl oder (C₂-C₆)-Alkinyl,
R¹² bedeutet Acetoxy, Acetamido oder (C₃-C₆)-Cycloalkyl,
R¹³ bedeutet (C₁-C₆)-Alkyl,
W bedeutet O, S oder NR¹³,
n bedeutet 0, 1 oder 2,
s bedeutet 0, 1, 2 oder 3.

4. Herbizide Mittel, **gekennzeichnet durch** einen herbizid wirksamen Gehalt an mindestens einer Verbindung der Formel (I) gemäß einem der Ansprüche 1 bis 3.

5. Herbizide Mittel nach Anspruch 4 in Mischung mit Formulierungshilfsmitteln.

6. Herbizide Mittel nach Anspruch 4 oder 5 enthaltend mindestens einen weiteren pestizid wirksamen Stoff aus der Gruppe Insektizide, Akarizide, Herbizide, Fungizide, Safener und Wachstumsregulatoren.

7. Herbizide Mittel nach Anspruch 6 enthaltend einen Safener.

8. Herbizide Mittel nach Anspruch 7 enthaltend cyprosulfamid, cloquintocet-mexyl, mefenpyr-diethyl oder isoxadifen-ethyl.

9. Herbizide Mittel nach einem der Ansprüche 6 bis 8 enthaltend ein weiteres Herbizid.

10. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder eines herbiziden Mittels nach einem der Ansprüche 4 bis 9 auf die Pflanzen oder auf den Ort des unerwünschten Pflanzenwachstums appliziert.

11. Verwendung von Verbindungen der Formel (I) gemäß einem der Ansprüche 1 bis 3 oder von herbiziden Mitteln nach einem der Ansprüche 4 bis 9 zur Bekämpfung unerwünschter Pflanzen.

12. Verwendung nach Anspruch 11, **dadurch gekennzeichnet, daß** die Verbindungen der Formel (I) zur Bekämpfung unerwünschter Pflanzen in Kulturen von Nutzpflanzen eingesetzt werden.

13. Verwendung nach Anspruch 12, **dadurch gekennzeichnet, daß** die Nutzpflanzen transgene Nutzpflanzen sind.

## Claims

1. Benzamides of the formula (I) or salts thereof 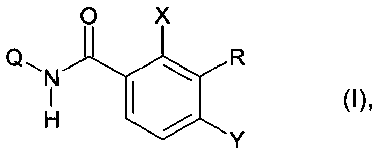 in which the substituents are defined as follows:
Q is a Q1, Q2, Q3 or Q4 radical, 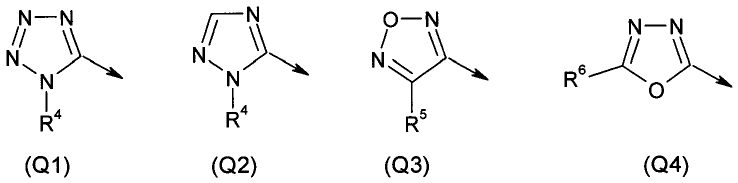
R is -CH=N-OR¹, -CH₂-O-N=CR²R³,
X is nitro, halogen, cyano, formyl, thiocyanato, (C₁-C₆)-alkyl, halo-(C₁-C₆)-alkyl, (C₂-C₆)-alkenyl, halo-(C₂-C₆)-alkenyl, (C₂-C₆)-alkynyl, halo- (C₃-C₆) -alkynyl, (C₃-C₆)-cycloalkyl, halo- (C₃-C₆) -cycloalkyl, (C₃-C₆)-cycloalkyl-(C₁-C₆)-alkyl, halo-(C₃-C₆)-cycloalkyl-(C₁-C₆-alkyl, COR⁷, COOR⁷, OCOOR⁸, NR⁷COOR⁸, C(O)N(R⁷)₂, NR⁷C (0) N (R⁷) ₂, OC(O)N(R⁷)2, C(O)NR⁷OR⁷, OR⁷, OCOR⁷, OSO₂R⁸, S(O)ₙR⁸, SO₂OR⁷, SO₂N(R⁷)₂, NR⁷SO₂R⁸, NR⁷OR⁷, (C₁-C₆)-alkylS(O)ₙR⁸, (C₁-C₆)-alkyl-OR⁷, (C₁-C₆-alkyl-OCOR⁷, (C₁-C₆)-alkyl-OSO₂R⁸, (C₁-C₆) -alkyl-CO₂R⁷, (C₁-C₆) -alkyl-SO₂OR⁸, (C₁-C₆) -alkyl-CON(R⁷)₂, (C₁-C₆) -alkyl-SO₂N (R⁷) ₂, (C₁-C₆)-alkyl-NR⁷COR⁷, (C₁-C₆)-alkyl-NR⁷SO₂R⁸, N(R⁷)₂, P(O)(OR⁹)₂, CH₂P(O) (OR⁹) ₂, heteroaryl, heterocyclyl, phenyl, (C₁-C₆)-alkyl-heteroaryl or (C₁-C₆)-alkyl-heterocyclyl, where the five last-mentioned radicals are each substituted by s radicals from the group consisting of halogen, nitro, cyano, (C₁-C₆)-alkyl, halo- (C₁-C₆) -alkyl, S(O)ₙ-(C₁-C₆)-alkyl, (C₁-C₆)-alkoxy and halo- (C₁-C₆) -alkoxy, and where heterocyclyl carries n oxo groups,
Y is nitro, halogen, cyano, formyl, thiocyanato, (C₁-C₆)-alkyl, halo-(C₁-C₆)-alkyl, (C₂-C₆)-alkenyl, halo-(C₂-C₆-alkenyl, (C₂-C₆) -alkynyl, halo- (C₂-C₆) -alkynyl, (C₃-C₆-cycloalkyl, halo- (C₃-C₆) -cycloalkyl, (C₃-C₆)-cycloalkyl-(C₁-C₆)-alkyl, halo-(C₃-C₆-cycloalkyl-(C₁-C₆-alkyl, COR⁷, COOR⁷, OCOOR⁸, NR¹COOR⁸, C(O)N(R⁷)₂, NR⁷C (0) N (R⁷) ₂, OC(O)N(R⁷)₂, C(O)NR⁷OR⁷, OR⁷, OSO₂R⁸, S(O)ₙR⁸, SO₂OR⁸, SO₂N(R⁷)₂, NR⁷SO₂R⁸, NR⁷COR⁷, (C₁-C₆)-alkyl-S(O)ₙR⁸, (C₁-C₆) -alkyl-OR⁷, (C₁-C₆) -alkyl-OCOR⁷, (C₁-C₆) -alkyl-OSO₂R⁸, (C₁-C₆) -alkyl-CO₂R⁷, (C₁-C₆) -alkyl-SO₂OR⁷, (C₁-C₆) -alkyl-CON (R⁷) ₂, (C₁-C₆) -alkyl-SO₂N (R⁷) ₂, (C₁-C₆) -alkyl-NR⁷COR⁷, (C₁-C₆) -alkyl-NR⁷SO₂R⁸, N(R⁷)₂, P(O)(OR⁹)₂, heteroaryl, heterocyclyl or phenyl, where the three last-mentioned radicals are each substituted by s radicals from the group consisting of halogen, nitro, cyano, (C₁-C₆)-alkyl, halo- (C₁-C₆) -alkyl, (C₃-C₆)-cycloalkyl, S (O)ₙ- (C₁-C₆) -alkyl, (C₁-C₆) -alkoxy and halo-(C₁-C₆)-alkoxy, and where heterocyclyl carries n oxo groups,
R¹ is hydrogen, (C₁-C₆)-alkyl, (C₂-C₆) -alkenyl, (C₂-C₆)-alkynyl, (C₃-C₆)-cycloalkyl, (C₃-C₆) -cycloalkenyl, (C₃-C₆)-cycloalkyl-(C₁-C₆)-alkyl, where the six last-mentioned radicals are each substituted by s radicals from the group consisting of cyano, halogen, nitro, thiocyanato, OR¹⁰, S(O)R¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰) an (C₁-C₄) -alkoxy- (C₂-C₆) -alkoxycarbonyl,
or R¹ is phenyl, phenyl- (C₁-C₆) -alkyl, heteroaryl, (C₁-C₆)-alkyl-heteroaryl, heterocyclyl, (C₁-C₆)-alkyl-heterocyclyl, (C₁-C₆)-alkyl-O-heteroaryl, (C₁-C₆)-alkyl-O-heterocyclyl, (C₁-C₆) -alkyl-NR¹⁰-heteroaryl, (C₁-C₆)-alkyl-NR¹⁰-heterocyclyl, where the ten last-mentioned radicals are each substituted by s radicals from the group consisting of cyano, halogen, nitro, thiocyanato, (C₁-C₆)-alkyl, halo-(C₁-C₆)-alkyl, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰) ₂ and (C₁-C₄)-alkoxy- (C₂-C₆)-alkoxycarbonyl, and where heterocyclyl carries n oxo groups,
R² and R³ independently of one another are each hydrogen, (C₁-C₆)-alkyl, halo- (C₁-C₆) -alkyl, (C₂-C₆)-alkenyl, halo- (C₂-C₆) -alkenyl, (C₂-C₆) -alkynyl, halo- (C₂-C₆)-alkynyl, (C₃-C₆) -cycloalkyl, halo-(C₃-C₆)-cycloalkyl, (C₃-C₆)-cycloalkyl-(C₁-C₆)-alkyl, halo-(C₃-C₆)-cycloalkyl- (C₁-C₆) -alkyl, (C₁-C₆) -alkoxy- (C₁-C₆) -alkyl, halo-(C₁-C₆)-alkoxy-(C₁-C₆)-alkyl, phenyl, heteroaryl or heterocyclyl, where the three last-mentioned radicals are each substituted by s radicals from the group consisting of cyano, halogen, nitro, thiocyanato, (C₁-C₆)-alkyl, halo-(C₁-C₆)-alkyl, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹⁰, CO₂R¹⁰, COSR¹¹, CON(R¹⁰) ₂ and (C₁-C₄) -alkoxy- (C₂-C₆)-alkoxycarbonyl, and where heterocyclyl carries n oxo groups,
or R² and R³ together with the atom to which they are attached form a 5- to 6-membered unsaturated, partially saturated or saturated ring which, in addition to carbon atoms, in each case contains n oxygen and sulfur atoms,
R⁴ is (C₁-C₆)-alkyl, halo-(C₁-C₆) -alkyl, (C₂-C₆) alkenyl, halo- (C₂-C₆) -alkenyl, (C₂-C₆) -alkynyl, halo- (C₂-C₆)-alkynyl, where the six last-mentioned radicals are each substituted by s radicals from the group consisting of nitro, cyano, SiR⁹₃, PO(OR⁹)₃, S(O)ₙ-(C₁-C₆)-alkyl, (C₁-C₆)-alkoxy, halo-(C₁-C₆)-alkoxy, N(R⁷)₂, COR⁷, COOR⁷, OCOR⁷, OCO₂R⁸, NR⁷COR⁷, NR⁷SO₂R⁸, (C₃-C₆) - cycloalkyl, heteroaryl, heterocyclyl, phenyl, W-heteroaryl, W-heterocyclyl, W-phenyl or W-benzyl, where the seven last-mentioned radicals for their part are substituted by s radicals from the group consisting of (C₁-C₆)-alkyl, halo-(C₁-C₆)-alkyl, (C₁-C₆)-alkoxy, halo-(C₁-C₆)-alkoxy and halogen, and where heterocyclyl carries n oxo groups,
or R⁴ is (C₃-C₇)-cycloalkyl, heteroaryl, heterocyclyl or phenyl, where the four last-mentioned radicals are each substituted by s radicals from the group consisting of halogen, nitro, cyano, (C₁-C₆)-alkyl, halo- (C₁-C₆) -alkyl, (C₃-C₆)-cycloalkyl, S (0) ₙ- (C₁-C₆) -alkyl, (C₁-C₆) -alkoxy, halo-(C₁-C₆)-alkoxy and (C₁-C₆)-alkoxy-(C₁-C₄)-alkyl,
R⁵ is hydrogen, (C₁-C₆)-alkyl, halo- (C₁-C₆) -alkyl, (C₂-C₆)-alkenyl, (C₂-C₆) -haloalkenyl, (C₂-C₆) -alkynyl, (C₂-C₆-haloalkynyl, (C₃-C₇) -cycloalkyl, (C₁-C₆) -alkoxy, halo- (C₁-C₆) -alkoxy, (C₂-C₆) -alkenyloxy, (C₂-C₆)-alkynyloxy, cyano, nitro, methylsulfenyl, methylsulfinyl, methylsulfonyl, acetylamino, benzoylamino, methoxycarbonyl, ethoxycarbonyl, methoxycarbonylmethyl, ethoxycarbonylmethyl, benzoyl, methylcarbonyl, piperidinylcarbonyl, trifluoromethylcarbonyl, halogen, amino, aminocarbonyl, methylaminocarbonyl, dimethylaminocarbonyl, methoxymethyl, or
heteroaryl, heterocyclyl or phenyl, each of which is substituted by s radicals from the group consisting of (C₁-C₆)-alkyl, halo-(C₁-C₆)-alkyl, (C₁-C₆)-alkoxy, halo-(C₁-C₆)-alkoxy and halogen, and where heterocyclyl carries n oxo groups,
R⁶ is hydrogen, (C₁-C₆)-alkyl, R⁷O- (C₁-C₆) -alkyl, CH₂R¹², (C₃-C₇) -cycloalkyl, halo- (C₁-C₆) -alkyl, (C₂-C₆)-alkenyl, halo- (C₂-C₆) -alkenyl, (C₂-C₆) -alkynyl, halo- (C₂-C₆)-alkynyl, OR⁷, NHR⁷, methoxycarbonyl, ethoxycarbonyl, methoxycarbonylmethyl, ethoxycarbonylmethyl, methylcarbonyl, trifluoromethylcarbonyl, dimethylamino, acetylamino, methylsulfenyl, methylsulfinyl, methylsulfonyl, or heteroaryl, heterocyclyl, benzyl or phenyl, each of which is substituted by s radicals from the group consisting of halogen, nitro, cyano, (C₁-C₆)-alkyl, halo-(C₁-C₆)-alkyl, (C₃-C₆) -cycloalkyl, S(O)ₙ-(C₁-C₆)-alkyl, (C₁-C₆)-alkoxy, halo- (C₁-C₆) -alkoxy and (C₁-C₆)-alkoxy-(C₁-C₄)-alkyl, where heterocyclyl carries n oxo groups,
R⁷ is hydrogen, (C₁-C₆)-alkyl, (C₂-C₆) -alkenyl, (C₂-C₆)-alkynyl, (C₃-C₆) -cycloalkyl, (C₃-C₆) -cycloalkenyl, (C₃-C₆)-cycloalkyl-(C₁-C₆)-alkyl, phenyl, phenyl-(C₁-C₆)-alkyl, heteroaryl, (C₁-C₆)-alkylheteroaryl, heterocyclyl, (C₁-C₆)-alkylheterocyclyl, (C₁-C₆)-alkyl-O-heteroaryl, (C₁-C₆)-alkyl-O-heterocyclyl, (C₁-C₆)-alkyl-NR¹⁰-heteroaryl, (C₁-C₆)-alkyl-NR¹⁰-heterocyclyl, where the 16 last-mentioned radicals are each substituted by s radicals from the group consisting of cyano, halogen, nitro, thiocyanato, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ and (C₁-C₄)-alkoxy-(C₂-C₆)-alkoxycarbonyl, and where heterocyclyl carries n oxo groups,
R⁸ is (C₁-C₆)-alkyl, (C₂-C₆) -alkenyl, (C₂-C₆) -alkynyl, (C₃-C₆-cycloalkyl, (C₃-C₆)-cycloalkenyl, (C₃-C₆)-cycloalkyl-(C₁-C₆)-alkyl, phenyl, phenyl-(C₁-C₆)-alkyl, heteroaryl, (C₁-C₆)-alkyl-heteroaryl, heterocyclyl, (C₁-C₆)-alkyl-heterocyclyl, (C₁-C₆) -alkyl-O-heteroaryl, (C₁-C₆)-alkyl-O-heterocyclyl, (C₁-C₆)-alkyl-NR¹⁰-heteroaryl, (C₁-C₆) -alkyl-NR¹⁰-heterocyclyl, where the 16 last-mentioned radicals are each substituted by s radicals from the group consisting of cyano, halogen, nitro, thiocyanato, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ and (C₁-C₄)-alkoxy-(C₂-C₆)-alkoxycarbonyl, and where heterocyclyl carries n oxo groups,
R⁹ is methyl or ethyl,
R¹⁰ is hydrogen, (C₁-C₆)-alkyl, halo-(C₁-C₆)-alkyl (C₂-C₆)-alkenyl, (C₂-C₆) -alkynyl, (C₃-C₆) -cycloalkyl or (C₃-C₆) -cycloalkyl- (C₁-C₆) -alkyl,
R¹¹ is (C₁-C₆)-alkyl, (C₂-C₆) -alkenyl or (C₂-C₆)-alkynyl,
R¹² is acetoxy, acetamido, N-methylacetamido, benzoyloxy, benzamido, N-methylbenzamido,
methoxycarbonyl, ethoxycarbonyl, benzoyl, methylcarbonyl, piperidinylcarbonyl, morpholinylcarbonyl, trifluoromethylcarbonyl, aminocarbonyl, methylaminocarbonyl, dimethylaminocarbonyl, (C₁-C₆)-alkoxy, (C₃-C₆)-cycloalkyl, or heteroaryl, heterocyclyl or phenyl each substituted by s radicals from the group consisting of methyl, ethyl, methoxy, trifluoromethyl and halogen,
R¹³ is (C₁-C₆)-alkyl, (C₂-C₆) -alkenyl or (C₂-C₆)-alkynyl, phenyl
W is O, S, or NR¹³,
n is 0, 1 or 2,
s is 0, 1, 2 or 3.

2. Benzamides according to Claim 1, in which
Q is a Q1, Q2, Q3 or Q4 radical, 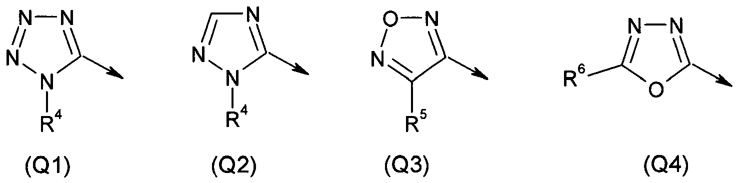
R is -CH=N-OR¹, -CH₂-O-N=CR²R³,
X is nitro, halogen, cyano, (C₁-C₆)-alkyl, halo-(C₁-C₆) -alkyl, (C₂-C₆)-alkenyl, (C₂-C₆) -alkynyl, (C₃-C₆)-cycloalkyl, (C₃-C₆) -cycloalkyl- (C₁-C₆) -alkyl, OR⁷, S(O)ₙR⁸, SO₂N(R⁷)₂, NR⁷SO₂R⁸, (C₁-C₆)-alkyl-S(O)ₙR⁸, (C₁-C₆) -alkyl-OR⁷, (C₁-C₆) -alkyl-CO₂R⁷, (C₁-C₆) -alkyl-CON (R⁷) ₂, (C₁-C₆) -alkyl-SO₂N (R⁷) ₂, (C₁-C₆) -alkyl-NR⁷COR⁷, (C₁-C₆)-alkyl-NR⁷SO₂R⁸, N(R⁷)₂, P(O)(OR⁹)₂, CH₂P(O)(OR⁹)₂, heteroaryl, heterocyclyl, phenyl, (C₁-C₆)-alkyl-heteroaryl or (C₁-C₆)-alkyl-heterocyclyl, where the five last-mentioned radicals are each substituted by s radicals from the group consisting of halogen, nitro, cyano, (C₁-C₆)-alkyl, halo- (C₁-C₆) -alkyl, S (O) ₙ-(C₁-C₆)-alkyl, (C₁-C₆)-alkoxy and halo- (C₁-C₆) -alkoxy, and where heterocyclyl carries n oxo groups,
Y is nitro, halogen, cyano, (C₁-C₆)-alkyl, halo-(C₁-C₆-alkyl, (C₂-C₆-alkenyl, halo- (C₂-C₆) -alkenyl, (C₂-C₆)-alkynyl, halo- (C₂-C₆) -alkynyl, (C₃-C₆) -cycloalkyl, OR⁷, OSO₂R⁸, S(O)ₙR⁸, SO₂OR⁸, SO₂N(R⁷)₂, NR⁷SO₂R⁸, N(R⁷)₂, P(O)(OR⁹)₂, heteroaryl, heterocyclyl or phenyl, where the three last-mentioned
radicals are each substituted by s radicals from the group consisting of halogen, nitro, cyano, (C₁-C₆)-alkyl, halo-(C₁-C₆)-alkyl, (C₃-C₆) -cycloalkyl, S(O)ₙ-(C₁-C₆)-Alkyl, (C₁-C₆)-alkoxy and halo- (C₁-C₆)-alkoxy, and where heterocyclyl carries n oxo groups,
R¹ is hydrogen, (C₁-C₆)-alkyl, (C₂-C₆)-alkenyl, (C₂-C₆)-alkynyl, (C₃-C₆)-cycloalkyl, (C₃-C₆) -cycloalkyl- (C₁-C₆)-alkyl, where the five last-mentioned radicals are each substituted by s radicals from the group consisting of cyano, halogen, nitro, OR¹⁰, S(O)ₙR¹¹, COR¹⁰, OCOR¹⁰, NR¹⁰COR¹⁰, NR¹⁰SO₂R¹¹, CO₂R¹⁰, CON(R¹⁰) and (C₁-C₄)-alkoxy-(C₂-C₆)-alkoxycarbonyl,
or R¹ is phenyl, phenyl-(C₁-C₆) -alkyl, heteroaryl, (C₁-C₆)-alkyl-heteroaryl, heterocyclyl, (C₁-C₆)-alkyl-heterocyclyl, (C₁-C₆)-alkyl-O-heteroaryl, (C₁-C₆)-alkyl-O-heterocyclyl, (C₁-C₆) -alkyl-NR¹⁰-heteroaryl or (C₁-C₆)-alkyl-NR¹⁰-heterocyclyl, where the ten last-mentioned radicals are each substituted by s radicals from the group consisting of cyano, halogen, nitro, thiocyanato, (C₁-C₆)-alkyl, halo-(C₁-C₆)-alkyl, OR¹⁰, S(O)ₙR¹¹, N(R¹⁰)₂, COR¹⁰, OCOR¹⁰, NR¹⁰COR¹⁰, NR¹⁰SO₂R¹¹, CO₂R¹⁰, CON(R¹⁰)₂ and (C₁-C₄)-alkoxy-(C₂-C₆)-alkoxycarbonyl, and where heterocyclyl carries n oxo groups,
R² and R³ independently of one another are each hydrogen, (C₁-C₆)-alkyl, halo- (C₁-C₆) -alkyl, (C₂-C₆)-alkenyl, halo- (C₂-C₆) -alkenyl, (C₂-C₆) -alkynyl, halo-(C₂-C₆-alkynyl, (C₃-C₆) -cycloalkyl, (C₁-C₆) -cycloalkyl- (C₁-C₆)-alkyl, (C₁-C₆)-alkoxy-(C₁-C₆)-alkyl, halo-(C₁-C₆)-alkoxy-(C₁-C₆)-alkyl, phenyl, heteroaryl or heterocyclyl, where the three last-mentioned radicals are each substituted by s radicals from the group consisting of cyano, halogen, nitro, (C₁-C₆)-alkyl, halo-(C₁-C₆-alkyl, OR¹⁰, S(O)ₙR¹¹, N(R¹⁰)₂, COR¹⁰, NR¹⁰COR¹⁰, NR¹⁰SO₂R¹¹, CO₂R¹⁰, CON(R¹⁰)₂ and (C₁-C₄)-alkoxy-(C₂-C₆)-alkoxycarbonyl, and where heterocyclyl carries n oxo groups,
or R² and R³ together with the atom to which they are attached form a 5- to 6-membered unsaturated, partially saturated or saturated ring which, in addition to carbon atoms, in each case contains n oxygen and sulfur atoms,
R⁴ is (C₁-C₆)-alkyl, halo- (C₁-C₆) -alkyl, (C₂-C₆)-alkenyl, halo- (C₂-C₆) -alkenyl, (C₂-C₆)-alkynyl, halo- (C₂-C₆)-alkynyl, where the six last-mentioned radicals are each substituted by s radicals from the group consisting of nitro, cyano, S(O)ₙ-(C₁-C₆)-alkyl, (C₁-C₆)-alkoxy, halo- (C₁-C₆) -alkoxy, COOR⁷, NR⁷COR⁷, NR⁷SO₂R⁸, (C₃-C₆)-cycloalkyl, heteroaryl, heterocyclyl, phenyl, W-heteroaryl, W-heterocyclyl, W-phenyl and W-benzyl, where the 7 last-mentioned radicals for their part are substituted by s radicals from the group consisting of (C₁-C₆)-alkyl, halo- (C₁-C₆) -alkyl, (C₁-C₆)-alkoxy, halo-(C₁-C₆)-alkoxy and halogen, and where heterocyclyl carries n oxo groups,
or R⁴ is (C₃-C₇)-cycloalkyl, heteroaryl, heterocyclyl or phenyl, where the four last-mentioned radicals are each substituted by s radicals from the group consisting of halogen, nitro, cyano, (C₁-C₆)-alkyl, halo- (C₁-C₆)-alkyl, (C₃-C₆-cycloalkyl, S(O)ₙ-(C₁-C₆)-alkyl, (C₁-C₆)-alkoxy, halo-(C₁-C₆)-alkoxy and (C₁-C₆)-alkoxy-(C₁-C₄)-alkyl,
R⁵ is hydrogen, (C₁-C₆)-alkyl, halo- (C₁-C₆) -alkyl, (C₂-C₆-alkenyl, (C₂-C₆) -haloalkenyl, (C₂-C₆) -alkynyl, (C₂-C₆-haloalkynyl, (C₃-C₇-cycloalkyl, (C₁-C₆)-alkoxy, halo- (C₁-C₆) -alkoxy, (C₂-C₆) -alkenyloxy, (C₂-C₆)-alkynyloxy, cyano, nitro, methylsulfenyl, methylsulfinyl, methylsulfonyl, acetylamino, methoxycarbonyl, methoxycarbonylmethyl, methylcarbonyl, trifluoromethylcarbonyl, halogen, amino, aminocarbonyl, methylaminocarbonyl, dimethylaminocarbonyl, methoxymethyl, or
heteroaryl, heterocyclyl or phenyl, each of which is substituted by s radicals from the group consisting of (C₁-C₆)-alkyl, halo-(C₁-C₆)-alkyl, (C₁-C₆)-alkoxy, halo-(C₁-C₆)-alkoxy and halogen, and where heterocyclyl carries n oxo groups,
R⁶ is hydrogen, (C₁-C₆)-alkyl, R⁷O- (C₁-C₆) -alkyl, CH₂R¹², (C₃-C₇) -cycloalkyl, halo- (C₁-C₆) -alkyl, (C₂-C₆)-alkenyl, (C₂-C₆)-alkynyl, OR⁷, NHR⁷, methoxycarbonyl, methoxycarbonylmethyl, methylcarbonyl, trifluoromethylcarbonyl, dimethylamino, acetylamino, methylsulfenyl, methylsulfinyl, methylsulfonyl, or heteroaryl, heterocyclyl, benzyl or phenyl, each of which is substituted by s radicals from the group consisting of halogen, nitro, cyano, (C₁-C₆)-alkyl, halo- (C₁-C₆) -alkyl, (C₃-C₆) -cycloalkyl, S (0) ₙ-(C₁-C₆)-alkyl, (C₁-C₆)-alkoxy, halo- (C₁-C₆) -alkoxy, (C₁-C₆)-alkoxy-(C₁-C₄)-alkyl, and where heterocyclyl carries n oxo groups,
R⁷ is hydrogen, (C₁-C₆)-alkyl, (C₂-C₆) -alkenyl, (C₂-C₆)-alkynyl, (C₃-C₆) -cycloalkyl, (C₃-C₆) -cycloalkenyl, (C₃-C₆)-cycloalkyl-(C₁-C₆)-alkyl, phenyl, heteroaryl, heterocyclyl, where the nine last-mentioned radicals are each substituted by s radicals from the group consisting of cyano, halogen, nitro, OR¹⁰, S(O)ₙR¹¹, N(R¹⁰)₂, NR¹⁰SO₂R¹¹, CO₂R¹⁰, CON(R¹⁰)₂ and (C₁-C₄)-alkoxy-(C₂-C₆)-alkoxycarbonyl, and where heterocyclyl carries n oxo groups,
R⁸ is (C₁-C₆)-alkyl, (C₂-C₆) -alkenyl, (C₂-C₆) -alkynyl, (C₃-C₆)-cycloalkyl, (C₃-C₆)-cycloalkenyl, (C₃-C₆)-cycloalkyl-(C₁-C₆)-alkyl, phenyl, heteroaryl, heterocyclyl, where the nine last-mentioned radicals are each substituted by s radicals from the group consisting of cyano, halogen, nitro, OR¹⁰, S(O)ₙR¹¹, N(R¹⁰)₂, NR¹⁰SO₂R¹¹, CO₂R¹⁰, CON(R¹⁰)₂ and (C₁-C₄)-alkoxy-(C₂-C₆)-alkoxycarbonyl, and where heterocyclyl carries n oxo groups,
R⁹ is methyl or ethyl,
R¹⁰ is hydrogen, (C₁-C₆)-alkyl, halo-(C₁-C₆)-alkyl (C₂-C₆)-alkenyl, (C₂-C₆) -alkynyl, (C₃-C₆)-cycloalkyl or (C₃-C₆)-cycloalkyl-(C₁-C₆)-alkyl,
R¹¹ is (C₁-C₆)-alkyl, (C₂-C₆) -alkenyl or (C₂-C₆)-alkynyl,
R¹² is acetoxy, acetamido, N-methylacetamido, benzoyloxy, benzamido, N-methylbenzamido, methoxycarbonyl, ethoxycarbonyl, benzoyl, methylcarbonyl, trifluoromethylcarbonyl, aminocarbonyl, methylaminocarbonyl, dimethylaminocarbonyl, (C₁-C₆)-alkoxy, (C₃-C₆)-cycloalkyl, or heteroaryl, heterocyclyl or phenyl each substituted by s radicals from the group consisting of methyl, methoxy, trifluoromethyl and halogen,
R¹³ is (C₁-C₆)-alkyl,
W is O, S or NR¹³,
n is 0, 1 or 2;
s is 0, 1, 2 or 3.

3. Benzamides according to Claim 1 or 2, in which
Q is a Q1 or Q2 radical, 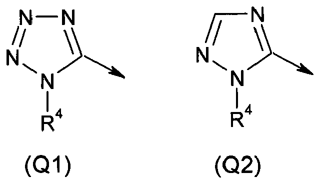
R is -CH=N-OR¹, -CH₂-O-N=CR²R³,
X is nitro, halogen, (C₁-C₆)-alkyl, halo-(C₁-C₆)-alkyl, S(O)ₙR⁸, (C₁-C₆)-alkyl-S(O)ₙR⁸, (C₁-C₆)-alkyl-OR⁷, (C₁-C₆)-alkyl-SO₂N (R⁷) ₂ or (C₁-C₆) -alkyl-NR⁷SO₂R⁸,
Y is nitro, halogen, halo-(C₁-C₆)-alkyl or S(O)ₙR⁸,
R¹ is hydrogen, (C₁-C₆)-alkyl, (C₂-C₆) -alkenyl, (C₂-C₆-alkynyl, (C₃-C₆) -cycloalkyl or (C₃-C₆) -cycloalkyl-(C₁-C₆)-alkyl, where the five last-mentioned radicals are each substituted by s radicals from the group consisting of cyano, halogen, OR¹⁰ and S(O)ₙR¹¹,
R² and R³ independently of one another are each hydrogen, (C₁-C₆)-alkyl, halo- (C₁-C₆) -alkyl, (C₂-C₆)-alkenyl, (C₂-C₆)-alkynyl, (C₁-C₆) -alkoxy- (C₁-C₆) -alkyl or halo-(C₁-C₆)-alkoxy-(C₁-C₆)-alkyl,
R⁴ is (C₁-C₆)-alkyl, (C₂-C₆)-alkenyl or (C₂-C₆)-alkynyl, where these three radicals are each substituted by s radicals (C₁-C₆)-alkoxy,
R⁵ is (C₁-C₆)-alkyl, (C₂-C₆) -alkenyl, (C₂-C₆)-alkynyl, (C₃-C₇)-cycloalkyl, (C₁-C₆)-alkoxy, (C₂-C₆) -alkenyloxy, (C₂-C₆)-alkynyloxy, acetylamino, halogen or methoxymethyl,
R⁶ is (C₁-C₆)-alkyl, R⁷O-(C₁-C₆)-alkyl, CH₂R¹², (C₃-C₇)-cycloalkyl, (C₂-C₆)-alkenyl or (C₂-C₆)-alkynyl,
R⁷ is hydrogen, (C₁-C₆)-alkyl, (C₂-C₆) -alkenyl, (C₂-C₆)-alkynyl, (C₃-C₆) -cycloalkyl or (C₃-C₆) -cycloalkyl-(C₁-C₆)-alkyl,
R⁸ is (C₁-C₆)-alkyl, (C₂-C₆) -alkenyl, (C₂-C₆) -alkynyl, (C₃-C₆) -cycloalkyl, (C₃-C₆) -cycloalkenyl or (C₃-C₆)-cycloalkyl-(C₁-C₆)-alkyl,
R⁹ is methyl or ethyl,
R¹⁰ is hydrogen, (C₁-C₆)-alkyl, (C₂-C₆) -alkenyl, (C₂-C₆)-alkynyl, (C₃-C₆) -cycloalkyl or (C₃-C₆) -cycloalkyl-(C₁-C₆)-alkyl,
R¹¹ is (C₁-C₆)-alkyl, (C₂-C₆)-alkenyl or (C₂-C₆)-alkynyl,
R¹² is acetoxy, acetamido or (C₃-C₆)-cycloalkyl,
R¹³ is (C₁-C₆)-alkyl,
W is 0, S or NR¹³,
n is 0, 1 or 2;
s is 0, 1, 2 or 3.

4. Herbicidal compositions **characterized by** a herbicidally effective amount of at least one compound of the formula (I) according to any of Claims 1 to 3.

5. Herbicidal compositions according to Claim 4 in a mixture with formulation auxiliaries.

6. Herbicidal compositions according to Claim 4 or 5, comprising at least one further pesticidally active substance from the group of insecticides, acaricides, herbicides, fungicides, safeners and growth regulators.

7. Herbicidal compositions according to Claim 6, comprising a safener.

8. Herbicidal compositions according to Claim 7, comprising cyprosulfamide, cloquintocet-mexyl, mefenpyr-diethyl or isoxadifen-ethyl.

9. Herbicidal compositions according to any of Claims 6 to 8, comprising a further herbicide.

10. Method for controlling unwanted plants, **characterized in that** an effective amount of at least one compound of the formula (I) according to any of Claims 1 to 3 or of a herbicidal composition according to any of Claims 4 to 9 is applied to the plants or the site of the unwanted plant growth.

11. Use of compounds of the formula (I) according to any of Claims 1 to 3 or of herbicidal compositions according to any of Claims 4 to 9 for controlling unwanted plants.

12. Use according to Claim 11, **characterized in that** the compounds of the formula (I) are used for controlling unwanted plants in crops of useful plants.

13. Use according to Claim 12, **characterized in that** the useful plants are transgenic useful plants.

## Revendications

1. Benzamides de formule (I) ou leurs sels 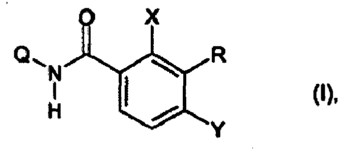 formule dans laquelle les substituants ont les significations suivantes :
Q représente un radical Q1, Q2, Q3 ou Q4, 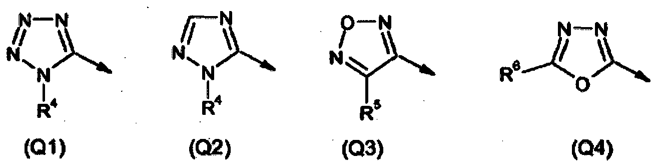
R représente -CH=N-OR¹, -CH₂-O-N=CR²R³,
X représente un groupe nitro, un atome d'halogène, un groupe cyano, formyle, sulfocyano, alkyle en C₁-C₆, halogéno-alkyle(C₁-C₆), alcényle en C₂-C₆, halogéno-alcényle(C₂-C₆), alcynyle en C₂-C₆, halogéno-alcynyle(C₃-C₆), cycloalkyle en C₃-C₆, halogéno-cycloalkyle(C₃-C₆), cycloalkyl(C₃-C₆)-alkyle(C₁-C₆), halogéno-cycloalkyl (C₃-C₆)-alkyle (C₁-C₆), COR⁷, COO_{R}⁷, OCOOR⁸, NR⁷COOR⁸, C(O)N(R⁷)₂, NR⁷C (0) N (R⁷) ₂, OC(O)N(R⁷)₂, C(O)NR⁷OR⁷, OR⁷, OCOR⁷, OSO₂R⁸, S(O)ₙR⁸, SO₂OR⁷, SO₂N(R⁷)₂, NR⁷SO₂R⁸, NR⁷OR⁷, alkyl(C₁-C₆)-S(O)ₙR⁸, alkyl(C₁-C₆)-OR⁷, alkyl(C₁-C₆)-OCOR⁷, alkyl(C₁-C₆)-OSO₂R⁸, alkyl (C₁-C₆)-CO₂R⁷, alkyl (C₁-C₆) -SO₂OR⁸, alkyl (C₁-C₆)-CON(R⁷)₂, alkyl (C₁-C₆)-SO₂N (R⁷) ₂, alkyl (C₁-C₆) -NR⁷COR⁷, alkyl (C₁-C₆) -NR⁷SO₂R⁸, N(R⁷)₂, P(O)(OR⁹)₂, CH₂P(O)(OR⁹)₂, hétéroaryle, hétérocyclyle, phényle, alkyl(C₁-C₆)-hétéroaryle ou alkyl(C₁-C₆)-hétérocyclyle, les cinq derniers radicaux nommés en dernier étant substitués chacun par s radicaux choisis dans le groupe constitué par halogéno, nitro, cyano, alkyle en C₁-C₆, halogéno-alkyle(C₁-C₆), S(O)ₙ-alkyle(C₁-C₆), alcoxy en C₁-C₆ et halogéno-alcoxy(C₁-C₆), et le groupe hétérocyclyle portant n groupes oxo,
Y représente un groupe nitro, un atome d'halogène, un groupe cyano, formyle, sulfocyano, alkyle en C₁-C₆, halogéno-alkyle(C₁-C₆), alcényle en C₂-C₆, halogéno-alcényle(C₂-C₆), alcynyle en C₂-C₆, halogéno-alcynyle(C₂-C₆), cycloalkyle en C₃-C₆, halogéno-cycloalkyle(C₃-C₆), cycloalkyl(C₃-C₆)-alkyle (C₁-C₆), halogéno-cycloalkyl(C₃-C₆)-alkyle (C₁-C₆), COR⁷, COOR⁷, OCOOR⁸, NR¹COOR⁸, C(O)N(R⁷)₂, NR⁷C(O)N(R⁷)₂, OC(O)N(R⁷)₂, C(O)NR⁷OR⁷, OR⁷, OSO₂R⁸, S(O)ₙR⁸, SO₂OR⁸, SO₂N(R⁷)₂, NR⁷SO₂R⁸, NR⁷COR⁷, alkyl (C₁-C₆)-S(O)ₙR⁸, alkyl (C₁-C₆) -OR⁷, alkyl (C₁-C₆) -OCOR⁷, alkyl (C₁-C₆) -OSO₂R⁸, alkyl (C₁-C₆)-CO₂R⁷, alkyl (C₁-C₆)-SO₂OR⁷, alkyl (C₁-C₆) -CON (R⁷) 2, alkyl (C₁-C₆) -SO₂N (R⁷) ₂, alkyl (C₁-C₆) -NR⁷COR⁷, alkyl (C₁-C₆) -NR⁷SO₂R⁸, N(R⁷)₂, P(O)(OR⁹)₂, hétéroaryle, hétérocyclyle ou phényle, les trois radicaux nommés en dernier étant substitués chacun par s radicaux choisis dans le groupe constitué par halogéno, nitro, cyano, alkyle en C₁-C₆, halogéno-alkyle(C₁-C₆), cycloalkyle en C₃-C₆, S(O)ₙ-alkyle(C₁-C₆), alcoxy en C₁-C₆ et halogéno-alcoxy(C₁-C₆), et le groupe hétérocyclyle portant n groupes oxo,
R¹ représente un atome d'hydrogène, un groupe alkyle en C₁-C₆, alcényle en C₂-C₆, alcynyle en C₂-C₆, cycloalkyle en C₃-C₆, cycloalcényle en C₃-C₆, cycloalkyl(C₃-C₆)-alkyle (C₁-C₆), les six derniers radicaux étant substitués chacun par s radicaux choisis dans le groupe constitué par cyano, halogéno, nitro, sulfocyano, OR¹⁰, S(O)ₙR¹¹, N(R¹⁰)₂ NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ et alcoxy (C₁-C₄) -alcoxycarbonyle (C₂-C₆),
ou R¹ représente un groupe phényle, phényl-alkyle(C₁-C₆), hétéroaryle, alkyl(C₁-C₆)-hétéroaryle, hétérocyclyle, alkyl(C₁-C₆)-hétérocyclyle, alkyl(C₁-C₆)-O-hétéroaryle, alkyl(C₁-C₆)-O-hétérocyclyle, alkyl (C₁-C₆) -NR¹⁰-hétéroaryle, alkyl (C₁-C₆)-NR¹⁰-hétérocyclyle, les dix radicaux nommés en dernier étant substitués chacun par s radicaux choisis dans le groupe constitué par cyano, halogéno, nitro, sulfocyano, alkyle en C₁-C₆, halogéno-alkyle(C₁-C₆), OR¹⁰, S(O)ₙR¹¹, N(R¹⁰)₂, NR¹⁰OR¹⁰, COR¹⁰, OCOR¹⁰), SCOR¹¹, NR¹⁰COR¹⁰, NR¹⁰SO₂R¹¹, CO₂R¹⁰, COSR¹¹, CON(R¹⁰)₂ et alcoxy (C₁-C₄)-alcoxycarbonyle(C₂-C₆), et le groupe hétérocyclyle portant n groupes oxo,
R² et R³ représentent chacun indépendamment l'un de l'autre un atome d'hydrogène, un groupe alkyle en C₁-C₆, halogéno-alkyle(C₁-C₆), alcényle en C₂-C₆, halogéno-alcényle (C₂-C₆) , alcynyle en C₂-C₆, halogéno-alcynyle(C₂-C₆), cycloalkyle en C₃-C₆, halogéno-cycloalkyle (C₃-C₆), cycloalkyl (C₃-C₆) -alkyle (C₁-C₆), halogéno-cycloalkyl (C₃-C₆) -alkyle (C₁-C₆) , alcoxy (C₁-C₆)-alkyle (C₁-C₆), halogéno-alcoxy(C₁-C₆)-alkyle(C₁-C₆), phényle, hétéroaryle ou hétérocyclyle, les trois radicaux nommés en dernier étant substitués chacun par s radicaux choisis dans le groupe constitué par cyano, halogéno, nitro, sulfocyano, alkyle en C₁-C₆, halogéno-alkyle(C₁-C₆), OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ et alcoxy(C₁-C₄)-alcoxycarbonyle(C₂-C₆), et le groupe hétérocyclyle portant n groupes oxo,
ou R² et R³ forment ensemble avec l'atome, auquel ils sont liés, un cycle à 5 ou 6 chaînons, insaturé, partiellement saturé ou saturé, qui en plus d'atomes de carbone contient n atomes respectivement d'oxygène et de soufre,
R⁴ représente un groupe alkyle en C₁-C₆, halogéno-alkyle(C₁-C₆), alcényle en C₂-C₆, halogéno-alcényle(C₂-C₆), alcynyle en C₂-C₆, halogéno-alcynyle(C₂-C₆), les six radicaux nommés en dernier étant substitués chacun par s radicaux choisi dans le groupe constitué par nitro, cyano, SiR⁹₃, PO(OR⁹)₃, S(O)ₙ-alkyle(C₁-C₆), alcoxy en C₁-C₆, halogéno-alcoxy(C₁-C₆), N(R⁷)₂, COR⁷, COOR⁷, OCOR⁷, OCO₂R⁸, NR⁷COR⁷, NR⁷SO₂R⁸, cycloalkyle en C₃-C₆, hétéroaryle, hétérocyclyle, phényle, W-hétéroaryle, W-hétérocyclyle, W-phényle ou W-benzyle, les sept radicaux nommés en dernier étant eux-mêmes à leur tour substitués par s radicaux choisis dans le groupe constitué par alkyle en C₁-C₆, halogéno-alkyle(C₁-C₆), alcoxy en C₁-C₆, halogéno-alcoxy(C₁-C₆) et halogéno, et le groupe hétérocyclyle portant n groupes oxo,
ou R⁴ représente un groupe cycloalkyle en C₃-C₇, hétéroaryle, hétérocyclyle ou phényle, les quatre radicaux nommés en dernier étant substitués chacun par s radicaux choisis dans le groupe constitué par halogéno, nitro, cyano, alkyle en C₁-C₆, halogéno-alkyle(C₁-C₆), cycloalkyle en C₃-C₆, S(O)ₙ-alkyle(C₁-C₆), alcoxy en C₁-C₆, halogéno-alcoxy(C₁-C₆) et alcoxy(C₁-C₆)-alkyle(C₁-C₄),
R⁵ représente un atome d'hydrogène, un groupe alkyle en C₁-C₆, halogéno-alkyle(C₁-C₆), alcényle en C₂-C₆, halogéno-alcényle(C₂-C₆), alcynyle en C₂-C₆, halogéno-alcynyle(C₂-C₆), cycloalkyle en C₃-C₇, alcoxy en C₁-C₆, halogéno-alcoxy(C₁-C₆), alcényloxy en C₂-C₆, alcynyloxy en C₂-C₆, cyano, nitro, méthylsulfényle, méthylsulfinyle, méthylsulfonyle, acétylamino, benzoylamino, méthoxycarbonyle, éthoxycarbonyle, méthoxycarbonylméthyle, éthoxycarbonylméthyle, benzoyle, méthylcarbonyle, pipéridinylcarbonyle, trifluorométhylcarbonyle, un atome d'halogène, amino, aminocarbonyl, méthylaminocarbonyle, diméthylamino-carbonyle, méthoxyméthyle, ou un groupe hétéroaryle, hétérocyclyle ou phényle chacun substitué par s radicaux choisis dans le groupe constitué par alkyle en C₁-C₆, halogéno-alkyle(C₁-C₆), alcoxy en C₁-C₆, halogéno-alcoxy(C₁-C₆) et halogéno, et le groupe hétérocyclyle portant n groupes oxo,
R⁶ représente un atome d'hydrogène, un groupe alkyle en C₁-C₆, R⁷O-alkyle(C₁-C₆), CH₂R¹², cycloalkyle en C₃-C⁷, halogéno-alkyle(C₁-C₆), alcényle en C₂-C₆, halogéno-alcényle(C₂-C₆), alcynyle en C₂-C₆, halogéno-alcynyle(C₂-C₆), OR⁷, NHR⁷, méthoxycarbonyle, éthoxycarbonyle, méthoxycarbonylméthyle, éthoxycarbonylméthyle, méthylcarbonyle, trifluorométhylcarbonyle, diméthylamino, acétylamino, méthylsulfényle, méthyl-sulfinyle, méthylsulfonyle ou un groupe hétéroaryle, hétérocyclyle, benzyle ou phényle chacun substitué par s radicaux choisis dans le groupe constitué par halogéno, nitro, cyano, alkyle en C₁-C₆, halogéno-alkyle(C₁-C₆), cycloalkyle en C₃-C₆, S(O)ₙ-alkyle(C₁-C₆), alcoxy en C₁-C₆, halogéno-alcoxy(C₁-C₆) et alcoxy(C₁-C₆)-alkyle(C₁-C4), le groupe hétérocyclyle portant n groupes oxo,
R⁷ représente un atome d'hydrogène, un groupe alkyle en C₁-C₆, alcényle en C₂-C₆, alcynyle en C₂-C₆, cycloalkyle en C₃-C₆, cycloalcényle en C₃-C₆, cycloalkyl(C₃-C₆)-alkyle(C₁-C₆), phényle, phényl-alkyle(C₁-C₆), hétéroaryle, alkyl(C₁-C₆)-hétéroaryle, hétérocyclyle, alkyl (C₁-C₆)-hétérocyclyle, alkyl(C₁-C₆)-O-hétéroaryle, alkyl(C₁-C₆)-O-hétérocyclyle, alkyl (C₁-C₆)-NR¹⁰-hétéroaryle, alkyl(C₁-C₆)-NR¹⁰-hétérocyclyle, les 16 radicaux nommés en dernier étant substitués chacun par s radicaux choisis dans le groupe constitué par cyano, halogéno, nitro, sulfocyano, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ et alcoxy (C₁-C₉)-alcoxycarbonyle(C₂-C₆) et le groupe hétérocyclyle portant n groupes oxo,
R⁸ représente un groupe alkyle en C₁-C₆, alcényle en C₂-C₆, alcynyle en C₂-C₆, cycloalkyle en C₃-C₆, cycloalcényle en C₃-C₆, cycloalkyl (C₃-C₆) -alkyle (C₁-C₆), phényle, phényl-alkyle(C₁-C₆), hétéroaryle, alkyl(C₁-C₆)-hétéroaryle, hétérocyclyle, alkyl(C₁-C₆)-hétérocyclyle, alkyl (C₁-C₆)-O-hétéroaryle, alkyl(C₁-C₆)-O-hétérocyclyle, alkyl(C₁-C₆)-NR¹⁰-hétéroaryle, alkyl (C₁-C₆)-NR¹⁰-hétérocyclyle, les 16 radicaux nommés en dernier étant substitués chacun par s radicaux choisis dans le groupe constitué par cyano, halogéno, nitro, sulfocyano, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ et alcoxy (C₁-C₄) -alcoxycarbonyle (C₂-C₆) et le groupe hétérocyclyle portant n groupes oxo,
R⁹ représente le groupe méthyle ou éthyle,
R¹⁰ représente un atome d'hydrogène, un groupe alkyle en C₁-C₆, halogéno-alkyle(C₁-C₆), alcényle en C₂-C₆, alcynyle en C₂-C₆, cycloalkyle en C₃-C₆ ou cycloalkyl(C₃-C₆)-alkyle(C₁-C₆),
R¹¹ représente un groupe alkyle en C₁-C₆, alcényle en C₂-C₆ ou alcynyle en C₂-C₆,
R¹² représente un groupe acétoxy, acétamido, N-méthyl-acétamido, benzoyloxy, benzamido, N-méthylbenzamido, méthoxycarbonyle, éthoxycarbonyle, benzoyle, méthylcarbonyle, pipéridinylcarbonyle, morpholinylcarbonyle, trifluorométhylcarbonyle, aminocarbonyle, méthylaminocarbonyle, diméthylaminocarbonyle, alcoxy en C₁-C₆, cycloalkyle en C₃-C₆ ou un groupe hétéroaryle, hétérocyclyle ou phényle chacun substitué par s radicaux choisis dans le groupe constitué par méthyle, éthyle, méthoxy, trifluorométhyle et halogéno,
R¹³ représente un groupe alkyle en C₁-C₆, alcényle en C₂-C₆ ou alcynyle en C₂-C₆, phényle,
W représente O, S ou NR¹³,
n représente 0, 1 ou 2,
s représente 0, 1, 2 ou 3.

2. Benzamides selon la revendication 1, dans lesquels
Q représente un radical Q1, Q2, Q3 ou Q4, 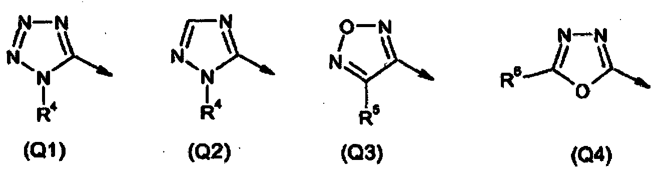
R représente -CH=N-OR¹, -CH₂-O-N=CR²R³,
X représente un groupe nitro, un atome d'halogène, un groupe cyano, alkyle en C₁-C₆, halogéno-alkyle (C₁-C₆), alcényle en C₂-C₆, alcynyle en C₂-C₆, cycloalkyle en C₃-C₆, cycloalkyl (C₃-C₆) -alkyle (C₁-C₆), OR⁷, S(O)ₙR⁸, SO₂N(R⁷)₂, NR⁷SO₂R⁸, alkyl(C₁-C₆)-S(O)ₙR⁸, alkyl(C₁-C₆)-OR⁷, alkyl (C₁-C₆) -CO₂R⁷, alkyl(C₁-C₆)-CON(R⁷)₂, alkyl(C₁-C₆)-SO₂N(R⁷)₂, alkyl (C₁-C₆)-NR⁷COR⁷, alkyl (C₁-C₆)-NR⁷SO₂R⁸, N(R⁷)₂, P(O) (OR⁹) ₂, CH₂P(O)(OR⁹)₂, hétéroaryle, hétérocyclyle, phényle, alkyl(C₁-C₆)-hétéroaryle ou alkyl (C₁-C₆)-hétérocyclyle, les cinq derniers radicaux nommés en dernier étant substitués chacun par s radicaux choisis dans le groupe constitué par halogéno, nitro, cyano, alkyle en C₁-C₆, halogéno-alkyle(C₁-C₆) , S(O)ₙ-alkyle(C₁-C₆), alcoxy en C₁-C₆ et halogéno-alcoxy(C₁-C₆), et le groupe hétérocyclyle portant n groupes oxo,
Y représente un groupe nitro, un atome d'halogène, un groupe cyano, alkyle en C₁-C₆, halogéno-alkyle(C₁-C6₎, alcényle en C₂-C₆, halogéno-alcényle(C₂-C₆), alcynyle en C₂-C₆, halogéno-alcynyle(C₂-C₆), cycloalkyle en C₃-C₆, OR⁷, OSO₂R⁸, S(O)ₙR⁸, SO₂OR⁸, SO₂N(R²)₂, NR⁷SO₂R⁸, N(R⁷)₂, P(O)(OR⁹)₂, hétéroaryle, hétérocyclyle ou phényle, les trois radicaux nommés en dernier étant substitués chacun par s radicaux choisis dans le groupe constitué par halogéno, nitro, cyano, alkyle en C₁-C₆, halogéno-alkyle(C₁-C₆), cycloalkyle en C₃-C₆, S(O)ₙ-alkyle(C₁-C₆), alcoxy en C₁-C₆ et halogéno-alcoxy(C₁-C₆), et le groupe hétérocyclyle portant n groupes oxo,
R¹ représente un atome d'hydrogène, un groupe alkyle en C₁-C₆, alcényle en C₂-C₆, alcynyle en C₂-C₆, cycloalkyle en C₃-C₆, cycloalkyl (C₃-C₆) -alkyle (C₁-C₆), les cinq radicaux nommés en dernier étant substitués chacun par s radicaux choisis dans le groupe constitué par cyano, halogéno, nitro, OR¹⁰, S(O)ₙR¹¹, COR¹⁰, OCOR¹⁰, NR¹⁰COR¹⁰, NR¹⁰SO₂R¹¹, CO₂R¹⁰, CON(R¹⁰)₂ et alcoxy(C₁-C₄)-alcoxycarbonyle(C₂-C₆),
ou R¹ représente un groupe phényle, phényl-alkyle(C₁-C₆), hétéroaryle, alkyl(C₁-C₆)-hétéroaryle, hétérocyclyle, alkyl(C₁-C₆)-hétérocyclyle, alkyl(C₁-C₆)-O-hétéroaryle, alkyl(C₁-C₆)-O-hétérocyclyle, alkyl (C₁-C₆) -NR¹⁰-hétéroaryle ou alkyl (C₁-C₆) -NR¹⁰-hétérocyclyle, les dix radicaux nommés en dernier étant substitués chacun par s radicaux choisis dans le groupe constitué par cyano, halogéno, nitro, sulfocyano, alkyle en C₁-C₆, halogéno-alkyle (C₁-C₆), OR¹⁰, S(O)ₙR¹¹, N(R¹⁰)₂, COR¹⁰, OCOR¹⁰, NR¹⁰COR¹⁰, NR¹⁰SO₂R¹¹, CO₂R¹⁰, CON(R¹⁰)₂ et alcoxy (C₁-C₄)-alcoxycarbonyle(C₂-C₆), et le groupe hétérocyclyle portant n groupes oxo,
R² et R³ représentent chacun indépendamment l'un de l'autre un atome d'hydrogène, un groupe alkyle en C₁-C₆, halogéno-alkyle(C₁-C₆), alcényle en C₂-C₆, halogéno-alcényle(C₂-C₆), alcynyle en C₂-C₆, halogéno-alcynyle(C₂-C₆), cycloalkyle en C₃-C₆, cycloalkyl(C₃-C₆)-alkyle(C₁-C₆), alcoxy (C₁-C₆) -alkyle (C₁-C₆), halogéno-alcoxy(C₁-C₆)-alkyle(C₁-C₆), phényle, hétéroaryle ou hétérocyclyle, les trois radicaux nommés en dernier étant substitués chacun par s radicaux choisis dans le groupe constitué par cyano, halogéno, nitro, alkyle en C₁-C₆, halogéno-alkyle (C₁-C₆), OR¹⁰, S(O)ₙR¹¹, N(R¹⁰)₂, COR¹⁰, NR¹⁰COR¹¹, NR¹⁰SO₂R¹¹, CO₂R¹⁰, CON(R¹⁰)₂ et alcoxy(C₁-C₄)-alcoxycarbonyle(C₂-C₆), et le groupe hétérocyclyle portant n groupes oxo,
ou R² et R³ forment ensemble avec l'atome, auquel ils sont liés, un cycle à 5 ou 6 chaînons, insaturé, partiellement saturé ou saturé, qui en plus d'atomes de carbone contient n atomes respectivement d'oxygène et de soufre,
R⁴ représente un groupe alkyle en C₁-C₆, halogéno-alkyle(C₁-C₆), alcényle en C₂-C₆, halogéno-alcényle(C₂-C₆), alcynyle en C₂-C₆, halogéno-alcynyle(C₂-C₆), les six radicaux nommés en dernier étant substitués chacun par s radicaux choisi dans le groupe constitué par nitro, cyano, S(O)ₙ-alkyle(C₁-C₆, alcoxy en C₁-C₆, halogéno-alcoxy(C₁-C₆), COOR⁷, NR⁷COR⁷, NR⁷SO₂R⁸, cycloalkyle en C₃-C₆, hétéroaryle, hétérocyclyle, phényle, W-hétéroaryle, W-hétérocyclyle, W-phényle ou W-benzyle, les 7 radicaux nommés en dernier étant eux-mêmes à leur tour substitués par s radicaux choisis dans le groupe constitué par alkyle en C₁-C₆, halogéno-alkyle(C₁-C₆), alcoxy en C₁-C₆, halogéno-alcoxy(C₁-C₆) et halogéno, et le groupe hétérocyclyle portant n groupes oxo,
ou R⁴ représente un groupe cycloalkyle en C₃-C₇, hétéroaryle, hétérocyclyle ou phényle, les quatre radicaux nommés en dernier étant substitués chacun par s radicaux choisis dans le groupe constitué par halogéno, nitro, cyano, alkyle en C₁-C₆, halogéno-alkyle(C₁-C₆) , cycloalkyle en C₃-C₆, S(O)ₙ-alkyle(C₁-C₆), alcoxy en C₁-C₆, halogéno-alcoxy(C₁-C₆) et alcoxy(C₁-C₆)-alkyle(C₁-C₄),
R⁵ représente un atome d'hydrogène, un groupe alkyle en C₁-C₆, halogéno-alkyle(C₁-C₆), alcényle en C₂-C₆, halogéno-alcényle(C₂-C₆), alcynyle en C₂-C₆, halogéno-alcynyle (C₂-C₆), cycloalkyle en C₃-C₇, alcoxy en C₁-C₆, halogéno-alcoxy(C₁-C₈), alcényloxy en C₂-C₆, alcynyloxy en C₂-C₆, cyano, nitro, méthylsulfényle, méthyl-sulfinyle, méthylsulfonyle, acétylamino, méthoxycarbonyle, méthoxycarbonylméthyle, méthylcarbonyle, trifluorométhylcarbonyle, un atome d'halogène, amino, aminocarbonyle, méthylaminocarbonyle, diméthylaminocarbonyle, méthoxyméthyle, ou
un groupe hétéroaryle, hétérocyclyle ou phényle chacun substitué par s radicaux choisis dans le groupe constitué par alkyle en C₁-C₆, halogéno-alkyle(C₁-C₆), alcoxy en C₁-C₆, halogéno-alcoxy(C₁-C₆) et halogéno, et le groupe hétérocyclyle portant n groupes oxo,
R⁶ représente un atome d'hydrogène, un groupe alkyle en C₁-C₆, R⁷O-alkyle(C₁-C₆), CH₂R¹², cycloalkyle en C₃-C₇, halogéno-alkyle(C₁-C₆), alcényle en C₂-C₆, alcynyle en C₂-C₆, OR⁷, NHR⁷, méthoxycarbonyle, méthoxycarbonylméthyle, méthylcarbonyle, trifluorométhylcarbonyle, diméthylamino, acétylamino, méthylsulfényle, méthyl-sulfinyle, méthylsulfonyle ou un groupe hétéroaryle, hétérocyclyle, benzyle ou phényle chacun substitué par s radicaux choisis dans le groupe constitué par halogéno, nitro, cyano, alkyle en C₁-C₆, halogéno-alkyle(C₁-C₆), cycloalkyle en C₃-C₆, S(O)ₙ-alkyle(C₁-C₆), alcoxy en C₁-C₆, halogéno-alcoxy(C₁-C₆), alcoxy(C₁-C₆)-alkyle(C₁-C₄), et le groupe hétérocyclyle portant n groupes oxo,
R⁷ représente un atome d'hydrogène, un groupe alkyle en C₁-C₆, alcényle en C₂-C₆, alcynyle en C₂-C₆, cycloalkyle en C₃-C₆, cycloalcényle en C₃-C₆, cycloalkyl(C₃-C₆)-alkyle(C₁-C₆), phényle, hétéroaryle, hétérocyclyle, les neuf radicaux nommés en dernier étant substitués chacun par s radicaux choisis dans le groupe constitué par cyano, halogéno, nitro, OR¹⁰, S(O)ₙR¹¹, N(R¹⁰)₂, NR¹⁰SO₂R¹¹, CO₂R¹⁰, CON(R¹⁰)₂ et alcoxy(C₁-C₄)-alcoxycarbonyle(C₂-C₆) et le groupe hétérocyclyle portant n groupes oxo,
R⁸ représente un groupe alkyle en C₁-C₆, alcényle en C₂-C₆, alcynyle en C₂-C₆, cycloalkyle en C₃-C₆, cycloalcényle en C₃-C₆, cycloalkyl(C₃-C₆)-alkyle (C₁-C₆), phényle, hétéroaryle, hétérocyclyle, les neuf radicaux nommés en dernier étant substitués chacun par s radicaux choisis dans le groupe constitué par cyano, halogéno, nitro, OR¹⁰, S(O)ₙR¹¹, N(R¹⁰)₂, NR¹⁰SO₂R¹¹, CO₂R¹⁰, CON(R¹⁰)₂ et alcoxy (C₁-C₄)-alcoxycarbonyle (C₂-C₆) et le groupe hétérocyclyle portant n groupes oxo,
R⁹ représente le groupe méthyle ou éthyle,
R¹⁰ représente un atome d'hydrogène, un groupe alkyle en C₁-C₆, halogéno-alkyle(C₁-C₆), alcényle en C₂-C₆, alcynyle en C₂-C₆, cycloalkyle en C₃-C₆ ou cycloalkyl(C₃-C₆)-alkyle(C₁-C₆),
R¹¹ représente un groupe alkyle en C₁-C₆, alcényle en C₂-C₆ ou alcynyle en C₂-C₆,
R¹² représente un groupe acétoxy, acétamido, N-méthyl-acétamido, benzoyloxy, benzamido, N-méthylbenzamido, méthoxycarbonyle, éthoxycarbonyle, benzoyle, méthylcarbonyle, trifluorométhylcarbonyle, aminocarbonyle, méthylaminocarbonyle, diméthylaminocarbonyle, alcoxy en C₁-C₆, cycloalkyle en C₃-C₆ ou un groupe hétéroaryle, hétérocyclyle ou phényle chacun substitué par s radicaux choisis dans le groupe constitué par méthyle, méthoxy, trifluorométhyle et halogéno,
R¹³ représente un groupe alkyle en C₁-C₆,
W représente 0, S ou NR¹³,
n représente 0, 1 ou 2,
s représente 0, 1, 2 ou 3.

3. Benzamides selon la revendication 1 ou 2, dans lesquels
Q représente un radical Q1 ou Q2, 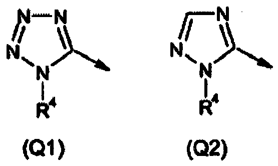
R représente -CH=N-OR¹, -CH₂-O-N=CR²R³,
X représente un groupe nitro, un atome d'halogène, un groupe alkyle en C₁-C₆, halogéno-alkyle(C₁-C₆), S(O)ₙR⁸, alkyl(C₁-C₆)-S(O)ₙR⁸, alkyl(C₁-C₆)-OR⁷, alkyl(C₁-C₆)-SO₂N(R⁷)₂ ou alkyl (C₁-C₆)-NR⁷SO₂R⁸,
Y représente un groupe nitro, un atome d'halogène, un groupe halogéno-alkyle(C₁-C₆) ou S(O)ₙR⁸,
R¹ représente un atome d'hydrogène, un groupe alkyle en C₁-C₆, alcényle en C₂-C₆, alcynyle en C₂-C₆, cycloalkyle en C₃-C₆ ou cycloalkyl (C₃-C₆) -alkyle (C₁-C₆), les cinq radicaux nommés en dernier étant substitués chacun par s radicaux choisis dans le groupe constitué par cyano, halogéno, OR¹⁰ et S(O)ₙR¹¹,
R² et R³ représentent chacun indépendamment l'un de l'autre un atome d'hydrogène, un groupe alkyle en C₁-C₆, halogéno-alkyle(C₁-C₆), alcényle en C₂-C₆, alcynyle en C₂-C₆, alcoxy(C₁-C₆)-alkyle(C₁-C₆) ou halogéno-alcoxy (C₁-C₆)-alkyle(C₁-C₆),
R⁴ représente un groupe alkyle en C₁-C₆, alcényle en C₂-C₆ ou alcynyle en C₂-C₆ ces trois radicaux étant substitués chacun par s radicaux alcoxy en C₁-C₆,
R⁵ représente un groupe alkyle en C₁-C₆, alcényle en C₂-C₆, alcynyle en C₂-C₆, cycloalkyle en C₃-C₇, alcoxy en C₁-C₆, alcényloxy en C₂-C₆, alcynyloxy en C₂-C₆, acétylamino, un atome d'halogène ou le groupe méthoxyméthyle,
R⁶ représente un groupe alkyle en C₁-C₆, R⁷O-alkyle(C₁-C₆) , CH₂R¹², cycloalkyle en C₃-C₇, alcényle en C₂-C₆ ou alcynyle en C₂-C₆,
R⁷ représente un atome d'hydrogène, un groupe alkyle en C₁-C₆, alcényle en C₂-C₆, alcynyle en C₂-C₆, cycloalkyle en C₃-C₆ ou cycloalkyl(C₃-C₆)-alkyle(C₁-C₆),
R⁸ représente un groupe alkyle en C₁-C₆, alcényle en C₂-C₆, alcynyle en C₂-C₆, cycloalkyle en C₃-C₆, cycloalcényle en C₃-C₆ ou cycloalkyl(C₃-C₆)-alkyle (C₁-C₆),
R⁹ représente le groupe méthyle ou éthyle,
R¹⁰ représente un atome d'hydrogène, un groupe alkyle en C₁-C₆, alcényle en C₂-C₆, alcynyle en C₂-C₆, cycloalkyle en C₃-C₆ ou cycloalkyl(C₃-C₆)-alkyle(C₁-C₆),
R¹¹ représente un groupe alkyle en C₁-C₆, alcényle en C₂-C₆ ou alcynyle en C₂-C₆,
R¹² représente un groupe acétoxy, acétamido ou cycloalkyle en C₃-C₆,
R¹³ représente un groupe alkyle en C₁-C₆,
W représente O, S ou NR¹³,
n représente 0, 1 ou 2,
s représente 0, 1, 2 ou 3.

4. Compositions herbicides, **caractérisées par** une teneur à activité herbicide en au moins un composé de formule (I) selon l'une quelconque des revendications 1 à 3.

5. Compositions herbicides selon la revendication 4, en mélange avec des adjuvants de formulation.

6. Compositions herbicides selon la revendication 4 ou 5, contenant au moins une autre substance à activité pesticide, choisie dans le groupe constitué par les insecticides, acaricides, herbicides, fongicides, phytoprotecteurs et régulateurs de croissance.

7. Compositions herbicides selon la revendication 6, contenant un phytoprotecteur.

8. Compositions herbicides selon la revendication 7, contenant du cyprosulfamide, du cloquintocet-mexyl, du méfenpyr-diéthyl ou de l'isoxadifène-éthyl.

9. Compositions herbicides selon l'une quelconque des revendications 6 à 8, contenant un autre herbicide.

10. Procédé pour la lutte contre des plantes indésirables, **caractérisé en ce qu'**on applique sur les plantes ou le lieu de la croissance de plantes indésirables une quantité efficace d'au moins un composé de formule (I) selon l'une quelconque des revendications 1 à 3 ou d'une composition herbicide selon l'une quelconque des revendications 4 à 9.

11. Utilisation de composés de formule (I) selon l'une quelconque des revendications 1 à 3 ou de compositions herbicides selon l'une quelconque des revendications 4 à 9, pour la lutte contre des plantes indésirables.

12. Utilisation selon la revendication 11, **caractérisée en ce que** les composés de formule (I) sont utilisés pour la lutte contre des plantes indésirables dans des cultures de plantes utiles.

13. Utilisation selon la revendication 12, **caractérisée en ce que** les plantes utiles sont des plantes utiles transgéniques.
